# FASCICULE DE BREVET EUROPEEN

(11) **EP 2 027 138 B1**
(45) Date de publication et mention de la délivrance du brevet: **05.12.2012**
(21) Numéro de dépôt: 07731342.7
(22) Date de dépôt: 23.04.2007
(51) Int. Cl.: C07H 7/00

(54) **MIMES STABLES DE SUCRES DE TYPE C-GLYCOSIDES ET C- GLYCOCONJUGUES, LEUR PROCEDE DE PREPARATION ET LEURS APPLICATIONS NOTAMMENT DANS LE DOMAINE DE LA COSMETIQUE ET DU MEDICAMENT.**
STABILE C-GLYCOSIDZUCKER UND C-GLYCOKONJUGATMIMETIKA, VERFAHREN ZU DEREN HERSTELLUNG UND DEREN ANWENDUNGEN, INSBESONDERE IN KOSMETIKA UND ARZNEIMITTELN
STABLE C-GLYCOSIDE SUGAR AND C-GLYCOCONJUGATE MIMETICS, METHOD FOR PREPARING SAME AND USES THEREOF IN PARTICULAR IN COSMETICS AND DRUGS

(30) Priorité: 27.04.2006 FR 0603823
(43) Date de publication de la demande: 25.02.2009
(73) Titulaire: TFCHEM, 76000 Rouen (FR)
(72) Inventeur: CASTELOT-DELIENCOURT-GODEFROY, Géraldine, F-76000 Rouen (FR); QUIRION, Jean-Charles, 27310 Bourg-Achard (FR); JUBAULT, Philippe, F-76160 Preaux (FR); ZOUTE, Ludivine, F-59390 Sailly lez Lannoy (FR)
(74) Mandataire: Regimbeau
(86) Numéro de dépôt international: PCT/FR2007/000683
(87) Numéro de publication internationale: WO 2007/128899

(56) Documents cités:
- EP-A- 0 354 323
- WO-A1-2006/059227
- FR-A1- 2 842 810
- GUTHRIE R D ET AL: "SYNTHESIS OF 1-, 6- AND 1,6-DERIVATIVES OF METHYL D-FRUCTOFURANOSIDES" AUSTRALIAN JOURNAL OF CHEMISTRY, vol. 35, no. 5, 1982, pages 1003-1018, XP009014310 ISSN: 0004-9425
- CUENCA, ANA B. ET AL: "Addition of ethyl bromo-difluoro-acetate to lactones: Reactivity and stereoselectivity" SYNLETT , (17), 2627-2630 CODEN: SYNLES; ISSN: 0936-5214, 2005, XP009076039
- MARCOTTE S ET AL: "Synthesis of new alpha- and beta-gem-difluoromethylene C-glycosides in the galactose and glucose series" TETRAHEDRON LETTERS, ELSEVIER, AMSTERDAM, NL, vol. 42, no. 34, 20 août 2001 (2001-08-20), pages 5879-5882, XP004295947 ISSN: 0040-4039
- TONY, KURISSERY A. ET AL: "Synthesis of .beta.-C-galacto-Pyranosides with Fluorine on the Pseudoanomeric Substituent" ORGANIC LETTERS , 9(8), 1441-1444 CODEN: ORLEF7; ISSN: 1523-7060, 2007, XP002464204

## Description

La présente invention concerne une nouvelle famille de mimes de sucres de type C-glycosides et C-glycoconjugués utilisables dans de nombreux domaines tels que la cosmétologie, l'imagerie médicale, ou même dans des applications pharmaceutiques par exemple en tant qu'agent antifongique, antiparasitaire, anti-thrombotique, antibiotiques, antiviral, anti-infectieux, anti-inflammatoire, antipsychotique, antidépresseur ou même antinéoplasique.

D'une manière générale, on sait que les sucres constituent une classe fondamentale de biomolécules, impliqués dans des fonctions très variées : Ils ne constituent pas seulement des formes de réserve de l'énergie, mais ils participent aux communications cellulaires, au fonctionnement du système immunitaire, aident l'organisme à lutter contre des microorganismes pathogènes, et interviennent dans les processus de cancérisation. C'est donc cette habilité à communiquer avec d'autres cellules, des protéines, des hormones, des virus, des toxines ou des bactéries qui font des sucres un véritable arsenal pour le développement de nouveaux traitements notamment dans le domaine des cancers, des virus, de l'inflammation et de nombreux autres encore.

Ils sont présents dans des médicaments connus tels que des médicaments du système cardiovasculaire avec les glucosides cardiotoniques, des anticoagulants avec l'héparine, des antibiotiques de type aminoglycosides ou glycopeptides, des antinéoplasiques de type antibiotiques cytotoxiques et d'autres encore. De plus, l'ajout de sucres solubles dans l'eau sur des principes actifs médicamenteux permet d'en améliorer la solubilité dans des milieux biologiques et d'en modifier les propriétés pharmacocinétiques (circulation, élimination et concentration dans les milieux biologiques). La glycosylation peut également retarder des processus de dégradation (c'est le cas notamment avec des peptides tels que les peptides opioides: les enképhalines), influencer le transport au travers de nombreuses barrières telles que la barrière hématoencéphalique, et bloquer ainsi l'entrée dans le cerveau ou au contraire en faciliter le transport en ciblant des systèmes de transport actif du glucose. De plus la glycosylation peut également renforcer des interactions avec des récepteurs ou des lectines présentes à la surface des cellules et ainsi induire une vectorisation et une sélectivité plus importante sous la forme de glycoconjugués.

Il n'en demeure pas moins que, malgré le potentiel thérapeutique des glycosides et de leurs dérivés, leur exploitation commerciale pour le développement de nouveaux médicaments est bien souvent occultée par des inconvénients majeurs :
- coûts importants et difficultés de synthèse; de purification et d'analyse,
- forte instabilité face à des processus d'hydrolyse chimique et enzymatique et donc dégradation rapide dans l'organisme notamment par les glycosidases présentes en grand nombre dans ce milieu.

La connaissance du potentiel thérapeutique des sucres et de leurs limitations, a renforcé la recherche de mimétiques de ces structures, comme nouvelle voie d'accès à des agents thérapeutiques plus efficaces. Et si certains d'entre eux ont trouvé des applications, c'est toujours de manière ponctuelle. Il existe ainsi une importante variété de structures hybrides de sucres qui ont été développées : parmi elles, les C-glycosides constituent une avancée majeure dans la résolution des problèmes de stabilité des sucres et de leurs dérivés.

Dans ce domaine, deux familles ont émergé les CH₂ et les CF₂-Glycosides (des exemples de ces derniers étant décrits notamment dans FR 0 354 323, Cuenca et al. Synlett 2005, 2627-2630, et Marcotte et al. Tetrahedron Lett. 2001, 42, 5879-5882), dans lesquels l'oxygène anomérique est remplacé par un groupement CH₂ ou CF₂ afin de lever les problèmes de stabilité des sucres. Cependant si le CH₂ présente un fort intérêt en termes de stabilité, le remplacement de l'oxygène anomérique par ce groupement induit des changements importants en termes d'électronégativité, de polarité et donc de comportement du nouveau sucre dans des phénomènes biologiques. Le remplacement de l'oxygène anomérique par un CF₂ induit quant à lui une bonne stabilité, mais surtout une excellente substitution en termes d'électronégativité. Cependant la présence du CF₂, de part l'effet inductif attracteur des 2 atomes de fluor peut sensibiliser des fonctions voisines, tels que des carbonyles qui peuvent ainsi subir des attaques par des fonctions nucléophiles telles que des amines.

L'invention a plus particulièrement pour but de supprimer ces inconvénients grâce à une nouvelle famille de mimes de sucres : C-glycosides et C-glycoconjugués dans laquelle l'oxygène de la fonction anomérique est remplacé par un groupement comprenant un atome de carbone portant un atome de fluor, stable face à des dégradations enzymatiques et d'hydrolyse acido-basique, et présentant une sensibilité réduite face à des attaques nucléophiles.

Elle propose plus particulièrement un composé C-glycoside stabilisé qui, utilisé comme analogue ou adduit/véhicule de composés biologiquement actifs, peut en améliorer l'activité.

Selon l'invention, ce composé C-glycoside présente la formule I suivante : 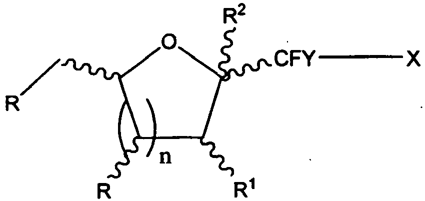 où
n est un nombre entier égal à 1 ou 2,
Y représente un atome d'hydrogène ou un atome de brome ou de chlore,
X est un atome d'hydrogène ou une chaîne alkyle linéaire ou ramifié possédant au moins une fonction amine, amide, acide, ester, carbonyle, alcool, aryle ou un groupe carbonyle, ester, amide, amine, alcool libre ou protégé,
R identiques ou différents,
représentent un groupement OH ou OR'
où R' est un groupement alkyle linéaire ou ramifié, benzyle, benzoyle, acétyle, pivaloyle, trialkylsilyle, tertiobutyldiphénylsilyle ou un ou plusieurs sucres,
R¹ représente OR', NR"R"', N₃, ou un phtalimide
   R"et R"', identiques ou différents, représentent un atome d'hydrogène ou un groupe alkyle linéaire ou ramifié, aryle, benzyle, benzoyle, acétyle, alkyloxycarbonyle, allyloxycarbonyle, benzyloxycarbonyle,
R² représente un atome d'hydrogène ou un halogène, de préférence un halogène choisi parmi F, Cl, Br, I, ou un groupement OH, OR', NR"R"' ou N₃,
ainsi que ses dérivés à l'état de base, de sel d'addition à un acide minéral ou organique, d'hydrate ou de solvate physiologiquement ou pharmaceutiquement acceptable,
à l'exclusion des composés suivants :
- 3,4,6-tri-O-benzoyl-1-déoxy-1-fluoro-β-D-fruetofuranoside de méthyle,
- Ester éthylique de l'acide 2-déoxy-2-fluoro-4,5,7-tris-O-(phénylméthyl)-D-arabino-3-heptulofuranosonique,
- acide 2-déoxy-2-fluoro-4,5,7-tris-O-(phénylméthyl)-D-arabino-3-heptulofurano sonique,
- 1-déoxy-1-fluoro-3,4,6-tris-O-(phénylméthyl)-D-fructofuranose,
- diacétate de 1-déoxy-1-fluoro-3,4-bis-O-(phénylméthyl)-D-fructofuranose, et
- 1-déoxy-1-fluoro-3,4-bis-O-(phénylméthyl)-D-fructofuranose.

Les composés exclus correspondent à des composés décrits dans Guthrie et al. Australian Journal of Chemistry 1982, 35, 1003-1018 et EP 0 354 323, sans qu'aucune activité biologique de ces composés ne soient rapportée dans ces deux documents.

Les groupes alkyles linéaires ou ramifiés pourront être des groupes possédant de 1 à 15 atomes de carbones.

Cette nouvelle famille de C-glycosides et de C-glycoconjugués monotluorée permet avantageusement d'obtenir soit :
1. **Une stabilisation de glycoconjugués** : les préparations d'analogues modifiés par cette technologie de glycosides et glycoconjugués, pourront ainsi présenter une meilleure stabilité, biodisponibililé et donc efficacité que les composés parents. De plus, elles pourront plus aisément être administrées par voie orale. Ceci améliorera leur utilisation en tant que médicament.
2. **Une néo-glycosylation** : la préparation de versions glycosylés de principes actifs médicamenteux. L'ajout de sucres solubles dans l'eau sur des principes actifs médicamenteux permet d'en améliorer la solubilité dans des milieux biologiques et d'en modifier les propriétés pharmacocinétiques (circulation, élimination et concentration dans les milieux biologiques). La glycosylation peut également retarder des processus de dégradation (c'est le cas notamment avec des peptides tels que les peptides opioides: les enképhalines), influencer le transport au travers de nombreuses barrières telles que la barrière hématocncéphalique, et bloquer ainsi l'entrée dans le cerveau ou au contraire en faciliter le transport en ciblant des systèmes de transport actif du glucose. La glycosylation est importante également pour des barrières tels que la barrière placentaire et peut ainsi empêcher l'intoxication du foetus. De plus la glycosylation peut également renforcer des interactions avec des récepteurs ou des lectines présentes à la surface des cellules et ainsi induire une vectorisation et une sélectivité plus importante sous la forme de glycoconjugués. De plus, ces composés bénéficieront d'une plus grande stabilité et de l'effet de l'introduction de l'atome de fluor.

L'invention a également pour objet un procédé de préparation des composés de formule I.

Selon une première variante, lesdits composés de formule I dans lesquels Y représente un atome d'hydrogène pourront être obtenus par un procédé comportant une réaction d'un dibromofluoroacétate d'alkyle en présence de diéthylzinc et de triphénylphosphine sur les lactones de formule II : 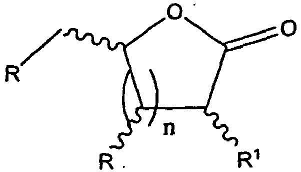 avec n, R, R¹ tels que définis précédemment.

Des composés de formule I dans lesquels X et Y sont des atomes d'hydrogène et R² représente un hydroxyle (OH) peuvent être obtenus comme produits secondaires de la réaction d'un composé de formule I dans lequel R² = OH, Y = H et X = CO₂H en présence d'un agent de couplage peptidique tel que le 3-éthyl-1-(N,N-diméthylaminopropylcarbodiimide (EDCI) ou la dicyclohexyl carbodiimide (DCC) en présence d'une amine tertiaire telle que la N-méthylmorpholine (NMM) ou la diisopropylethylamine (DIEA).

Selon une deuxième variante, lesdits composés de formule 1 dans lesquels Y représente un atome d'hydrogène pourront être obtenus par un procédé comportant une réaction d'addition de Reformatsky d'un bromofluoroacétate d'alkyle en présence de zinc sur les lactones de formule II.

Avantageusement, le choix de l'une ou l'autre de ces deux variantes permet de privilégier l'une ou l'autre des configurations du centre asymétrique portant l'atome de fluor.

Selon une troisième variante, lesdits composés de formule I dans lesquels Y représente un atome d'halogène tel que chlore et brome pourront être obtenus par un procédé comportant une réaction du dihalogénofluoroacétate d'alkyle en présence de diéthylzinc sur les lactones de formule II.

Lesdites lactones pourront être obtenues par des étapes classiques de protection par benzylation du sucre, suivi de l'hydrolyse acide de la position anomérique, puis de son oxydation.

Les composés de structure générale I avec R²=OH pourront être halogénés pour obtenir des composés de structure générale I avec R²= Cl ou Br puis réduits pour obtenir des composés de structure générale I avec R²=H.

Les composés de formule III, également obtenus avec le procédé de préparation utilisant le diéthylzinc, le dibromofluoroacétate et la triphénylphosphine pourront constituer des composés actifs. 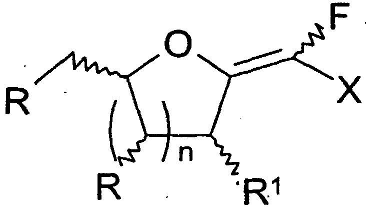 avec n, R, R¹, X tels que définis précédemment.

Plus spécifiquement, des composés de formule III dans lesquels X = Br pourront être obtenus par réaction de lactone de formule II en présence de tribromofluorométhane (CFBr₃), de triphénylphosphine et de diéthylzinc.

Des composés de formule III dans lesquels X=H pourront être obtenus par réaction d'un composé de formule I dans lequel X = CO₂H, R²= OH et Y = H est mis à réagir en présence d'un agent de couplage peptidique tel que le 3-éthyl-1(N,N-diméthylaminopropylcarbodiimide (EDCI) ou la dicyclohexyl carbodiimide (DCC) en présence d'une amine tertiaire telle que la N-méthylmorpholine (NMM) ou la diisopropylethylamine (DIEA).

Dans les composés de formule générale III, la double liaison pourra être réduite pour donner des composés de formule générale I avec R²=H et Y=H, mais également :
- des composés de formule IV 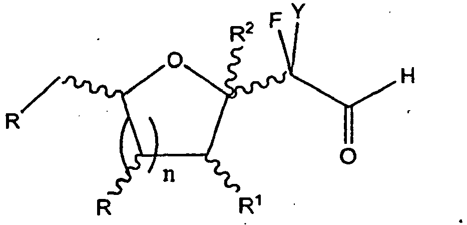 où
   n est un nombre entier égal 2,
   Y représente un atome d'hydrogène,
   R² représente un atome d'hydrogène ou un groupement OH, OR', et obtenu à partir de l'ester (X=CO₂Et dans la formule I), soit par réduction avec l'Hydrure de diisobutylaluminium (DIBALH), soit par réduction en alcool (formule V) suivi par une oxydation
- des composés de formule V 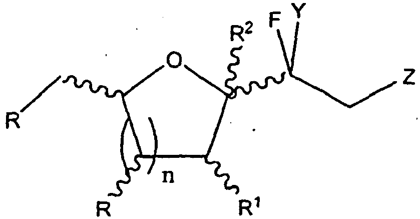 où
   n est un nombre entier égal 2,
   Y représente un atome d'hydrogène,
   R² représente un atome d'hydrogène ou un groupement OH, OR'
   Z représente un OH, OR³ avec R³= alkyl, benzyl, mesyl, tosyl, triflate ou un halogène tel que Cl, Br, I.
- des composés de formule VI 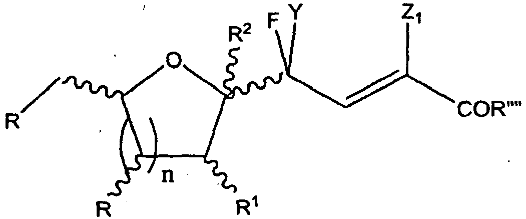 où
   n est un nombre entier égal 2,
   Y représente un atome d'hydrogène,
   R² représente un atome d'hydrogène ou un groupement OH, OR'
   Z₁ représente un H ou NR"R"' avec R"et R"', identiques ou différents, représentent un atome d'hydrogène ou un groupe alkyle linéaire ou ramifié, aryle, benzyle, benzoyle, acétyle, alkyloxycarbonyle, allyloxycarbonyle, benzyloxycarbonyle,
   R"" représentent OR" ou NR"R"' ou un acide aminé obtenu par réaction de type Wittig-Wadsworth-Horner-Emmonth d'un phosphonate sur l'aldéhyde de formule IV
- des composés de formule VII. 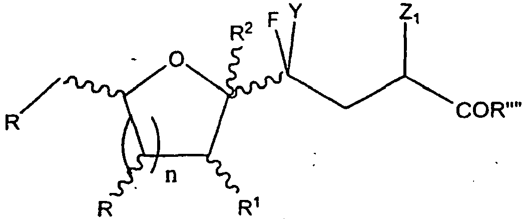 où
   n est un nombre entier égal 2,
   Y représente un atome d'hydrogène,
   R² représente un atome d'hydrogène ou un groupement OH, OR'
   Z₁ représente un H ou NR"R"' avec R" et R"', identiques ou différents, représentent un atome d'hydrogène ou un groupe alkyle linéaire ou ramifié, aryle, benzyle, benzoyle, acétyle, alkyloxycarbonyle, allyloxycarbonyle, benzyloxycarbonyle,
   R"" représentent OR" ou NR"R"' ou un acide aminé obtenu par réduction de la double liaison de la formule VI
- des composés de formule VIII 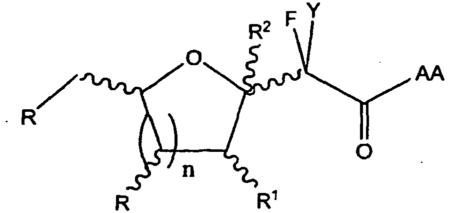 où
   n est un nombre entier égal 2,
   Y représente un atome d'hydrogène,
   R² représente un atome d'hydrogène ou un groupement OH, OR',
      et obtenu à partir de l'ester (X=CO₂Et dans la formule I), par saponification en acide (X=CO₂H dans la formule I) par action de lalithine ou de la soude ou de la potasse, suivi d'un couplage peptidique avec AA : un acide aminé ou un peptide c'est-à-dire un enchaînement d'acides aminés en présence d'agents de couplage classique tels que le dicycohexylcarbodümide (DCC), le 3-éthyl-1(N,N-diméthylaminopropylcarbodiimide (EDCI), le (2-(7-Aza-1H benzotriazole-1-yl)-1,1,3,3-tetraméthyluronium hexafluorophosphate) (HATU), (2-1H benzotriazole-1-yl)-1,1,3,3-tetraméthyluronium hexafluorophosphate (HBTU) avec ou sans Hydroxybenzotriazole (HOBt), et d'une amine tertiaire telle que la N-méthylmorpholine (NMM) ou la diisopropyléthylamine (DIEA) ou la triéthylamine (Et₃N).
- des composés de formule IX. 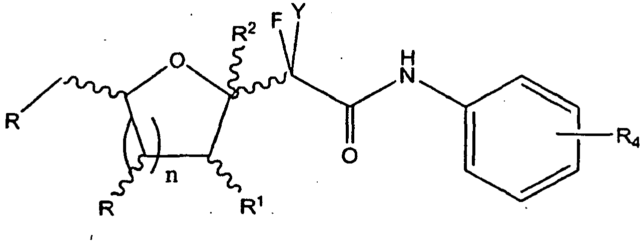 où
   n est un nombre entier égal 2,
   Y représente un atome d'hydrogène,
   R² représente un atome d'hydrogène ou un groupement OH, OR',
   R4 représente un hydrogène, halogène, NR"R"', OH ou OR"
   avec R"et R"', identiques ou différents, représentent un atome d'hydrogène ou un groupe alkyle linéaire ou ramifié, aryle, benzyle, benzoyle, acétyle, alkyloxycarbonyle, allyloxycarbonyle, benzyloxycarbonyle, ou obtenu à partir de l'ester (X=CO₂Et dans la formule I), par saponification en acide (X=CO₂H dans la formule I) par action de lalithine ou de la soude ou de la potasse, suivi d'un couplage avec une amine aromatique en présence d'agents de couplage classique tels que DCC, EDCI, HATU, HBTU) avec ou sans HOBT, et d'une amine tertiaire telle que NMM, DIEA, Et3N.

L'invention concerne également un composé C-glycoside de formule I dans lequel le radical R² consiste en un groupement OH se présentant, lorsqu'il est en solution dans des solvants polaires et protiques, sous les différentes formes classiques des sucres en solution, à savoir : des formes ouverte, furanose et pyranose.

Ainsi, par exemple, en série galactose, le composé pourra présenter la formule X suivante :

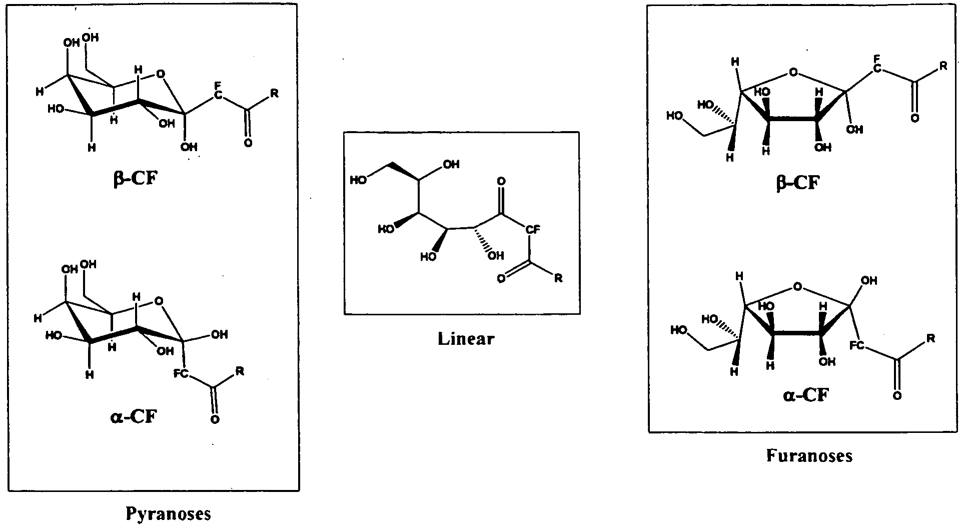

L'intérêt des composés C-glycosides ou C-glycoconjugés selon l'invention par rapport aux technologies antérieures et aux glycosides et glycoconjugués naturels réside dans :
- L'existence d'une électronégativité due à la présence d'un atome de fluor, permet de conserver la polarité de la molécule.
- Son caractère particulièrement résistant aux dégradations enzymatiques et aux conditions d'hydrolyses acido-basique permet donc l'obtention de composés non hydrolysables (chimiquement et enzymatiquement), ce qui aura un effet bénéfique au niveau de la biodisponibilité, du temps de ½ vie de ces composés lors de leur utilisation comme médicaments. Il améliore ainsi la stabilité métabolique, en bloquant un site métabolique par un atome de fluor, atome suffisamment petit pour ne pas défavoriser les interactions avec les récepteurs.
- Un effet inductif du à l'atome de fluor, qui n'a pas d'effet de fragilisation des fonctions voisines face à des nucléophiles.
- L'introduction d'un centre asymmétrique sur la position anomérique, pourra induire une meilleure affinité dans des interactions avec les récepteurs, car cette position est souvent impliquée dans les glycosides naturels dans des interactions avec des récepteurs.
- Les modifications des propriétés physicochimiques, et notamment au niveau de l'acidité et de la basicité des fonctions voisines sont extrêmement proches dans le cas de l'introduction d'un seul atome de fluor, des composés possédant un oxygène. Souvent, des changements de pKa ont un effet très fort sur les propriétés pharmacocinétiques et les interactions d'une molécule.
- L'impact dans des interactions avec les protéines, cet effet peut être direct entre le fluor et protéine ou indirect via des fonctions voisines au fluor dont la polarité est ainsi modifiée, étant entendu que des interactions C-F...C=O peuvent jouer un rôle important en augmentant ainsi de manière significative les interactions.

Des exemples de préparation de composés selon l'invention seront décrits ci-après, à titre d'exemples non limitatifs, avec référence aux dessins annexés dans lesquels :
La figure 1 est une équation de réaction pour obtenir le composé **2a₁/2a₂; 2b₁/2b₂**; **2c₁/2c₂**
La figure 2 est une équation de réaction pour obtenir le composé **2a₁/2a₂**; **2b₁/2b₂** ; **2c₁/2c₂** ; ainsi que **3a₁/3a₂; 3b₁/3b₂ ; 3c₁/3c₂**
La figure 3 est une équation de réaction pour obtenir le composé **4a₁/4a₂**; **4b₁/4b₂** ; **4c₁/4c₂** ;
La figure 4 est une équation de réaction pour obtenir le composé **5a₁/5a₂**; **5b₁**.
La figure 5 est une équation de réaction pour obtenir le composé **6a₂; 6b₂** ;
La figure 6 est une équation de réaction pour obtenir le composé **7a₂; 7b₁/7b₂**;
La figure 7 est une équation de réaction pour obtenir le composé **8b₁/8b₂** ;
La figure 8 est une équation de réaction pour obtenir le composé **9b₁/9b₂** ;
La figure 9 est une équation de réaction pour obtenir le composé **10b₂** ;
La figure 10 est une équation de réaction pour obtenir le composé **11a₁** ; **11b₂** ;
La figure 11 est une équation de réaction pour obtenir le composé **10a₁;**
La figure 12 est une équation de réaction pour obtenir le composé **12a₁.**
La figure 13 est une équation de réaction pour obtenir le composé **13a₂;**
La figure 14 est une équation de réaction pour obtenir le composé 10a₂ ;
La figure 15 est une équation de réaction pour obtenir le composé 14a₁/14a₂;
La figure 16 est une équation de réaction pour obtenir le composé **15a₁**;
La figure 17 est une équation de réaction pour obtenir le composé **16a₁** ;
La figure 18 est une équation de réaction pour obtenir le composé **17a₁** ;
La figure 19 est une équation de réaction pour obtenir le composé **18b₁** .
La figure 20 est une équation de réaction pour obtenir le composé **19b₁/19b₂**;
La figure 21 est une équation de réaction pour obtenir le composé **20b₁/20b₂**;
La figure 22 est une équation de réaction pour obtenir le composé **21b₂**;
La figure 23 est une équation de réaction pour obtenir le composé **22a₁/22a₂**; **23**
La figure 24 est une équation de réaction pour obtenir le composé **24b₁/24b₂**
La figure 25 est un exemple de dégradation enzymatique de O-glycopeptides par des glycosidases
La figure 26 est un exemple de résistance des CHF-glycopeptides aux glycosidases

Les abréviations rencontrées sont définies ainsi :

| | | |
|---|---|---|
| éq. : équivalent | g : gramme | Hz : Hertz |
| mg : milligramme | MHz : mégaHertz | min.: minute |
| mL: millilitre | mmol: millimole | µmol: micromole |
| nmol : nanomole | ed : excès diastéréomérique | |

Les caractéristiques des appareils utilisés pour effectuer les analyses de tous les composés décrits dans la présente demande sont indiquées ci-dessous :

Les spectres RMN ¹H, ¹³C, ¹⁹F ont été enregistrés sur des spectromètres BRUMER DPX 300 et DPX 600. En RMN ¹H et ¹³C, le tétraméthylsilane est utilisé comme référence interne. En RMN ¹⁹F, la référence externe est le fluorotrichlorométhane CFCl₃. Les déplacements chimiques sont exprimés en partie par million (ppm), les constantes de couplage J en Hertz (Hz).

Les abréviations suivantes ont été utilisées :
s pour singulet, bs pour un large singulet, d pour doublet, t pour triplet, qdt pour quadruplet, m pour multiplet ou massif, dd pour doublet de doublet...

Les spectres de masse ont été obtenus sur un spectrophotomètre de type Micromass TOF-SPEC, E 20 kV, α-cyano. pour l'ionisation Maldi et JEOL AX500, 3 kV, Canon FAB JEOL, Xe, 4 kV, courant limite 10 µA, Gly-NBA 50 :50 pour l'ionisation FAB.

Les séparations par chromatographie sur colonne sont réalisées sous pression légère en suivant les techniques de chromatographie sur silice Kieselgel 60 (230-400 Mesh, Merck).
Le suivi est assuré par chromatographie sur couches minces (CCM) avec des plaques Kieselgel 60F-254-0.25mm. On appelle rapport frontal (Rf) le rapport de la distance de migration d'un composé sur un support donné sur la distance de migration d'un éluant.

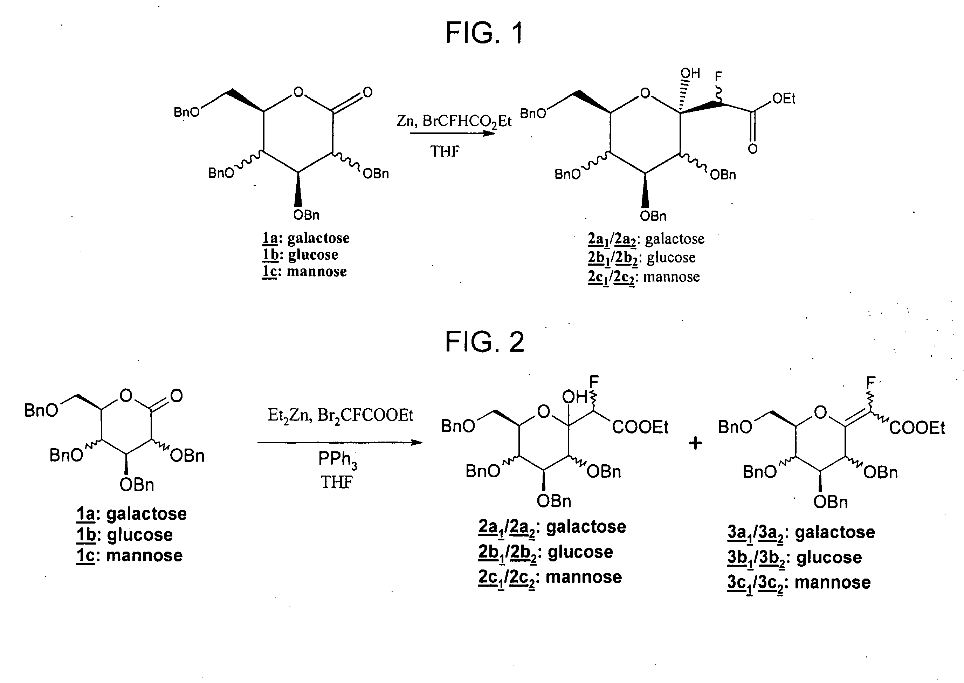

### Synthèse des composés 2a₁/2a₂ (Figure 1)

Dans un ballon sous atmosphère inerte contenant le Zinc (2,55 g ; 38,96 mmol ; 7 éq.) préalablement activé et décapé, on additionne le THF (40 mL). Le milieu est porté au reflux, puis on additionne goutte à goutte un mélange constitué de la lactone **1a** (3 g ; 5,57 mmol ; 1 éq.) et de bromofluoroacétate d'éthyle (1,97 mL ; 16,7 mmol ; 3 éq.) dans le THF (40 mL). La réaction est laissée au reflux pendant 3 heures. Après retour à la température ambiante, on ajoute au milieu réactionnel une solution de HCl 1N (60 mL). On filtre le mélange sur Buchner pour éliminer le zinc en excès. On ajoute à la solution du dichlorométhane (40 mL). On sépare les 2 phases et on extrait à nouveau deux fois la phase aqueuse au dichlorométhane. Les phases organiques sont rassemblées, séchées sur sulfate de magnésium, filtrées puis concentrées.

Le mélange est ensuite purifié sur colonne de silice avec comme éluant un mélange cyclohexane/acétate d'éthyle dans des proportions de 8 pour 2 pour obtenir une huile incolore pour le diastéréoisomère minoritaire **2a₁** et une huile jaune claire pour le diastéréoisomère majoritaire **2a₂** avec un rendement pondéral global de 70%.
ed= 38 (69-31) déterminé en RMN ¹⁹F sur le brut réactionnel

### Caractérisation des composés 2a₁/2a₂

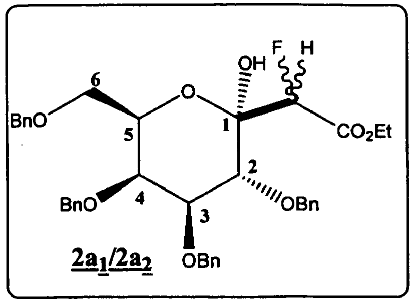

C₃₈H₄₁FO₈ M= 644,73 g.mol⁻¹

### 2a₁ -Diastéréoisomère minoritaire

Rf : 0,53 (cyclohexane/acétate d'éthyle 7/3)
**RMN ¹⁹F** (CDCl₃, 282,5 MHz)
-200,5 (dd, J_{F-H}=47 et 2 Hz)
**RMN ¹H (CDCl₃, 300MHz)**
1,2 (t, 7,2, 3H, CH₃); 3,4 (dd, 5,8-9,2 1H, H6) ; 3,6 (dd, 7,7-9,3, 1H, H6) ; 3,9 (s, 1H, H4) ; 4 (dd , 2,6-10, 1H, H3); 4,1 (dd, 6,5, 1H, H5) ; 4,2 (m, 2H, CH₂) ; 4,3 (d, 11,4, 1 H, H2) ; 4,3-4,9 (m, 8H, 40CH₂Ph) ; 5 (d, 47Hz, 1H, CHF) ; 7,2 (m, 20H, Har)
**RMN ¹³C (CDCl₃, 75,5MHz)**
14,4 (**C**H₃) ; 62,9 (**C**H₂) ; 69,1 (C**6**) ; 71,5 (C**5**) ; 73,1 (O**C**H₂Ph) ; 73,8 (O**C**H₂Ph) ; 74,8 (C**4**) ; 74,9 (O**C**H₂Ph) ; 75,1 (C**2**) ; 76,1 (O**C**H₂Ph); 80,6 (C**3**) ; 86,9 (d, 203 Hz, **C**HF); 98,5 (d, 21 Hz, C**1**); 127,9-128,8 (**C**ar.); 138,4 ;138,5 ; 138,8 ; 139,2 (**C**ar. quat.) ; 169,5 (d, 22Hz, **C**O₂Et).

### 2a₂-Diastéréoisomère majoritaire

Rf = 0,61 (cyclohexane/acétate d'éthyle 7/3)
**RMN ¹⁹F (CDCl₃, 282,5 MHz)**
-205,2 (d, J_{F-H}=47 Hz)
**RMN ¹H (CDCl₃, 300MHz)**
0,9 (t, 7,2, 3H, C**H**₃); 3,4 (dd, 5,5-9,1 ¹H, H**6**) ; 3,5 (dd, 9, 1H, H**6**) ; 3,6 (s, 1H, OH) ; 3,9 (qdt, 7,2, 2H, C**H**₂) ; 4 (m , 2H, H**4**, H**3**); 4,1 (dd, 6,7 , 1 H, H**5**) ; 4,3 (d, 9, 1H, H**2**) ; 4,4-5 (m, 8H, 40C**H**₂Ph) ; 4,8 (d, 47Hz, 1H, C**H**F) ; 7,2 (m, 20H, **H**ar) **RMN ¹³C** (**CDCl₃,75,5MHz**)
14,1 (CH₃) ; 62,3 (CH₂) ; 68,6 (C6) ; 71,3 (C5) ; 72,7 (OCH₂Ph) 73,9 (OCH₂Ph) ; 74,3 (C4) ; 75 (OCH₂Ph) ; 75,2 (C2) ; 75,4 (OCH₂Ph) ; 81,1 (C3) ; 88,7 (d, 193 Hz, CHF) ; 97,8 (d , 20 Hz, C1) ; 127,9 -128,9 (Car.) ; 138,3 ;138,5 ; 138,6 ; 139,2 (Car. quat.) ; 166,6 (d, 25Hz, CO₂Et).

Les composés 2a₁/2a₂ peuvent également être obtenu selon une autre voie de synthèse qui conduit cette fois au diastéréoisomère 2a₁ majoritaire et 2a₂ minontaire.

### Synthèse des composés 2b₁/2b₂ (Figure 1)

Dans un ballon sous atmosphère inerte contenant le Zinc (2,55 g ; 38,96 mmol ; 7 éq.) préalablement activé et décapé, on additionne le THF (40 mL). Le milieu est porté au reflux, puis on additionne goutte à goutte un mélange constitué de la lactone 1b (3 g ; 5,57 mmol ; 1 éq.) et de bromofluoroacétate d'éthyle (1,97 mL ; 16,7 mmol ; 3 éq.) dans le THF (40 mL). La réaction est laissée au reflux pendant 3 heures. Après retour à la température ambiante, on ajoute au milieu réactionnel une solution de HCl 1N (60 mL). On filtre le mélange sur Buchner pour éliminer le zinc en excès. On ajoute à la solution du dichlorométhane (40 mL). On sépare les deux phases et on extrait à nouveau deux fois la phase aqueuse au dichlorométhane. Les phases organiques sont rassemblées, séchées sur sulfate de magnésium, filtrées puis concentrées.

Le mélange est ensuite purifié sur colonne de silice avec comme éluant un mélange cyclohexane/ acétate d'éthyle dans des proportions de 9,3 pour 0,7, pour obtenir des cristaux blancs pour le diastéréoisomère minoritaire **2b₁** et une huile jaune claire pour le diastéréoisomère majoritaire **2b₂** avec un rendement pondéral de 61%.
ed= 46 (73-27) en RMN ¹⁹F
ed= 54 (77-23) en HPLC (colonne kromasil C18, UV 254nm, 90/10 CH₃CN/H₂O 1 mL/mn)

### Caractérisation de 2b₁/2b₂

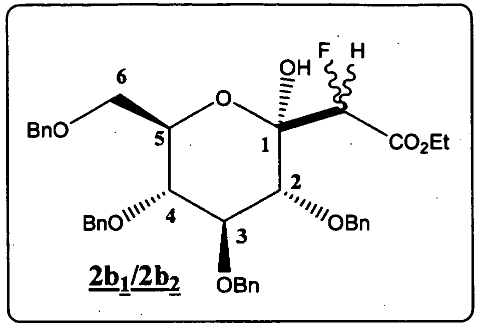

C₃₈H₄₁FO₈ M= 644,73 g.mol⁻¹

### 2b₁ Diastéréoisomère minoritaire

Rf : 0,23 (cyclohexane/acétate d'éthyle 8/2).
**RMN ¹⁹F (CDCl₃, 282,5 MHz)**
-200,2 (d, J_{F-H}=47Hz)
**RMN ¹H (CDCl₃, 300MHz)**
1,2 (t, 7,2, 3H, C**H**₃); 3,5 (dd, 1,5-12,3, 1H, H**6**) ; 3,6 (dd, 9,8, 1H, H**4**) ; 3,65 (dd, 4,6-11,4,1H, H**₆**) ; 3,7 (dd ,1,5-9,7, 1H, H**2**); 4,0 (dd, 2,7-10, 1H, H**5**) ; 4,1 (dd, 9,7, 1H, H**3**) ; 4,2 (m, 2H, C**H**₂) ; 4,4-4,9 (m, 8H, 40C**H**₂Ph) ; 4,9 (d, 44, 1H, C**H**F) ; 7,2 (m, 20H, **H**ar)
**RMN ¹³C (CDCl₃, 75,5MHz)**
14,5 (**C**H₃) ; 63,0 (**C**H₂) ; 69,0 (C**6**) ; 72,9 (C**5**) ; 73,7 (O**C**H₂Ph) ; 75,5 (O**C**H₂Ph) ; 76 (O**C**H₂Ph) ; 76,1 (O**C**H₂Ph) ; 78,5 (C**2**) ; 78,7 (C**4**) ; 83 (C**3**) ; 86,9 (d, 203 Hz, **C**HF) ; 98 (d , 21 Hz, C**1**) ; 127,9 -128,9 (**C**ar.) ; 138,2 ;138,5 ; 138,8 ; 138,9 (**C**ar. quat.) ; 163,3 (d, 23Hz, **C**O₂Et).

### 2b₂ Diastéréoisomère majoritaire

Rf= 0,17 (cyclohexane/acétate d'éthyle 8/2).
**RMN ¹⁹F (CDCl₃, 282,5 MHz)**
-205,5 (dd, J_{F-H}=47 et 11Hz)
**RMN ¹H (CDCl₃,300MHz)**
1,1 (t, 7,2, 3H, C**H**₃); 3,6 (m, 1H, H**6**) ; 3,7 (m, 2H, H**4**, H**6**) ; 3,8 (d , 9,4, 1H, H**2**); 3,9 (m, 3H, H**5**,2 C**H**₂) ; 4,1 (dd, 9,4, 1H, H**3**) ; 4,4-5 (m, 8H, 40C**H**₂Ph) ; 4,8 (d, 47Hz, 1H, C**H**F) ; 7,2 (m, 20H, **H**ar)
**RMN ¹³C (CDCl₃,75,5MHz)**
14,4 (**C**H₃) ; 62,5 (**C**H₂) ; 68,7 (C**6**) ; 72,9 (C**5**) ; 73,7 (O**C**H₂Ph) ; 75,3 (O**C**H₂Ph) ; 75,5 (O**C**H₂Ph); 76,1 (O**C**H₂Ph) ; 78,4 (C**4**); 78,7 (C**2**) ; 83,6 (C**3**) ; 86,8 (d, 195 Hz, **C**HF) ; 97,5 (d, 20 Hz, C1); 127,9 -129,9 (**C**ar.); 138,3 ;138,5 ; 138,7; 138,8 (**C**ar. quat.) ; 166,8 (d, 24Hz, **C**O₂Et).
Les composés **2b₁/2b₂** peuvent également être obtenu selon une autre voie de synthèse qui conduit cette fois au diastéréoisomère **2b₁** majoritaire et **2b₂** minoritaire.

### Synthèse des composés 2c₁/2c₂ (Figure 1)

Dans un ballon sous atmosphère inerte contenant le Zinc (2,55g; 38,96 mmol ; 7 éq.) préalablement activé et décapé, on additionne le THF (40 mL). Le milieu est porté au reflux, puis on additionne goutte à goutte un mélange constitué de la lactone **1c** (3 g ; 5,57 mmol ; 1 éq.) et de bromofluoroacétate d'éthyle (1,97 mL ; 16,7 mmol ; 3 éq.) dans le THF (40 mL). La réaction est laissée au reflux pendant trois heures. Après retour à la température ambiante, on ajoute au milieu réactionnel une solution de HCl 1N (60 mL). On filtre le mélange sur Buchner pour éliminer le zinc en excès. On ajoute à la solution du dichlorométhane (40 mL). On sépare les 2 phases et on extrait à nouveau deux fois la phase aqueuse au dichlorométhane. Les phases organiques sont rassemblées, séchées sur sulfate de magnésium, filtrées puis concentrées.

Le mélange est ensuite purifié sur colonne de silice avec comme éluant un mélange cyclohexane/ acétate d'éthyle dans des proportions de 8,5 pour 1,5, pour obtenir des cristaux blancs pour le diastéréoisomère majoritaire **2c₁** et une huile jaune claire pour le diastéréoisomère minoritaire **2c₂** avec un rendement pondéral de 67% et un ed= 56 (78-22) en RMN ¹⁹F

### Caractérisation de 2c₁/2c₂

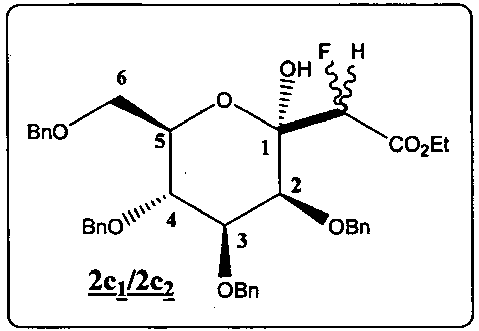

C₃₈H₄₁FO₈ M= 644,73 g.mol⁻¹

### 2c₁Diastéréoisomère majoritaire

Rf : 0,53 (cyclohexane/acétate d'éthyle 7/3).
**RMN ¹⁹F (CDCl₃, 282,5 MHz)**
-200,0 (d, J_{F-H}=47Hz)
**RMN ¹H (CDCl₃, 300MHz)**
1,1 (t, 7,2, 3H, C**H**₃); 3,5 (dd, 1,1-11,5Hz, 1H, H**6**) ; 3,7 (dd, 4,4-11,5Hz, 1H, H**6**) ; 3,9 (bs,1H, H2) ; 4,0 (m, 2H, H**5** ,H**4**) ; 4,1 (dd, 2,4-9,5Hz, 1H, H**3**) ; 4,1 (dqdt, 2,3-7,2Hz, 2H, C**H**₂) ; 4,3-4,9 (m, 8H, 40C**H**₂Ph) ; 5,0 (d, 48Hz, 1H, C**H**F) ; 7,2 (m, 20H, **H**ar).
**RMN ¹³C (CDCl₃, 75,5MHZ)**
14,4 (**C**H₃) ; 62,7 (**C**H₂) 69,6 (C**6**) ; 73,1 (O**C**H₂Ph) ; 73,9 (O**C**H₂Ph) ; 74,0 (C**5**) ; 75,1 et 75,2 (C**4** et C**2**) ; 75,4 (O**C**H₂Ph) ; 75,6 (O**C**H₂Ph) ; 81,6 (C**3**) ; 85,6 (d, 181Hz, **C**HF) ; 97,9 (d, 26Hz, C**1**); 128-128,9 (**C**ar); 138,7 ;138,8 ; 138,9; 139,0 (**C**ar. quat.) ; 169,3 (d, 23Hz, **C**O₂Et).

### 2c₂ Diastéréoisomère minoritaire

Rf= 0,44 (cyclohexane/acétate d'éthyle 8/2).
**RMN ¹⁹F (CDCl₃, 282,5 MHz)**
-210,3 (d, J_{F-H}=48Hz)
**RMN ¹H (CDCl₃, 300MHz)**
1,2 (t, 7Hz, 3H, C**H**₃); 3,7 (d, 2,9Hz, 2H, H**6**) ; 3,9 (td, 3,2-9,4Hz, 1H, H**5**) ; 4 (t, 9,2Hz, 1H, H**4**) ; 4 (m,2H, H**2**, H**3**) ; 4,1 (m, 2H, C**H**₂) ; 4,4-5 (m, 8H, 40C**H**₂Ph) ; 5,2 (d, 48Hz, 1H, C**H**F) ; 7,2 (m, 20H, **H**ar)
**RMN ¹³C (CDCl₃, 75,5MHz)**
14,5 (**C**H₃) ; 62,2 (**C**H₂) ; 69,5 (C**6**) ; 73,2 (O**C**H₂Ph) ; 73,8 (O**C**H₂Ph) ; 74,2 (C**5**) ; 74,4 (O**C**H₂Ph) ; 75,2 (C**4**) ; 75,6 (O**C**H₂Ph) ; 76 (C**2**) ; 82 (C**3**) ; 90 (d, 192Hz, **C**HF) ; 97,9 (d, 19Hz, C**1**) ; 127,7 -128,8 (**C**ar.) ; 138,7 ; 138,8 ; 138,9 ; 139,3 (**C**ar. quat.) ; 167,1 (d, 24Hz, **C**O₂Et).

### Synthèse des composés 2a₁/2a₂ et 3a₁/3a₂ (Figure 2)

A une solution de triphenylphosphine (0,5 g, 2 mmol, 4 éq.) dans THF anhydre (5 mL) placée sous atmosphère inerte est ajouté la lactone **1a** (269 mg, 0,5 mmol, 1 éq.) solubilisée dans 2 mL de THF. Le Et₂Zn (C=1,0 M dans hexanes, 2 mmol, 4 éq.) et le dibromofluoroactétate d'éthyle (0,14 mL, 1 mmol, 2 éq.) sont ensuite additionnés successivement au mélange. Le milieu est agité à température ambiante pendant trois heures, avant d'être hydrolysée par une solution aqueuse saturée en NH₄Cl, les sels sont alors filtrés sur célite et le filtrat est évaporé sous pression réduite.
Le mélange réactionnel révèle la présence de deux produits : 90% des produits **2a₁/2a₂** sous forme de deux diastéréoisomères : **2a₁** le diastéréoisomère majoritaire et 2**a₂** le diastéréoisomère minoritaire (ed : 94/6 déterminé en RMN¹⁹F) et 10% des produit **3a₁/3a₂** sous la forme de deux isomères (e.d = 60 (80-20) en RMN ¹⁹F).

Les produit **2a₁/2a₂** sont purifiés sur gel de silice avec comme éluant un mélange de cyclohexane/ acétate d'éthyle dans des proportions de huit pour deux) afin avec un rendement pondéral de 56%. Les produits **2a₁/2a₂** ont déjà été caractérisés précédemment.

Les produit **3a₁/3a₂** sont purifiés sur gel de silice avec comme éluant un mélange de cyclohexane/ acétate d'éthyle dans des proportions de neuf pour et obtenus sous forme d'un mélange de deux diastéréoisomères avec un rendement pondéral de 20 %.

### Caractérisation des composés 3a₁/3a₂

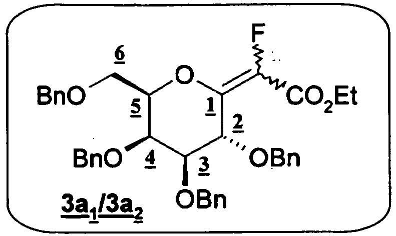

C₃₈H₃₉FO₇ M: 626,73g.mol⁻¹
Rf : 0,6 (cyclohexane/ acétate d'éthyle 8/2),
**RMN ¹⁹F CCDCl₃, 282,5Mz) :**
   **Diastéréoisomère majoritaire 3a₁ :**
      -144,2 ppm
   **Diastéréoisomère minoritaire 3a₂ :**
      -144,6 ppm
**RMN ¹H (CDCl₃, 300Mz) :**
   1,2 (2t, 0,8H, J=7,2Hz, C**H**₃); 3,8-3,6 (m, 3H, H**6** et H**4**); 3,8 (m, 1H, H**3**); 4,1 (dq, 2H, J=7,3Hz, C**H**₂); 4,3 (m, 1H, H**5**); 4,6-4,4 (m, 8H, 4 OC**H**₂Ph); 7,3 (m, 20H, **H**ar.).
**RMN ¹³C (CDCl₃, 75,5Mz) :**
   14,4 (**C**H₃**a₂**); 14,6 (**C**H₃**a₁** ; 61,8 (**C**H₂**a₁**); 63,1 (**C**H₂**a₂**); 69,2 (C**6**); 70,8 (C**2**); 71,2 (C**H**₂-Ph); 72,0 (C**H**₂-Ph); 72,5 (C**H**₂-Ph); 73,8 (C**H**₂-Ph); 70,8 (C**4**); 72,8 (C**5**); 78.4 (C**3a₁**); 79,4 (C**3a₂**); 128,8-127,8 (20 **C**ar.); 138,7-138,2 (**C**ar. quat.); 147.7 (d, *J* = 7.92 Hz, C**1**); 162.1 (d, *J* = 26.87 Hz, **C**O₂Et).

### Synthèse des composés 2b₁/2b₂ et 3b₁ (Figure 2) :

A une solution de triphenylphosphine (0,5 g, 2 mmol, 4 éq) dans THF anhydre (5 mL) placée sous atmosphère inerte est ajouté la lactone **1b** (269 mg, 0,5 mmol, 1 éq.) solubilisée dans 2 mL de THF. le Et₂Zn (C=1,0 M dans hexanes, 2 mmol, 4 éq.) et le dibromofluoroactétate d'éthyle (0,14 mL, 1 mmol, 2 éq.) sont ensuite additionnés successivement au mélange. Le milieu est agité à température ambiante pendant trois heures, avant d'être hydrolysée par une solution aqueuse saturée en NH₄Cl, les sels sont alors filtrés sur célite et le filtrat est évaporé sous pression réduite.

Le milieu brut révèle la présence de deux produits : 90% des produit **2b₁/2b₂** sous la forme de deux diastéréoisomères (ed : 91/9) et 10% du produit **3b₁** sous forme d'un seul isomère.
Les produit **2b₁/2b₂** sont purifiés sur gel de silice avec comme éluant un mélange cyclohexane/ acétate d'éthyle dans des proportions de 8,5 pour 1,5 afin d'obtenir le composé sous forme d'un mélange deux diastéréoisomères **2b₁/2b₂** avec un rendement pondéral de 58%. Dans ces conditions de réaction le composé **2b₁** est le diastéréoisomère majoritaire et le **2b₂** le diastéréoisomère minoritaire. Les produits **2b₁/2b₂** ont déjà été caractérisés précédemment.

Le produit **3b₁** est purifié sur gel de silice avec comme éluant un mélange cyclohexane/ acétate d'éthyle dans des proportions de 9,8 pour 0,2 afin d'obtenir le composé sous forme d'un seul isomère avec un rendement pondéral de 20%.

### Caractérisation du composé 3b₁

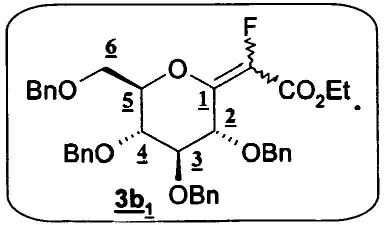

C₃₈H₃₉FO₇ M: 626,73g.mol⁻¹
Rf : 0.4 (cyclohexane/ acétate d'éthyle 8/2).
**RMN ¹⁹F (CDCl₃, 282,5Mz) :**
   -149,8 ppm
**RMN ¹H (CDCl₃, 300Mz) :**
   1,2 (t, 0,8H, J=7,2Hz, C**H**₃) ; 3,8-3,6 (m, 3H, H**6** et H**4**); 3,8 (m, 1H, H**3**); 4,1 (dq, 2H, J=7,0-1.9Hz, C**H**₂); 4,3 (m, 1H, H**5**); 4,6-4,4 (m, 8H, 4 OC**H**₂Ph); 5,6 (s, H**2**); 7,3 (m, 20H, **H**ar.).
**RMN ¹³C (CDCl₃, 75,5Mz) :**
   14,6 (**C**H₃); 61,4 (**C**H₂) ; 68,6 (C**6**); 70,7 (C**2**); 71,0 (O**C**H₂Ph); 71,5 (O**C**H₂Ph); 73,0 (O**C**H₂Ph); 73,7 (O**C**H₂Ph); 76,1 (C**5**); 78,2 (C**4**) ; 81,4 (C**3**) ; 128,8-128,1 (**C**ar.); 138,6 ; 138,2 ; 138,1; 137,6 ; 137,3 (**C**ar.q.); 137,3 (d, J=252Hz , **C**F); 147,5 ( d, J=8Hz, C**1**); 162,5 (d, J=27Hz, **C**O₂Et).

### Synthèse des composés 2c₁/2c₂ et 3c₁/3c₂ (Figure 2)

A une solution de triphenylphosphine (0,5 g ; 2 mmol ; 4 éq) dans THF anhydre (5 mL) placée sous atmosphère inerte est ajouté la lactone **1c** (269 mg ; 0,5 mmol ; 1 éq.) solubilisée dans 2 mL de THF. Le Et₂Zn (C=1,0 M dans hexanes, 2 mmol, 4 éq.) et le dibromofluoroactétate d'éthyle (0,14 mL ; 1 mmol ; 2 éq.) sont ensuite additionnés successivement au mélange. Le milieu est agité à température ambiante pendant trois heures, avant d'être hydrolysée par une solution aqueuse saturée en NH₄Cl, les sels sont alors filtrés sur célite et le filtrat est évaporé sous pression réduite.
Le milieu brut révèle la présence de deux produits : 88% des produits **2c₁l2c₂** sous la forme de deux diastéréoisomères (ed : 75/25) et 12% du produit **3c₁/3c₂** sous forme de deux isomères (78/22).

Les produit **2c₁/2c₂** sont purifiés sur gel de silice avec comme éluant un mélange cyclohexane/ acétate d'éthyle dans les proportions 8,5 pour 1,5 afin d'obtenir les composé **2c₁/2c₂** sous forme de deux diastéréoisomères avec un rendement de 66%. Cette fois ci le procédé conduit au même daistéréomère majoritaire **2c₁** et minoritaire **2c₂** que par la méthode précédente. Les produits **2c₁/2c₂** ont déjà été caractérisés précédemment.

Les produits **3c₁/3c₂** sont purifiés sur gel de silice avec comme éluant un mélange cyclohexane/ acétate d'éthyle dans des proportions de neuf pour un afin d'obtenir le composé sous forme de mélange de deux isomères (e.d =78/22 en RMN ¹⁹F) avec un rendement pondéral de 12 %.

### Caractérisation des composés 3c₁/3c₂

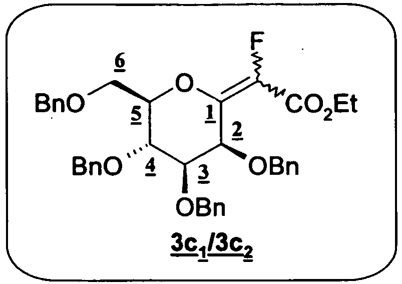

C₃₈H₃₉FO₇ M: 626,73g.mol⁻¹
Rf : 0,6 (cyclohexane/ acétate d'éthyle 8/2),
**RMN ¹⁹F (CDCl₃, 282,5Mz) :**
   **Diastéréoisomère minoritaire 3c₁ :**
      -143,9 ppm
   **Diastéréoisomère majoritaire 3c₂ :**
      -149,5 ppm
**RMN ¹H (CDCl₃, 300Mz) :**
   1,2 (t, 1H, J=7,1Hz, C**H**₃); 3,8-3,6 (m, 3H, H**6** et H**4**); 3,9 (t, J=4.2 Hz, H**3**); 4,1-4,3 (m, 2H, C**H**₂); 4,6-4,4 (m, 8H, 4 OC**H**₂Ph) ; 4.9 (td, 1H, J = 6.64, 4.47, 4.47 Hz, H**5**); 5.71 (dd, 1H, J = 3.31, 2.60 Hz, H**2**); 7,3 (m, 20H, **H**ar).
**RMN ¹³C (CDCl₃, 75,5Mz):**
   14,6 (**C**H₃); 61,7 (**C**H₂); 68,2 (C**2**); 68,8 (C**6**); 71,0 (**C**H₂-Ph); 71,6 (**C**H₂-Ph); 72,9 (**C**H₂-Ph); 73,9 (**C**H₂-Ph); 78,0 (C**4**); 78,2 (C**5**); 79.9 (d, J = 3.6 Hz, C**3**); 128,8-128,0 (20 **C**ar.); 138,4; 138,3; 138,2; 137,7 (4 **C**ar.quat.); 136.47 (d, J = 254.24 Hz, **C**F); 147.89 (d, J = 7.92 Hz, C**1**); 162.43 (d, J = 26.87 Hz, **C**O₂Et).

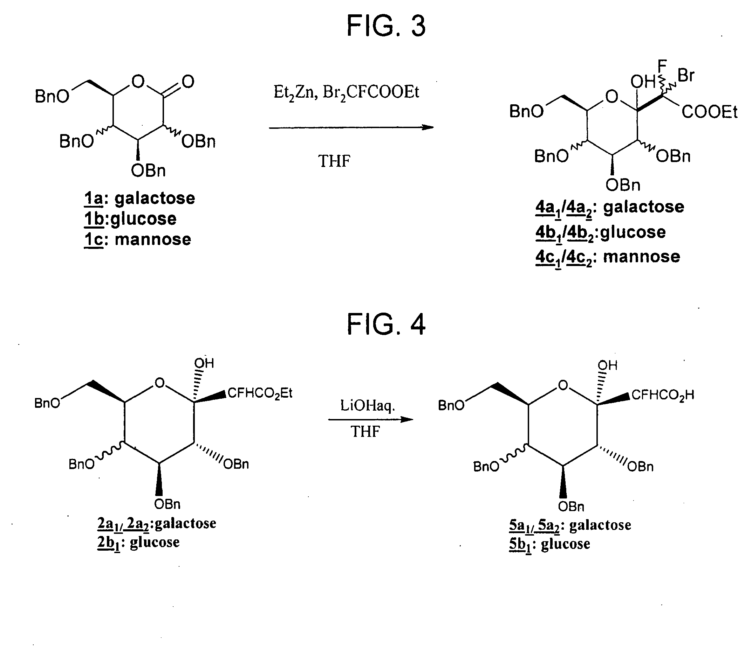

### Synthèse des composés 4a₁/4a₂ (Figure 3):

Dans un ballon sous atmosphère inerte contenant la lactone **1a** (269 mg, 0,5 mmol, 1 éq.) en solution dans le THF (5 mL), le diéthylzinc Et₂Zn (C=1,0 M dans hexanes, 1 mmol, 2 éq.) et le dibromofluoroactétate d'éthyle (0,14 mL, 1 mmol, 2 éq.) sont additionnés successivement au mélange. La solution est ensuite agité à température ambiante pendant trois heures avant d'être hydrolysée par de l'Ethanol, et évaporé sous pression réduite.

Le mélange est ensuite purifié sur gel de silice avec comme éluant un mélange cyclohexane/ acétate d'éthyle dans des proportions de 9,5 pour 0,5 afin d'obtenir les composés **4a₁/4a₂** sous forme d'un mélange de deux diastéréoisomères (ed = 50 (75-25) en RMN ¹⁹F) avec un rendement de 62 %. 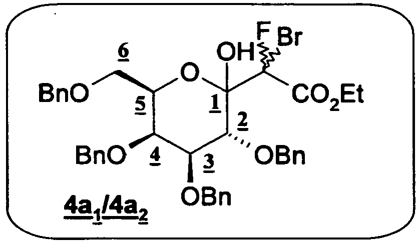 C₃₈H₄₀FBrO₈ M=723,74 g,mol⁻¹
Rf : 0,4 (cyclohexane/ acétate d'éthyle 8/2),
**RMN ¹⁹F (CDCl₃, 282,5Mz) :**
   **Diastéréoisomère majoritaire** **4**a₁ :
      -126,5 ppm
   **Diastéréoisomère minoritaire 4a₂ :**
      -126,7 ppm
         **RMN ¹H (CDCl₃, 300Mz) :**
         1,1 (t, 0,8H, J=7,2Hz, C**H3a₂**); 2,2 (t, 1,2H, J=7,2Hz, C**H3a₁**); 3,6-3,5 (dd, 1H, J=7-12Hz, H**6**); 3,9-3,7 (dd, 1H, J=7,3Hz, H**6**); 4,0 (s, 1H, H**4**); 4,0 (m, 1H, H**3**); 4,0 (m, 1H, H**5**); 4,1 (q, 2H, J=7,3Hz, C**H**₂); 4,2 (m, 1H, H**2**); 4,3-4,8 (m, 8H, 4 OC**H**₂Ph); 7,3 (m, 20H, **H**ar.).
**RMN ¹³C (CDCl₃, 75,5Mz) :**
   **Diastéréoisomère majoritaire 4a₁** :
      14,2 (**C**H₃); 64,0 (**C**H₂) ; 68,9 (C**6**); 72,4 (C**5**); 73,5 (O**C**H₂Ph); 73,8 (O**C**H₂Ph); 74,4 (C**4**); 74,9 (O**C**H₂Ph); 75,5 (C**2**); 75,7 (O**C**H₂Ph) ; 81,4 (C**3**) ; 98,0 (d, J=274Hz, **C**F) ; 98,5 (d, J=25Hz, C**1**) ; 127-129 (**C**ar.); 139-138 (**C**ar.quat.); 166,4 (d, J=26Hz, **C**O₂Et).
   **Diastéréoisomère minoritaire 4a₂:**
      13,9 (**C**H₃); 63,8 (**C**H₂); 68,7 (C**6**); 72,2 (C**5**); 72,8 (O**C**H₂Ph); 74,0 (O**C**H₂Ph); 74,8 (O**C**H₂Ph); 75,0 (C**4**); 75,1 (O**C**H₂Ph); 75,5 (C**2**) ; 81,9 (C**3**) ; 99,0 (d, J=295Hz, **C**F) ; 98,0 (d, J=23Hz, C**1**) ; 127-129 (**C**ar.); 139-138 (**C**ar.quat.); 165,6 (d, J=26Hz, **C**O₂Et).

### Synthèse des composés 4b₁/4b₂ (Figure 3) :

Dans un ballon sous atmosphère inerte contenant la lactone **1b** (269 mg, 0,5 mmol, 1 éq.) en solution dans le THF (5mL), le Et₂Zn (C=1,0 M dans hexanes, 1 mmol, 2 éq.) et le dibromofluoroactétate d'éthyle (0,14 mL, 1 mmol, 2 éq.) sont additionnés successivement au mélange. La solution est ensuite agitée à température ambiante pendant trois heures avant d'être hydrolysée par de l'éthanol, et évaporé sous pression réduite.

Le mélange est ensuite purifié sur gel de silice avec comme éluant un mélange cyclohexane/ acétate d'éthyle dans des proportions de 9,5 pour 0,5 afin d'obtenir les composés **4b₁/4b₂** sous forme d'un mélange de deux diastéréoisomères (e.d = (92-8) en RMN ¹⁹F) avec un rendement de 41 %.

### Caractérisation des composés 4b₁/4b₂

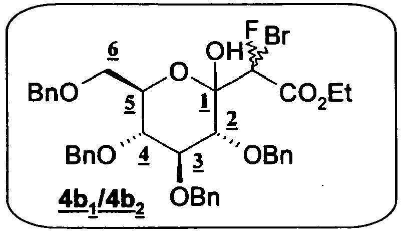

C₃₈H₄₀FBrO₈ M=723,74 g, mol⁻¹
Rf : 0,37 (cyclohexane/ acétate d'éthyle 8/2),
**RMN ¹⁹F (CDCl₃, 282,5Mz) :**
   **Diastéréoisomère minoritaire 4b₁ :**
      -127,2 ppm
   **Diastéréoisomère majoritaire 4b₂ :**
      -127,8 ppm
**RMN ¹H (CDCl₃, 300Mz) :**
   1,1 (t, 0,8H, J=7,2Hz, C**H₃b₁**) ; 2,2 (t, 1,2H, J=7,2Hz, , C**H₃b₂**); 3,6-3,5 (dd, 1H, J=7-12Hz, H**6**); 3,9-3,7 (dd, 1H, J=7,3Hz, H**6**); 4,0 (s, 1H, H**4**); 4,0 (m, 1H, H**3**); 4,0 (m, 1H, H**5**); 4,1 (q, 2H, J=7,3Hz, C**H**₂); 4,2 (m, 1H, H**2**); 4,3-4,8 (m, 8H, 4 OC**H**₂Ph); 7,3 (m, 20H, **H**ar.).
**RMN ¹³C (CDCl₃, 75,5Mz):**
   **Diastéréoisomère minoritaire 4b₁ :**
      13,9 (**C**H₃); 63,8 (**C**H₂) ; 68,7 (C**6**); 72,2 (C**5**); 72,8 (O**C**H₂Ph); 74,0 (O**C**H₂Ph); 74,8 (O**C**H₂Ph); 75,0 (C**4**); 75,1 (O**C**H₂Ph); 75,5 (C**2**) ; 81,9 (C**3**) ; 99,0 (d, J=295Hz, **C**F) ; 98,0 (d, J=23Hz, C**1**) ; 127-129 (**C**ar.); 139-138 (**C**ar.quat.); 165,6 (d, J=26Hz, **C**O₂Et).
   **Diastéréoisomère majoritaire 4b₂:**
      14,2 (**C**H₃); 64,0 (**C**H₂) ; 68,9 (C**6**); 72,4 (C**5**); 73,5 (O**C**H₂Ph); 73,8 (O**C**H₂Ph); 74,4 (C**4**); 74,9 (O**C**H2Ph); 75,5 (C**2**); 75,7 (O**C**H₂Ph) ; 81,4 (C**3**) ; 98,0 (d, J=274Hz, **C**F ; 98,5 (d, J=25Hz, C**1**) ; 127-129 (**C**ar.); 139-138 (**C**ar.quat.); 166,4 (d, J=26Hz, **C**O₂Et).

### Synthèse des composés 4c₁/4c₂ (Figure 3)

Dans un ballon sous atmosphère inerte contenant la lactone **1c** (269 mg ; 0,5 mmol ; 1 éq.) en solution dans le THF (5 mL), le Et₂Zn (C=1,0 M dans hexanes, 1 mmol, 2 éq.) et le dibromofluoroactétate d'éthyle (0,14 mL ; 1 mmol ; 2 éq.) sont additionnés successivement au mélange. La solution est ensuite agité à température ambiante pendant trois heures avant d'être hydrolysée par de l'éthanol, et évaporé sous pression réduite.

Le mélange est ensuite purifié sur gel de silice avec comme éluant le mélange cyclohexane/ acétate d'éthyle dans des proportions de 9,5 pour 0,5 afin d'obtenir le composé sous forme de mélange de deux diastéréoisomères (ed = (42-58) en RMN ¹⁹F) **4c₁/4c₂** avec un rendement pondéral de 43 %.

### Caractérisation des composés 4c₁/4c₂

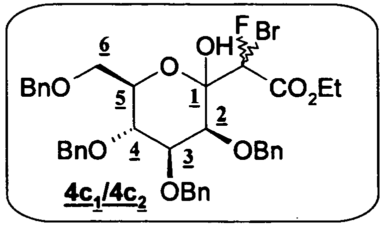

C₃₈H₄₀FBrO₈ M=723,74 g,mol⁻¹
Rf : 0,34 (cyclohexane/ acétate d'éthyle 8/2),
**RMN ¹⁹F (CDCl₃, 282,5Mz) :**
   **Diastéréoisomère minoritaire 4c₁ :**
      -120,3 ppm
   **Diastéréoisomère majoritaire 4c₂ :**
      -124,9 ppm
**RMN ¹H (CDCl₃, 300Mz) :**
   0,9 (t, 1H, J=7,1Hz, C**H₃c₂**) ; 1,1 (t, 1,2H, J=7,1Hz, C**H₃c₁**); 3,6-3,5 (dd, 1H, J=7-12Hz, H**6**); 3,9-3,7 (dd, 1H, J=7,3Hz, H**6**); 4,0 (s, 1H, H**4**); 4,0 (m, 1H, H**3**); 4,0 (m, 1H, H**5**); 4,1 (q, 2H, J=7,3Hz, C**H**₂); 4,2 (m, 1H, H**2**); 4,3-4,9 (m, 8H, 4 OC**H**₂Ph); 7,3-7,1 (m, 20H, **H**ar).
**RMN ¹³C (CDCl₃, 75,5Mz):**
   **Diastéréoisomère minoritaire 4c₁ :**
      14,1 (**C**H₃); 64,0 (**C**H₂) ; 69,2 (**C**H₂); 73,4 (O**C**H₂Ph); 74,0 (O**C**H₂Ph); 74,6 (C**5**); 74,8 (O**C**H₂Ph); 75,1 (C**4**); 75,6 (O**C**H₂Ph); 76,7 (C**2**) ; 82,2 (C**3**) ; 98,2 (d, J=20Hz, C**1**); 103,2 (d, J=282Hz, **C**F) ; 128,9-127,8 (**C**ar.); 139,1-138,6 (**C**ar.quat.); 165,0 (d, J=25Hz, **C**O₂Et).
   **Diastéréoisomère majoritaire 4c₂ :**
      14,0 (**C**H₃); 63,6 (**C**H₂) ; 69,5 (C**6**); 73,3 (O**C**H₂Ph); 73,8 (O**C**H₂Ph); 74,8 (O**C**H₂Ph); 74,9 (C**5**); 75,0 (C**2**); 75,6 (O**C**H₂Ph) ; 82,0 (C**3**) ; 98,9 (d, J=25Hz, C**1**); 100,6 (d, J=272Hz, **C**F) ; 127-129 (**C**ar.); 139-138 (**C**ar.quat.); 165,7 (d, J=27Hz, **C**O₂Et).

### Synthèse des composés 5a₁/5a₂ (Figure 4)

Dans un ballon contenant l'ester **2a₁/2a₂** (500mg; 0,776mmol; 1éq.) en solution dans le THF (5mL), on additionne une solution constituée de LiOH (37mg; 1,55mmol ; 2 éq.) solubilisée dans le minimum d'eau. La réaction est maintenue sous agitation toute la nuit. Une solution d'HCl 1M est ajoutée et le milieu est extrait trois fois à l'acétate d'éthyle. Les phases organiques sont rassemblées, séchées sur sulfate de magnésium, filtrées puis évaporées pour obtenir le produit attendu sous la forme d'une huile jaune avec un rendement de 96%.

### Caractérisation des composés 5a₁/5a₂

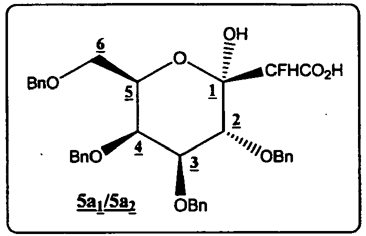

C₃₆H₃₇FO₈ M= 616,67 g.mol⁻¹
**RMN ¹⁹F (CDCl₃, 282,5MHz)**
**-197,9 (d, J_{F-H}=49 Hz, 1F):5a₁**
-202,7 (d, J_{F-H}=47 Hz,1F):**5a₂**
**RMN ¹H (CDCl₃, 300MHz)**
3,4 (dd, 2,2-6,2 2H, H**6**) ; 3,8 (s, 1H, H**4**) ; 3,9 (dd, 2,4 et10 Hz, 1H, H**3**); 4,1 (tapp., 6,1Hz, 1H, H**5**) ; 4,2 (dd, 2,8 et Hz, 1H, H**2**) ; 4,3 (d, 11,9Hz, 1H, OC**H**₂Ph) ; 4,4 (d, 12Hz, 1H, OC**H**₂Ph) ; 4,5 (d, 11,7Hz, 1H, OC**H**₂Ph) ; 4,6 (d, 11,1Hz, 1H, OC**H**₂Ph) ; 4,7 (s, 2H, OC**H**₂Ph) ; 4,8 (d, 12Hz, 1H, OC**H**₂Ph) ; 4,9 (d, 11,2Hz, 1H, OC**H**₂Ph) ; 4,9 (d, 47Hz, 1H, C**H**F) ; 7,2 (m, 20H, **H**ar).
**RMN ¹³C (CDCl₃, 75,5MHZ)**
69,2 (C**6**) ; 71,5 (C**5**) ; 73,2 (O**C**H₂Ph) ; 73,7 (O**C**H₂Ph) ; 74,8 (O**C**H₂Ph) ; 74,9 (C**4**) ; 75,2 (C**2**) ; 76,2 (O**C**H₂Ph) ; 80,6 (C**3**) ; 86,0 (d, 200Hz, **C**HF) ; 98,4 (d, 21Hz, C**1**) ; 128,0 -128,9 (**C**ar.) ; 137,9 ;138,3 ; 138,7 ; 139,0 (**C**ar. quat.) ; 171,0 (d, 24Hz, **C**O₂H).

### Synthèse du composé 5b₁ (Fig.4)

Dans un ballon sous atmosphère inerte contenant l'ester **2b₁** (275 mg ; 0.43 mmol ; 1 éq.) en solution dans l'éthanol (7 mL), une solution aqueuse de lithine (2M ; 2 éq.) est ajoutée et le mélange est agité pendant la nuit à température ambiante. Le milieu est concentré et dissout dans le DCM (5 mL), il est ensuite acidifié avec une solution d'HCl 1M (20 mL). Le mélange est extrait avec du DCM (3 x 20 mL), et les phases organiques sont combinées, lavées avec une solution saturée de NaCl et concentrées directement. L'acide **5b₁** est isolé ainsi comme un solide jaune qui peut être utilisée directement pour la prochaine étape sans purifications supplémentaires avec un rendement brut de 92%.

### Caractérisation du composé 5b₁

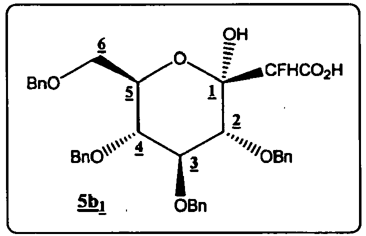

**RMN ¹⁹F (CDCl₃, 282MHz)**
-197,9 (d, ²J_{F-H} 46.1).
**RMN ¹H (CDCl₃, 300MHz)**
3.42-3.71 (m, 4H), 3.94-4.09 (m, 2H), 4.29-4.91 (m, 9H), 7.03-7.28 (m, 20H, H_{Ar}).

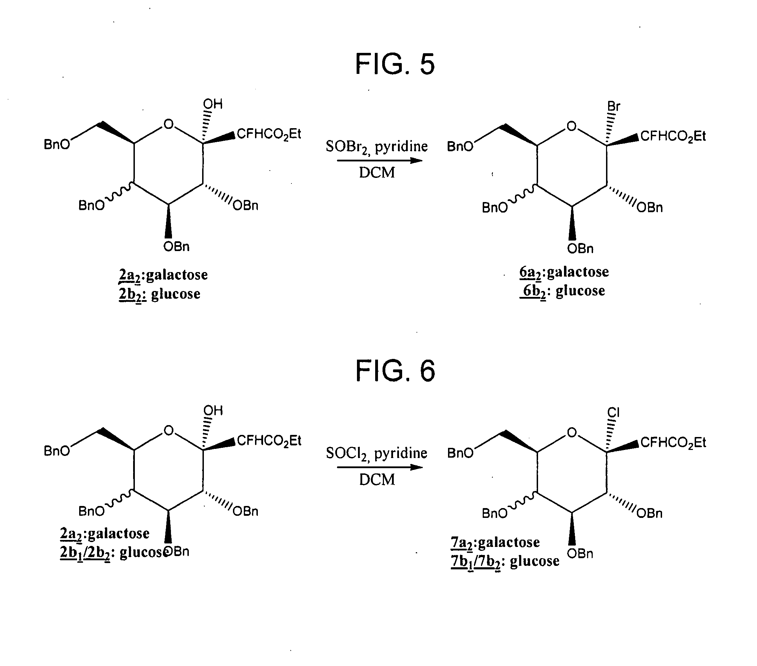

### Synthèse du composés 6a₂ (Figure 5)

Dans un ballon sous atmosphère inerte contenant le monofluoroester **2a₂** (230 mg ; 0,36 mmol : 1 éq.) en solution dans le dichlorométhane anhydre (3 mL) à -30°C, on additionne goutte à goutte le SOBr₂ (41 µL ; 0,535 mmol ; 1,5 éq.). Après 30 minutes, on introduit la pyridine (42 µL; 0,535 mmol ; 1,5 éq.). et on laisse sous agitation 30 minutes de plus à -30°C. Une solution d'HCl 2M est ajoutée et la phase est extraite trois fois avec du dichlorométhane. Les phases organiques sont rassemblées, séchées sur MgSO₄, filtrées et concentrées sous pression réduite. Le produit brut est obtenu sous forme d'une huile jaune claire avec un rendement pondéral de 80%.

### Caractérisation du composé 6a₂

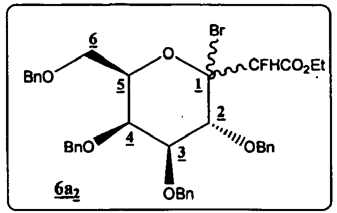

C₃₈H₄₀BrFO₇ M= 706,62 g.mol⁻¹
**RMN ¹⁹F (CDCl₃, 282MHz)**
-188,0 (d, 45Hz).
**RMN ¹H (CDCl₃, 300MHz)**
0,97 (t, 7,1Hz, 3H, C**H**₃); 3,51 (dd, 5,4 et 9,1Hz, 1H, 1H**6**) ; 3,60 (tapp, 8,6Hz, 1H, 1H**6**) ; 3,91-4,1 (m, 5H, H**4**, H**3**, C**H**₂, H**5**); 4,22 (d, 9,4Hz, 1H, H**2**) ; 4,37 (d, 11,8Hz, 1H, OC**H**₂Ph); 4,43 (d, 11,8Hz, 1H, OC**H**₂Ph); 4,45 (d, 11Hz, 1H, OC**H**₂Ph) ; 4,63 (d, 11,5Hz, 1H, OC**H**₂Ph) ; 4,68 (d, 11,8Hz, 1H, OC**H**₂Ph) ; 4,78 (d, 11,6Hz, 1H, OC**H**₂Ph); 4,86 (d, 11 Hz, 1H, OC**H**₂Ph) ; 5,01 (d, 11,5Hz, 1H, OC**H**₂Ph) ; 5,07 (d, 46Hz, 1H, C**H**F); 7,2 (m, 20H, **H**ar)
**RMN ¹³C (CDCl₃,75,5MHz)**
14,2 (**C**H₃) ; 62,7 (**C**H₂) ; 67,4 (C**6**) ; 73,0 (OC**H**₂Ph) ; 73,6 (C**4**) ; 74,0 (OC**H**₂Ph) ; 75,0 (OC**H**₂Ph) ; 75,3 (OC**H**₂Ph) ; 76,2 (C**2**) ; 76,4 (C**5**) ; 82,3 (C**3**) ; 89,5 (d, 199Hz, **C**HF); 106,9 (d, 18 Hz, C**1**); 127,5 -128,9 (**C**ar.) ; 138,0; 138,3; 138,7; 138,9 (**C**ar. quat.) ; 164,9 (d, 27Hz, **C**O₂Et).

### Synthèse du composé 6b₂ (Figure 5)

Dans un ballon sous atmosphère inerte contenant le monofluoroester **2b₂** (500 mg ; 0,775 mmol : 1 éq.) en solution dans le dichlorométhane anhydre (6 mL) à -30°C, on additionne goutte à goutte le SOBr₂ (88 µL; 1,13 mmol ; 1,5 éq.). Après 30 minutes, on introduit la pyridine (92 µL ; 1,13 mmol ; 1,5 éq.) et on laisse sous agitation 30 minutes de plus à -30°C. Une solution d'HCl 2M est ajoutée et la phase est extraite trois fois avec du dichlorométhane. Les phases organiques sont rassemblées, séchées sur MgSO₄, filtrées et concentrées sous pression réduite.

Le produit **6b₂** brut est obtenu sous forme d'une huile marron avec un rendement pondéral de 85%.

### Caractérisation du composé 6b₂

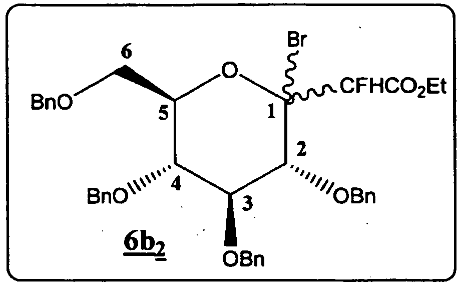

C₃₈H₄₀BrFO₇ M= 706,62 g.mol⁻¹
**RMN ¹⁹F (CDCl₃, 282MHz)**
-188,09 (d, 45Hz).
**RMN ¹H (CDCl₃, 300MHz)**
1,1 (t, 7,1Hz, 3H, C**H**₃); 3,6 (dd,1,7-11,5Hz, 1H, 1H**6**); 3,7 (m, 2H, H**2**, 1H**6**); 3,8 (dd , 9Hz, 1H, H**4**); 3,9 (td, 2,2-10,1 Hz, 1H, H**5**) ; 4,1 (qdt, 7,1 Hz, 2H, C**H**₂) ; 4,1 (dd, 9,2, 1H, H**3**) ; 4,4-5 (m, 8H, 40C**H**₂Ph) ; 5,06 (d, 46Hz, 1H, C**H**F) ; 7,2 (m, 20H, **H**ar)
**RMN ¹³C (CDCl₃,75,5MHz)**
   14,4 (**C**H₃) ; 62,8 (**C**H₂) ; 67,7 (C**6**) ; 73,7 (O**C**H₂Ph) ; 75 (O**C**H₂Ph) ; 75,7 (O**C**H₂Ph) ; 76,2 (O**C**H₂Ph) ; 76,6 (C**2**) ; 78,1 (C**5**) ; 79,9 (C**4**) ; 84,7 (C**3**) ; 89,5 (d, 203 Hz, **C**HF) ; 105,7 (d, 18 Hz, C**1**); 127,5 -128,9 (**C**ar.) ; 138,2 ;138,4 ; 138,5 (**C**ar. quat.) ; 165,4 (d, 26Hz, **C**O₂Et).

La même réaction conduite dans les mêmes conditions sur le composé **2b₁** conduit au composé **6b₁.**
**RMN ¹⁹F (CDCl₃ 282MHz)**
-182,2 (d, 47Hz).

### Synthèse du composé 7a₂ (Figure 6)

Dans un ballon sous atmosphère inerte contenant le monofluoroester **2a₂** (95 mg ; 0,147 mmol : 1 éq.) en solution dans le dichlorométhane anhydre (2mL) à -30°C, on additionne goutte à goutte le chlorure de thionyle SOCl₂ (16 µL ; 0,221 mmol ; 1,5 éq.). Après 30 minutes, on introduit la pyridine (17 µL; 0,221 mmol ; 1,5 éq.). et on laisse sous agitation 30 minutes de plus toujours à -30°C. Une solution d'HCl 2M est ajoutée et la phase est extraite trois fois avec du dichlorométhane. Les phases organiques sont rassemblées, séchées sur MgSO₄, filtrées et concentrées sous pression réduite. Le produit brut est obtenu sous forme d'une huile jaune claire avec un rendement pondéral de 83%.

### Caractérisation du composé 7a₂

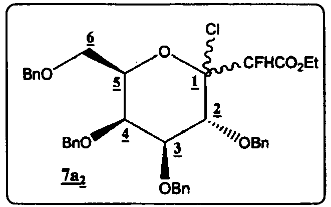

C₃₈H₄₀ClFO₇ M= 663,17 g.mol⁻¹
**RMN ¹⁹F (CDCl₃, 282MHz)**
-194,9 (d, 1F, 46Hz)
**RMN ¹H (CDCl₃, 300MHz)**
0,98 (t, 7,1Hz, 3H, C**H**₃); 3,50 (dd, 5,4-9,1, 1H, H**6**) ; 3,58 (tapp., 8,6Hz, 1H, H**6**) ; 3,92-4,09 (m, 4H, H**4** H**3**, C**H**₂); 4,16 (m, 1H, H**5**) ; 4,37 (d, 11,8Hz, 1H, OC**H**₂Ph) ; 4,43 (d, 11,4Hz, 1H, OC**H**₂Ph) ; 4,46 (d, 10,9Hz, 1H, OC**H**₂Ph) ; 4,48 (d, 9,3Hz, 1H, H**2**); 4,63 (d, 11,4Hz, 1H, OC**H**₂Ph) ; 4,68 (d, 11,4Hz, 1H, OC**H**₂Ph) ; 4,75 (d, 11,5Hz, 1H, OC**H**₂Ph); 4,86 (d, 11,1Hz, 1H, OC**H**₂Ph) ; 4,99 (d, 11,4Hz, 1H, OC**H**₂Ph); 5,02 (d, 46Hz, 1H, C**H**F); 7,23 (m, 20H, **H**ar)
**RMN ¹³C (CDCl₃,75,5MHz)**
14,2 (**C**H₃) ; 62,6 (**C**H₂) ; 67,6 (C**6**) ; 73,0 (O**C**H₂Ph) ; 73,8 ; 74,0 (O**C**H₂Ph) 74,4 ; 75,2 (2O**C**H₂Ph) 76,0 ; 81,3 ; 89,2 (d, 200Hz ; **C**FH) ; 127,7-128,9 (**C**ar.) ; 138,3 ; 138,6 ; 139,0 (**C**ar. quat.) ; 161,4 et 161,9 (2t, 32 Hz, **C**O₂Et).

### Synthèse du composé 7b₁/7b₂, (Figure 6)

Le mélange des deux diastéréomères de l'ester **2b₁/2b₂** (500 mg ; 0.77 mmol ; 1 éq.) est placé dans le dichlorométhane (20 mL) et additionné l'halogénure de thionyl (138 mg ; 1.16 mmol ; 1.5 éq.) à -30°C. Après 30 min à -30°C, la pyridine (92 mg ; 1.16 mmol ; 1.5 éq.) est ajoutée et la solution est agitée 30 min supplémentaires. La solution est hydrolysée avec HCl 2N (20 mL) puis extraite avec du dichlorométhane (3 x 20 mL). Les phases organiques sont lavées avec une solution saturée de chlorure de sodium (30 mL) puis séchées sur sulfate de sodium et concentrées. Le brut réactionnel est alors purifié par colonne chromatographique sur gel de silice avec un éluant cyclohexane / acétate d'éthyle (80 : 20).

Les 2 diatéréomères **2b₁/2b₂** sont isolés sous forme d'une huile incolore avec un rendement de 78%.

### Caractérisation du composé 7b₁/7b₂

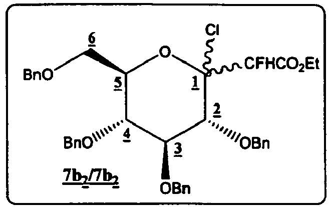

C₃₈H₄₀ClFO₇ M= 663,17 g.mol⁻¹
Rf= 0.50, éluant : cyclohexane / acétate d'éthyle (8 : 2).
**RMN ¹⁹F (CDCl₃, 282MHz)**
-186.4 (1F, d, ²J_{F-H7} 47.2) **7b₁**
-195.0 (1F, d, ²J_{F-H7} 46.1) **7b₂**
**RMN ¹H (CDCl₃, 300MHz)**
1.10 (t, 3H, ³J_{H10-H10} 7.2, C**H₃** ), 1.11-1.21 (m, 3H, C**H₃**), 3.55-3.78 (m, 3H, H**6**), 3.91-4.08 (m, 3H), 4.19 (q, 2H, ³J_{H9-H10} 7.2, C**H₃**), 4.39-4.57 (m, 4H), 4.67-5.20 (m, 5H, H7), 7.20-7.25 (20H_{Ar}).
**RMN ¹³C (CDCl₃, 75MHz)**
14.4 (**C**H₃), 14.5 (**C**H₃), 62.4 (**C**H₃), 62.8 (**C**H₃), 67.9 (C**6**), 68.2 (C**6**), 73.7, 73.9, 74.7, 75.4, 76.3, 79.3, 79.6, 83.0, 83.5, 88.9 (d, ¹J_{C7-F} 201.0, **C**F), 89.2 (d, ¹J_{C7-F} 195.9, **C**F), 103.0 (d, ²J_{C1-F} 22.8, C**1**), 105.0 (d, ²J_{C1-F} 18.3, C**1**), 127.3, 127.7, 127.7, 127.8, 127.8, 127.8, 127.9, 127.9, 128.0, 128.0, 128.1, 128.1, 128.3, 128.4, 128.5, 128.5, 128.6, 137.8, 137.9, 138.0, 138.1, 138.2, 138.3, 138.3, 138.3, 165.1 (d, ²J_{C8-F} 33.1, **C**O₂Et), 165.5 (d, ²J_{C8-F} 33.1, **C**O₂Et).

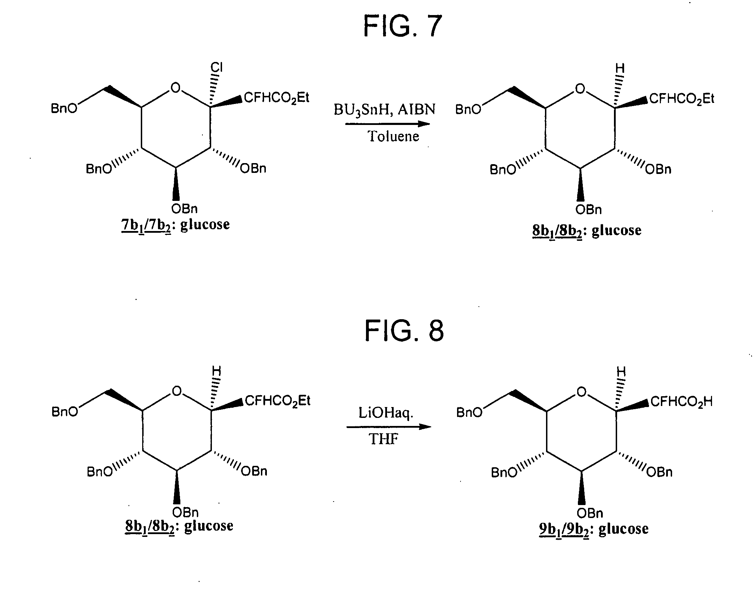

### Synthèse du composé 8b₁/8b₂ (Figure 7)

Le produit chloré **7b₁/7b₂** (240 mg ; 0.36 mmol ; 1.0 éq.) est placé avec le tributyl étain (422 mg ; 1.50 mmol ; 4.0 éq.) dans du toluène sec (20 mL) et la solution est porté à reflux pendant quatre heures. Après retour à la température ambiante, le milieu est concentré et purifié par colonne chromatographique sur gel de silice avec un éluant cyclohexane / acétate d'éthyle (80 : 20). Le produit est isolé avec 63% de rendement.

La réaction peut être conduite dans les mêmes conditions à partir des dérivés bromés **6b₁/6b₂** pour conduire aux mêmes composés **8b₁/8b₂**

### Caractérisation du composé 8b₁/8b₂

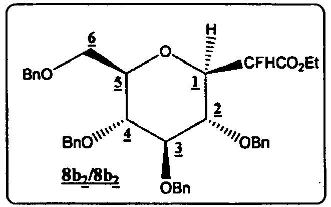

C₃₈H₄₁FO₇ M= 628 g.mol⁻¹
Rf = 0.43, éluant : cyclohexane / acétate d'éthyle (8 : 2).
**RMN ¹⁹F (CDCl₃, 282MHz)**
-203.1 (1F, dd, ²J_{F-H7} 48.7, ³J_{F-H1} 23.6) **8b₂**.
-208.4 (1F, dd, ²J_{F-H7} 48.0, ³J_{F-H1} 31.1) **8b₁**.
**RMN ¹H (CDCl₃, 300MHz)**
1.03 (t, 3H, ³J_{H10-H9} 7.2, C**H₃**), 1.15 (t, 3H, ³J_{H10-H9} 6.7, C**H₃**), 3.50-3.72 (m, 7H, H**2**, H**3**, H**4**, H**5**, 2H**6** ), 3.88 (qₐₚₚ, 2H, ³J_{H9-H10} 6.7, C**H₂**), 4.15 (dd, 2H, ³J_{H9-H10} 7.2, 3.2, C**H₂**), 4.45-4.64 (m, 4H), 4.72-4.88 (m, 5H), 5.08 (d, 1H, ²J_{H7-F} 48.0, CF**H**), 5.08 (d, 1H, ²J_{H7-F} 48.7, CF**H**), 7.10-7.28 (m, 20H, **H**_{Ar}).
**RMN ¹³C (CDCl₃, 75MHz)**
14.4 (**C**H₃), 14.6 (**C**10), 61.6 (**C**H₃), 61.8 (**C**H₂), 68.9 (C**6**), 73.8, 74.0, 75.0, 75.6, 75.7, 76.1, 76.8 (d, J_{C-F} 7.0), 77.5 (d, J_{C-F} 4.0), 78.6, 78.8, 79.4, 79.6, 79.9, 80.5, 87.0 (d, ¹J_{C7-F} 191.8, **C**F), 87.5 (d, ²J_{C1-F} 22.1, C**1**), 88.6 (d, ¹J_{C7-F} 190.8, **C**F), 127.6, 127.6, 127.7, 127.8, 127.8, 127.9, 127.9, 128.0, 128.0, 128.1, 128.2, 128.2, 128.3, 128.4, 128.5, 128.6, 128.6, 128.7, 137.9, 138.0, 138.2, 138.2, 138.3, 167.0 (d, ²J_{C8-F} 24.6, **C**O₂Et), 167.7 (d, ²J_{C8-F} 24.6, **C**O₂Et).

### Synthèse du composé 9b₁/9b₂ (Figure 8)

Dans un ballon sous atmosphère inerte contenant l'ester **8b₁/8b₂** (140 mg ; 0.223 mmol ; 1 éq.) en solution dans l'éthanol (12 mL), une solution aqueuse de lithine (2M ; 2 éq.) est ajoutée et le mélange est agité pendant la nuit à température ambiante. Le milieu est concentré et dissout dans le DCM (15 mL), il est ensuite acidifié avec une solution d'HCl 2M (20 mL). Le mélange est extrait avec du DCM (3 x 20 mL), et les phases organiques sont combinées, lavées avec une solution saturée de NaCl (30 mL) et concentrées directement. L'acide **9b₁/9b₂** est isolé ainsi comme une huile incolore avec un rendement brut de 99%.

### Caractérisation du composé 9b₁/9b₂

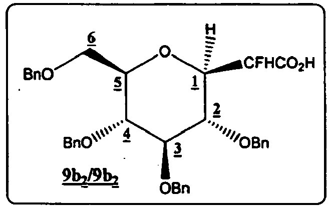

C₃₆H₃₇FO₇ M= 600 g.mol⁻¹
**RMN ¹⁹F (CDCl₃, 282MHz)**
-202.1 (1F, dd, ²J_{F-H7} 45.1, ³J_{F-H1} 19.3) **9b₂**.
-207.2 (1F, dd, ²J_{F-H7} 46.1, ³J_{F-H1} 30.0) **9b₁**.
**RMN ¹H (CDCl₃, 300MHz)**
3.46-3.72 (m, 7H, H**1**, H**2**, H**3**, H**4**, H**5**, 2H**6**), 4.43-4.50 (m, 2H), 4.54-4.87 (m, 6H), 5.02 (d, ²J_{H7-F} 47.8, CF**H**), 5.06 (d, ²J_{H7-F} 48.5, CF**H** ), 7.09-7.31 (20**H**_{Ar}).
**RMN ¹³C (CDCl₃, 75MHz)**
68.6, 68.8, 73.3, 73.6, 74.8, 75.3, 75.4, 75.8, 76.4, 76.5, 77.5, 77.5, 78.2, 78.7, 79.0, 79.5, 79.7, 86.3 (d, ¹J_{C7-F} 191.0, **C**F), 86.6 (d, ²J_{C1-F} 18.8, C**1**), 87.7 (d, ¹J_{C7-F} 191.9, C7), 127.5, 127.6, 127.8, 127.8, 127.9, 128.0, 128.1, 128.1, 128.2, 128.2, 128.4, 128.5, 128.6, 128.6, 128.7, 137.8, 137.9, 138.3, 138.4, 171.5 (d, ²J_{C8-F} 24.6, **C**O₂Et).

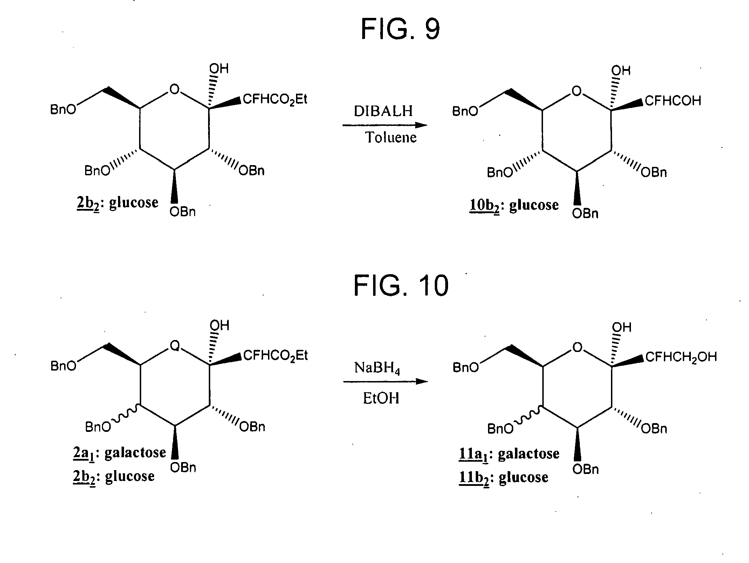

### Synthèse des composés 10b₂ (Figure 9)

Dans un ballon sous atmosphère inerte contenant l'ester **2b₂** (290mg ; 0,45 mmol ; 1 éq.) en solution dans le toluène anhydre (5 mL) à -78°C, une solution de DIBAL-H 1M dans le toluène est ajouté (1,35 mL ; 1,35 mmol ; 3 éq.). Le milieu est laissé sous agitation à - 78°C pendant 2h30mn, puis une solution de DIBAL-H est à nouveau ajoutée (450 µL ; 0,45 mmol ; 1 éq.). Le milieu est encore agité pendant 30 mn à -78°C puis l'éthanol est additionné (2mL). La solution est remontée à -20°C pendant 10mn, et une solution de sels de Rochelles à 20% (40mL) est ajoutée. Le mélange est laissé sous forte agitation pendant plusieurs heures. L'acétate d'éthyle est ajouté (20mL),puis après décantation, la phase aqueuse est extraite deux fois à l'acétate d'éthyle (2*15mL).Puis les phases organiques sont rassemblées et lavées deux fois avec une solution aqueuse saturée de NaCl (2*15mL), séchées sur sulfate de magnésium, filtrées puis évaporées.

### Caractérisation de 10b₂

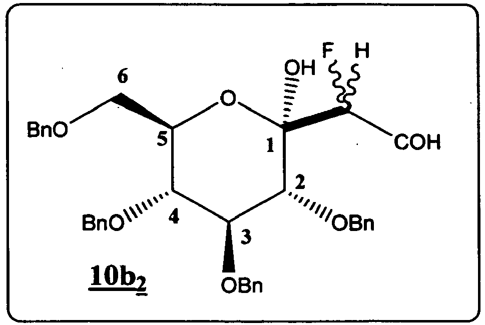

C₃₆H₃₇FO₇ M= 600,67 g.mol⁻¹
**RMN ¹⁹F (CDCl₃, 282,5 MHz)**
-211,4 (dd, J_{F-H}=48Hz et 8Hz)
**RMN ¹H (CDCl₃, 300MHz)**
3,57-3,62 (m, 4H, H**6**, H**2**, H**4**); 3,96 (m, 1H, H**5**) ; 3,97 (t, 9,1Hz, 1H, H**3**); 4,44 (d, 12,3Hz, 1H, OC**H**₂Ph); 4,48 (d, 12,3Hz, 1H, OC**H**₂Ph) ; 4,50 (d, 11,8Hz, 1H, OC**H**₂Ph) ; 4,53 (d, 10,8Hz, 1H, OC**H**₂Ph) ; 4,6 (d, 49Hz, 1H, C**H**F) ; 4,72 (d, 10,8Hz, 1H, OC**H**₂Ph); 4,77 (d, 11,2Hz, 1H, OC**H**₂Ph) ; 4,85 (d, 11,1Hz, 1H, OC**H**₂Ph) ; 7,07-7,25 (m, 20H, **H**ar) ; 9,45 (d, 7,4Hz, 1H, C**H**O).
**RMN ¹³C (CDCl₃,75,5MHz)** 68,8 (C**6**) ; 72,6 (C**5**) ; 73,8 (O**C**H₂Ph) ; 75,4 (O**C**H₂Ph) ; 75,5 (OCH₂Ph) ; 76,0 (O**C**H₂Ph) ; 77,9 (C**2**) ; 78,4 (C**4**) ; 83,5 (C**3**) ; 94,1 (d, 192 Hz, **C**HF) ; 98,2 (d, 20 Hz, C**1**); 128,0 -128,9 (**C**ar.) ; 137,6 ;138,3 ; 138,4 ; 138,6 (**C**ar. quat.) ; 196,2 (d, 30Hz, **C**HO).

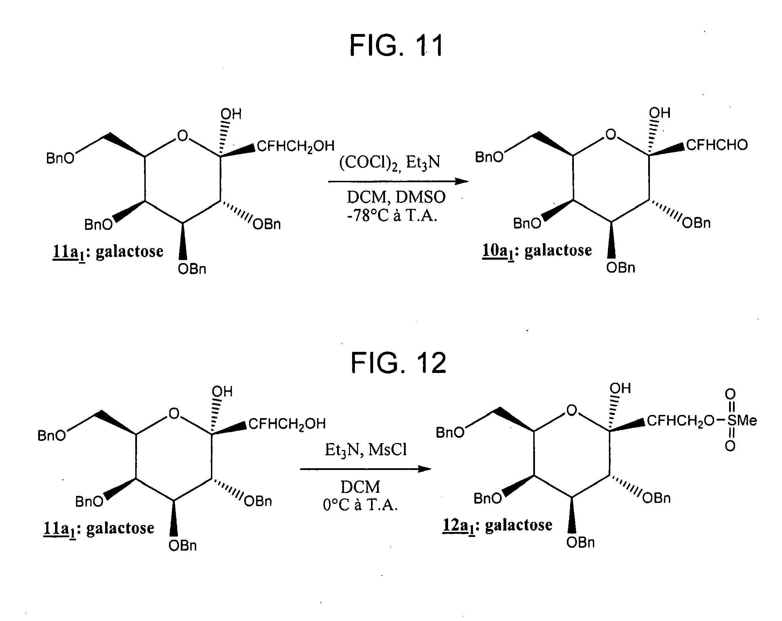

### Synthèse des composés 10a₁ (Figure 11)

Dans un ballon sous atmosphère inerte à -78°C, contenant du DMSO (12µL ; 0,16mmol ; 5éq.) dans le dichlorométhane (1mL), une solution de chlorure d'oxalyle (7µL, 0,073mmol, ; 2,2éq.) dans le dichlorométhane (1mL) est ajoutée. Le milieu réactionnel est agité pendant 15 mn à -78°C, puis l'alcool **11a₁** (20mg ; 0,033 mmol ; 1 éq.) est additionné. On laisse la température remonter à -40°C en 1 heure, puis la triéthylamine (24µL ; 0,17mmol), 5 éq.) est introduite. La température est laissée remonter à l'ambiante en deux heures, et une solution saturé de NaCl est ajoutée. Le milieu est extrait trois fois au dichlorométhane, puis les phases organiques sont rassemblées et lavées avec une solution aqueuse, séchées sur sulfate de magnésium, filtrées puis concentrées. Le produit est ensuite purifié par colonne chromatographique sur gel de silice avec un éluant cyclohexane / acétate d'éthyle (80 : 20). Le produit est isolé avec 31 % de rendement sous la forme d'une huile incolore.

### Caractérisation de 10a₁

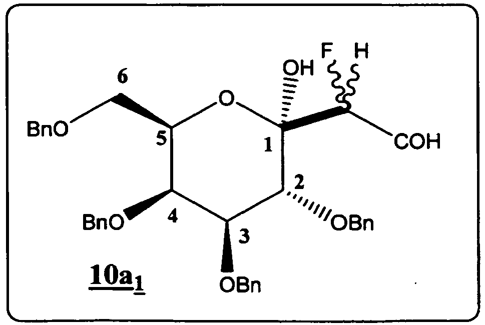

C₃₆H₃₇FO₇ M= 600,67 g.mol⁻¹
Rf = 0.45, éluant : cyclohexane / acétate d'éthyle (6 : 4).
**RMN ¹⁹F (CDCl₃, 282,5 MHz)**
-205,4 (dd, J_{F-H}=47,3Hz et 7,5Hz)
**RMN ¹H (CDCl₃, 300MHz)**
3,51 (dd, 11,4Hz et 1,7Hz, 1H, 1H**6**) ; 3,63-3,77 (m, 3H, 1H**6**, H**2**, H**4**) ; 3,88-3,97 (m, 2H, H**5**, H**3**); 4,39-4,9 (m, 8H, 40C**H**₂Ph) ; 4,62 (d, 47,7Hz, 1H, C**H**F) ; 7,07-7,25 (m, 20H, **H**ar) ; 9,74 (d, 7Hz, 1 H, C**H**O).
**RMN ¹³C (CDCl₃, 75,5MHz)**
68,8 (C**6**) ; 73,0 (C**5**) ; 73,7 (O**C**H₂Ph); 75,5 (O**C**H₂Ph); 76,0 (O**C**H₂Ph); 76,1 (O**C**H₂Ph) ; 78,2 (C**2**) ; 78,4 (C**4**) ; 83,2 (C**3**) ; 91,1 (d, 175 Hz, **C**HF) ; 128,0 -129,0 (**C**ar.) ; 137,8 ;138,3 ; 138,4 ; 138,7 (**C**ar. quat.) ; 200 (d, 30Hz, **C**HO).
**Masse** (ESI+) : 601 (M+H⁺) ; 618,27(M :hydrate H₂O) ; 636,27 (M :hydrate 2H₂O).

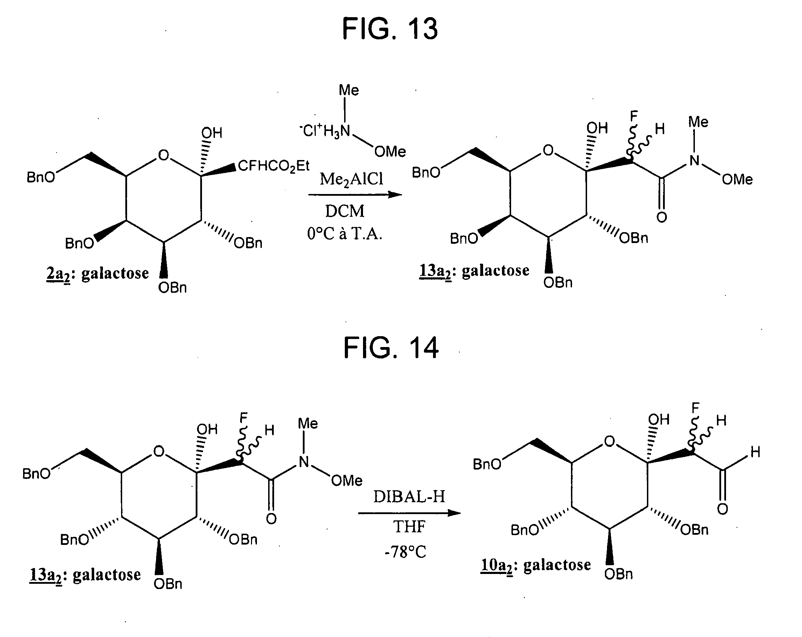

### Synthèse des composés 10a₂ (Figure 14)

Dans un ballon sous atmosphère inerte à -78°C, contenant le produit **13a₂** (0,165mmol ; 1éq.) dans le tétrahydrofurane anhydre (3mL), une solution de DIBAL-H 1M dans le toluène (0,25mL, 0,25mmol, 1,5éq.) est additionnée lentement. Le milieu est laissé sous agitation à -78°C pendant 4 heures. Puis une solution saturée de NH4Cl est ajoutée et le milieu est ramené à température ambiante. Après ajout d'une solution d(HCl 1M, le milieu est extrait trois fois au dichlorométhane, puis les phases organiques sont rassemblées et lavées avec une solution saturée de NaHCO₃, séchées sur sulfate de magnésium, filtrées puis concentrées. Le produit est ensuite purifié par colonne chromatographique sur gel de silice avec un éluant cyclohexane / acétate d'éthyle (80 : 20). Le produit est isolé avec 30% de rendement sous la forme d'une huile incolore.

### Caractérisation de 10a₂

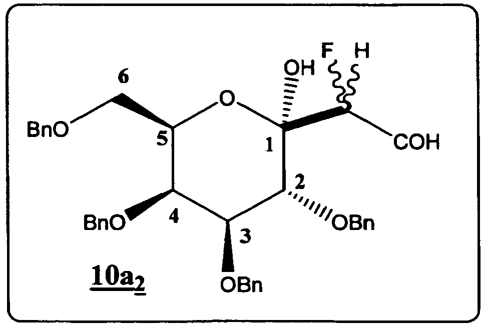

C₃₆H₃₇FO₇ M= 600,67 g.mol⁻¹

Rf = 0.6, éluant : cyclohexane / acétate d'éthyle (7 : 3).
**RMN ¹⁹F (CDCl₃, 282,5 MHz)**
-211,1 (dd, J_{F-H}=47,2Hz et 7,5Hz)
**RMN ¹H (CDCl₃, 300MHz)**
3,55-3,46 (m, 2H, H**6**) ; 3,8 (s,1H, O**H**) ; 3,90 (dd, 9,7Hz et 2,7Hz, 1H, H**3**) ; 3,92 (s, 1H, H**4**) ; 4,06 (t, 9,6Hz, 1H, H**5**,); 4,12 (d, 9,6Hz, 1H, H**2**,) ; 4,34 (d, 11,9Hz, 1H, OC**H**₂Ph); 4,39 (d, 11,9Hz, 1H, OC**H**₂Ph) ; 4,51 (d, 11,8Hz, 1H, OC**H**₂Ph) ; 4,52 (d, 10,6Hz, 1H, OC**H**₂Ph); 4,57 (d, 11,6Hz, 1H, OC**H**₂Ph) ; 4,59 (d, 47,6Hz, 1H, C**H**F) ; 4,67 (d, 11,5Hz, 1H, OC**H**₂Ph) ; 4,85 (d, 11,8Hz, 1H, OC**H**₂Ph); 4,88 (d, 10,6Hz, 1H, OC**H**₂Ph) ; 7,30 (m, 20H, **H**ar) ; 9,41 (d, 8,3Hz, 1H, C**H**O).
**RMN ¹³C (CDCl₃, 75,5MHZ)**
68,8 (C**6**) ; 71,4 (C5) ; 72,7 (O**C**H₂Ph) ; 73,9 (O**C**H₂Ph) ; 74,0 (C**4**) ; 74,5 (C**2**) ; 74,7 (O**C**H₂Ph); 75,4 (O**C**H₂Ph); 81,1 (C**3**) ; 94,4 (d, 190,8Hz, **C**HF); 98,6 (d, 20Hz, C**1**); 127,9 ; 128,0 ; 128,1; 128,2; 128,3; 128,4; 128,7; 128,8; 128,9; 129,0 (**C**ar.) ; 137,8 ;138,1 ; 138,3 ; 139,0 (**C**ar. quat.) ; 195,5 (d, 28Hz, **C**HO).
**Masse** (ESI+) : 655,33 (M :hydrate OMe+Na).

### Synthèse du composé 11a₁ (Figure 10)

Dans un ballon sous atmosphère inerte contenant l'ester **2a₁** (52mg ; 0,08 mmol ; 1 éq.) en solution dans l'éthanol (5 mL) le NaBH₄ (10mg, 0,241mmol, 3 éq.) est ajouté et le milieu est laissé sous agitation pendant 12 heures. Le mélange est concentré puis repris dans un mélange dichlorométhane, eau ,puis après décantation, la phase aqueuse est extraite trois fois au dichlorométhane. Puis les phases organiques sont rassemblées et lavées avec une solution aqueuse saturée de NaCl (15mL), séchées sur sulfate de magnésium, filtrées puis évaporées. Le brut est ensuite purifié par colonne chromatographique sur gel de silice avec un éluant cyclohexane / acétate d'éthyle (50: 50). Le produit est isolé avec 38% de rendement sous la forme d'une huile incolore.

### Caractérisation de 11a₁

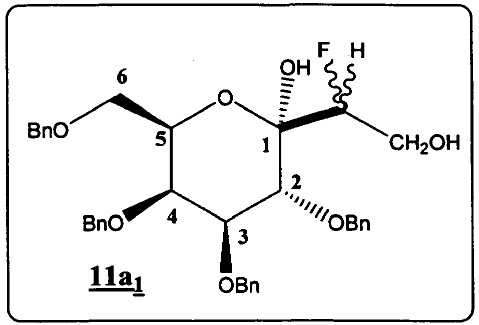

**C₃₆H₃₉FO₇** M= 602,69 g.mol⁻¹
Rf : 0,53 (cyclohexane/ acétate d'éthyle 5/5),
**RMN ¹⁹F (CDCl₃, 282,5 MHz)**
-199,2 (dt, J_{F-H}=46,1Hz et 19,3Hz)
**RMN ¹H (CDCl₃, 300MHz)**
2,11 (s, , 1H, O**H**) ; 3,58-3,65 (m, 3H,H**2** et 2H**6**); 3,81-4,00 (m, 5H, C**H**₂, H**4**, H**5**, H**3**); 4,44 (td, 3,7Hz et 46,5Hz, 1H, C**H**F) ; 4,48 -4,81 (m, 8H, 4 OC**H**₂Ph) ; 7,26 (m, 20H, **H**ar.).
**RMN ¹³C (CDCl₃, 75,5MHz)**
60,4 (d, 24,6Hz, **C**H₂) ; 68,8 (C**6**) ; 72,1 (C**5**) ; 73,6 (O**C**H₂Ph) ; 75,2 (O**C**H₂Ph) ; 75,9 (2C) (O**C**H₂Ph) ; 78,1 (C**2**); 78,6 (C**4**) ; 83,3 (C**3**); 90,1 (d, 183Hz, **C**HF) ; 97,9 (d , 22Hz, C**1**) ; 127,8 ; 127,9 (2C) ; 128,0 ; 128,1 ; 128,3 ; 128,6 (2C) (**C**ar.) 137,8 ;138,0 (2C) ; 138,4 (**C**ar. quat.).
**Masse** (ESI+) : 625,33 (M+Na).

### Synthèse du composé 11b₂ (Figure 10)

Dans un ballon sous atmosphère inerte contenant l'ester **2b₂** (52mg ; 0,08 mmol ; 1 éq.) en solution dans l'éthanol (5 mL) le NaBH₄ (10mg, 0,241mmol, 3 éq.) est ajouté et le milieu est laissé sous agitation pendant 12 heures. Le mélange est concentré puis repris dans un mélange dichlorométhane, eau ,puis après décantation, la phase aqueuse est extraite trois fois au dichlorométhane. Puis les phases organiques sont rassemblées et lavées avec une solution aqueuse saturée de NaCl (15mL), séchées sur sulfate de magnésium, filtrées puis évaporées. Le brut est ensuite purifié par colonne chromatographique sur gel de silice avec un éluant cyclohexane / acétate d'éthyle (50 : 50). Le produit est isolé avec 42% de rendement sous la forme d'une huile incolore.

### Caractérisation de 11b₂

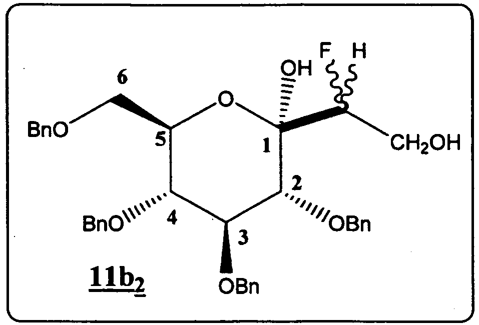

C₃₆H₃₉FO₇ M= 602,69 g.mol⁻¹
Rf : 0,53 (cyclohexane/ acétate d'éthyle 5/5),
**RMN ¹⁹F (CDCl₃, 282,5 MHz)**
-209,7 (dddd, J_{F-H}=47,3Hz, 27Hz, 22,6Hz et 4,3Hz)
**RMN ¹H (CDCl₃, 300MHz)**
2,26 (dd, 5,1Hz et 7Hz, 1H, O**H**) ; 3,44 (dd, 0,9Hz et 9,1Hz, 1H,H**2**) ; 3,50-3,67 (m, 5H, 2H**6**, C**H**₂, H**4**); 3,89-3,94 (ddd, 2,2Hz, 3,2Hz et 10Hz, 1H, H**5**) ; 4,00 (t, 9,1Hz, 1H, H**3**); 4,36 (td, 3,7Hz et 46,5Hz, 1H, C**H**F); 4,38 (d, 12Hz, 1H, OC**H**₂Ph) ; 4,47 (d, 12Hz, 1H, OC**H**₂Ph) ; 4,48 (d, 10,9Hz, 1H, OC**H**₂Ph) ; 4,63 (d, 11,2Hz, 1H, OC**H**₂Ph); 4,74 (d, 11Hz, 1H, OC**H**₂Ph); 4,82 (d, 10,1Hz, 1H, OC**H**₂Ph); 4,86 (d, 11,2Hz, 1H, OC**H**₂Ph) ; 7,09-7,12 (m, 20H, **H**ar) ; 9,45 (d, 7,4Hz, 1H, C**H**O).
**RMN ¹³C (CDCl₃, 75,5MHz)**
61,2 (d, 22Hz, **C**H₂) ; 68,5 (C**6**) ; 71,8 (C**5**) ; 73,8 **(OCH₂Ph) ;** 75,4 (O**C**H₂Ph) ; 76,1 (O**C**H₂Ph) ; 77,6 (O**C**H₂Ph) ; 78,1 (C**2** ou C**4**) ; 79,2 (C**2** ou C**4**) ; 83,5 (C**3**) ; 93,2 (d, 179Hz, **C**HF); 97,6 (d , 20Hz, C**1**); 128,1 ; 128,2 (2C) ; 128,3 ; 128,7 ; 128,8 ; 128,9 ; 129,0 ; 129,3 (**C**ar.) ; 137,7 ;138,3 ; 138,4 ; 138,7 (**C**ar. quat.).

### Synthèse du composé 12a₁ (Figure 12)

Dans un ballon sous atmosphère inerte contenant l'alcool **11a₁** (34mg ; 0,056 mmol ; 1 éq.) en solution dans le dichlorométhane anhydre (1 mL) à 0°C, la triéthylamine (10µL ; 0,075mmol ; 1,3éq.) et puis lentement le MsCl (7µL, 0,075mmol, 1,3 éq.) sont ajoutés. Le milieu est laissé sous agitation à 0°C pendant 30mn, puis à température ambiante pendant 3 heures. Le mélange est hydrolysé, puis extrait trois fois au dichlorométhane. Puis les phases organiques sont rassemblées et lavées avec une solution aqueuse saturée de NaCl, séchées sur sulfate de magnésium, filtrées puis évaporées. Le brut est ensuite purifié par colonne chromatographique sur gel de silice avec un éluant cyclohexane / acétate d'éthyle (70 : 30). Le produit est isolé avec 50% de rendement sous la forme d'une huile blanche.

### Caractérisation de 12a₁

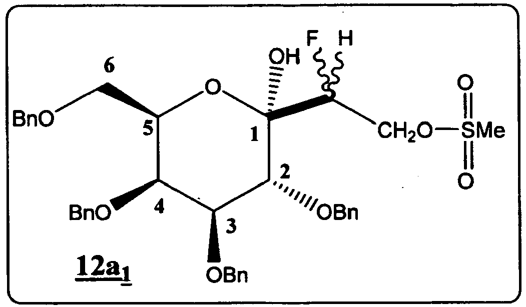

**C₃₇H₄₁FO₉S** M= 680,8 g.mol⁻¹
Rf : 0,83 (cyclohexane/ acétate d'éthyle 5/5),
**RMN ¹⁹F (CDCl₃, 282,5 MHz)**
-186,3 (ddd, J_{F-H}=51,5Hz, 35,4Hz et 18,2Hz)
**RMN ¹H (CDCl₃, 300MHz)**
2,95 (s, 3H, C**H**₃) ; 3,45 (dd, 5,6Hz et 9,1Hz, 1H, 1H**6**) ; 3,55 (dd, 7,6Hz et 9,1Hz, 1H, 1H**6**) ; 3,98 (ls, 2H, H3, H**4**) ; 4,08 (t, 6,8Hz, 1H, H**5**) ; 4,37 (ls, 3H, H**2** et OC**H**₂Ph); 4,54 (d, 11,5Hz, 1H, OC**H**₂Ph) ; 4,67 (s, 2H, OC**H**₂Ph) ; 4,68 (d, 1H, OC**H**₂Ph) ; 4,73(dd, 30Hz et 12,3Hz, 2H, C**H**₂) ; 4,9 (d, 11,4Hz, OC**H**₂Ph); 4,93 (d, 11,3Hz, 1H, OC**H**₂Ph); 4,97 (dd, 8,3Hz et 48Hz, 1H, C**H**F) ; 7,24 (m, 20H, **H**ar).
**RMN ¹³C (CDCl₃, 75,5MHz)**
37,8 (C**H**₃); 67,7 (C**6**) ; 68,3 (d, 21,7Hz, **C**H₂); 73,1 (O**C**H₂Ph); 73,6 (C**4**) ; 73,7 (O**C**H₂Ph) ; 74,9 (d, 3,5Hz, C**2**); 75,0 (O**C**H₂Ph) ; 75,1 (C**5**) ; 75,7 (O**C**H₂Ph) ; 80,3 (C**3**) ; 90,5 (d, 189,6Hz, **C**HF); 106,0 (d, 21,1Hz, C**1**); 127,7 ; 127,8 ; 128,0; 128,1 (2C) ; 128,5 ; 128,6 ; 128,7 (2C) (**C**ar.); 137,7 ;137,9 ; 138,1 ; 138,5 (**C**ar. quat.).
**Masse** (ESI+) : 680 (M+H⁺); 716 (M :hydrate 2H₂O).

### Synthèse des composés 13a₂ (Figure 13)

Dans un ballon sous atmosphère inerte à -0°C, contenant Me₂AlCl 1M dans le cyclohexane (1,5mL; 1,44mmol, 3 éq.) dans le dichlorométhane (15mL), l'amine de Weinreb (141mg ; 1,44mmol, ; 3 éq.),puis le mélange est agité pendant 1heure. L'ester 2**a₂** (311mg ; 0,481mmol ; 1éq.) dans le dichlorométhane anhydre (5mL) est additionné à 0°C, puis la température de la solution est laissée remontée à température ambiante et sous agitation pendant 12 heures. La réaction est hydrolysée avec une solution d'HCl 1M, filtrée sur célite, séchée sur sulfate de magnésium, puis concentrée. Le produit est ensuite purifié par colonne chromatographique sur gel de silice avec un éluant cyclohexane /acétate d'éthyle (50: 50). Le produit est isolé avec 75% de rendement sous la forme d'une huile jaune.

### Caractérisation de 13a₂

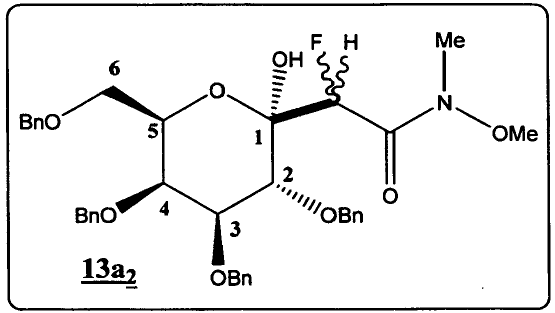

C₃₈H₄₂FO₈ M= 659,76 g.mol⁻¹
Rf = 0.62, éluant : cyclohexane / acétate d'éthyle (5 : 5).
**RMN ¹⁹F (CDCl₃, 282,5 MHz)**
-196,8 (d, J_{F-H}=48,3Hz)
**RMN ¹H (CDCl₃, 300MHz)**
3,1 (s, 3H, C**H**₃) ; 3,44-3,57 (m, 2H, H**6**) ; 3,59 (s, 3H, OC**H**₃) ; 3,93 (s, 1H, H**4**) ; 4,00-4,04 (m, 2H, H**2**, H**3**) ; 4,15 (t, 9,6Hz, 1H, H**5**,); 4,37 (m, 2H, OC**H**₂Ph) ; 4,53 (d, 11,5Hz, 1H, OC**H**₂Ph) ; 4,64-4,78 (m, 3H, OC**H**₂Ph); 4,84-4,94 (m, 2H, OC**H**₂Ph) 5,14 (d, 48,2Hz, 1H, C**H**F) ; 7,21-7,29 (m, 20H, **H**ar).
**RMN ¹³C (CDCl₃, 75,5MHz)**
32,3 (**C**H₃) ; 62,3 (O**C**H₃); 68, (C**6**) ; 70,5 (C**5**) ; 73,4 (O**C**H₂Ph) ; 73,7 (O**C**CH₂Ph) ; 75,0 (O**C**H₂Ph) ; 75,2 (C**4**) ; 75,6 (O**C**H₂Ph) ; 80,7 (C**2** et C**3**) ; 127,9 ; 128,0 ; 128,2 (2C); 128,6 (2C); 128,8; 128,9; 129,1 (Car.); 138,4 ;138,7 ; 138,9 ; 139,4 (**C**ar. quat.).
**Masse** (ESI+) : 660 (M+H) ; 677 (M+H₂O)

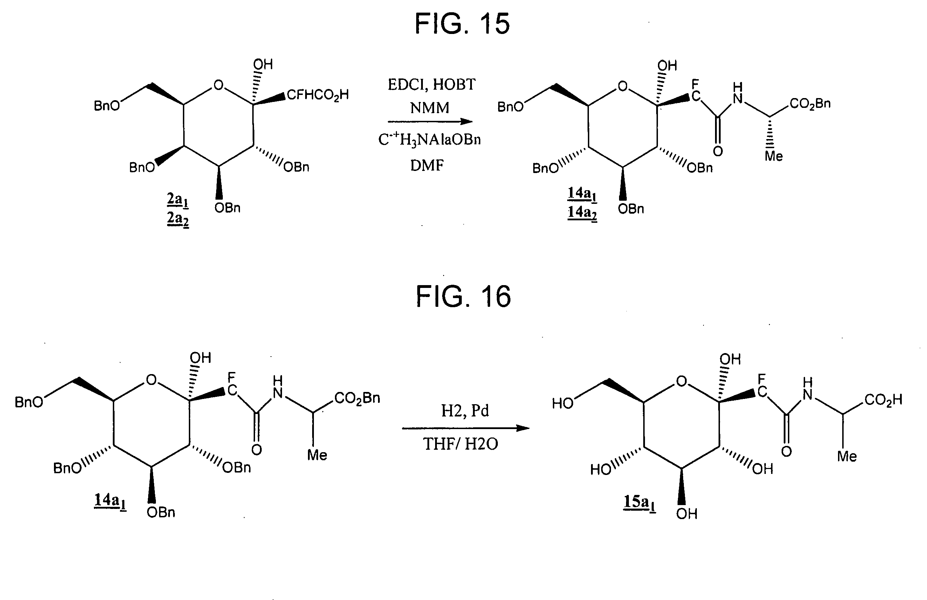

### Synthèse des composés 14a₁/14a₂ (Figure 15)

Sur une suspension de l'acide **2a₁/2a₂** (253 mg ; 0.42 mmol ; 1.0 éq.), de l'alanine (0.42 mmol ; 1.0 éq.), d'HOBT (65 mg ; 0.47 mmol ; 1.1 éq.), et de NMM (143 mg ; 1.41 mmol ; 3.3 éq.) dans le DMF (15 mL) sous atmosphère d'argon, l'EDCI (90 mg ; 0.47 mmol ; 1.1 éq.) est additionné après 15 minutes. La réaction est agitée à température ambiante pendant 24 heures et concentrée. Une solution d'acide chlorhydrique 1N (10 mL) est ajoutée ainsi que du dichlorométhane, et la phase aqueuse est extraite avec du DCM (3 x 20 mL). Les phases organiques sont lavées avec une solution saturée de NaCl (20 mL), séchées sur MgSO₄ et concentrées sous vide. Le résidu est purifié par chromatographie sur gel de silice avec un mélange cyclohexane l AeOEt (70 : 30) comme éluant pour permettre la séparation des 2 diastéréoisomères **14a₁** et **14a₂** sous forme de 2 solides blancs avec un rendement global de 60%.

### Caractérisation de 14a₁/14a₂

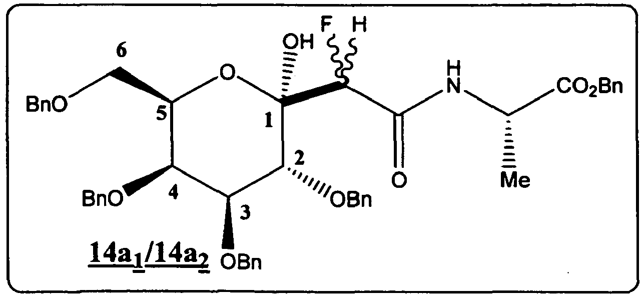

C₄₆H₄₈FNO₉ M= 777,87g.mol⁻¹

### Composé 14a₁

Rf = , éluant : cyclohexane / acétate d'éthyle 0.
**RMN ¹⁹F (CDCl₃, 282,5 MHz)**
- 200 (d, J_{F-H}=47,3Hz)
**RMN ¹H (CDCl₃, 300MHz)**
1,25 (d, 7,2Hz, 3H, C**H**₃); 3,43 (dd, 5,6Hz et 9,4Hz, 1H, 1H**6**) ; 3,59 (dd, 7,6Hz et 9,4Hz, 1H, 1H**6**) ; 4,01 (s, 1H, H**4**) ; 4,11 (dd, 2,6Hz et 10Hz, 1H, H**3**) ; 4,19 (t, 6,3Hz, 1H, H**5**,); 4,24 (d, 10Hz, 1H, H**2**) ; 4,37 (d, 11,7Hz, 1H, OC**H**₂Ph) ; 4,43 (d, 11,7Hz, 1H, OC**H**₂Ph) ; 4,62 (m, 1H, C**H**) ; 4,66 (d, 11,7Hz, 1H, OC**H**₂Ph) ; 4,71 (d, 11,3Hz, 1H, OC**H**₂Ph); 4,76 (s, 2H, OC**H**₂Ph) ; 4,56 (d, 11,7Hz, 1H, OC**H**₂Ph); 4,99 (d, 47,7Hz, 1H, C**H**F) ; 5,00 (d, 11,3Hz, 1H, OC**H**₂Ph); 5,19 (s, 2H, CO₂C**H**₂Ph) ; 5,55 (s, 1H, O**H**) ; 7,07 (dd, 3,5Hz et 7,6Hz, 1H, N**H**) ; 7,21-7,29 (m, 25H, **H**ar).
**RMN ¹³C (CDCl₃, 75,5MHz)**
18,3 (**C**H₃) ; 48,1 (**C**H); 67,5 (CO₂**C**H₂Ph) 68,8 (C**6**) ; 70,6 (C**5**) ; 72,9 (O**C**H₂Ph); 73,5 (O**C**H₂Ph) ; 74,4 et 74,5 (C**2** et C**4**) ; 74,6 (O**C**H₂Ph) ; 75,6 (O**C**H₂Ph) ; 80,4 (C**3**) ; 86,6 (d, 201,8Hz, **C**FH); 98,1 (d, 20,7Hz, C**1**); 127,6 ; 127,9 ; 128,3 ; 128,4; 128,5; 128,6 ; 128,8 (**C**ar.); 135,2 ; 138,0 ;138,1 ; 138,5 ; 138,9 (**C**ar. quat.) ; 168,9 (d, 19,6Hz, **C**OCFH) ; 171,8 (**C**O).
**Masse** (ESI+) : 760,27 (M+H) ; 795,2 (M+H₂O)

### Composé 14a₂

Rf = , éluant : cyclohexane / acétate d'éthyle 0.
**RMN ¹⁹F (CDCl₃, 282,5 MHz)**
- 201,9 (d, J_{F-H}=47,3Hz)
**RMN ¹H (CDCl₃, 300MHz)**
1,23 (d, 7,0Hz, 3H, C**H**₃) ; 3,49 (dd, 5,8Hz et 9,1Hz, 1H, 1H**6**) ; 3,56 (dd, 7,5Hz et 9,1Hz, 1H, 1H**6**) ; 3,94 (s, 1H, H**4**) ; 4,0 (dd, 2,7Hz et 9,7Hz, 1 H, H**3**) ; 4,12-4,23 (m, 2H, H**5** et H**2**); 4,35 (d, 11,8Hz, 1H, OC**H**₂Ph) ; 4,42 (d, 11,8Hz, 1H, OC**H**₂Ph) ; 4,49-5,10 (m, 10H, C**H** ; 4OC**H**₂Ph; C**H**F) ; 7,09-7,24 (m, 25H, **H**ar).

### Synthèse des composés 15a₁ (Figure 16)

Dans un ballon, le composé **14a₁** (0.100 mmol) est dissout dans le tétrahydrofurane (10 mL) avec de l'eau (5mL) et le palladium sur charbon et placé sous une atmosphère d'hydrogène. Le mélange est agité pendant 2 jours à température ambiante. Le mélange réactionnel est filtré, et concentré. Le brut est repris avec du dichlorométhane (20 mL) qui est éliminé, puis avec de l'eau (10 mL) qui est filtrée. La phase aqueuse est ensuite concentrée pour ainsi laisser le produit désiré en tant qu'un solide jaune pâle avec un rendement de 98%.

### Caractérisation de 15a₁

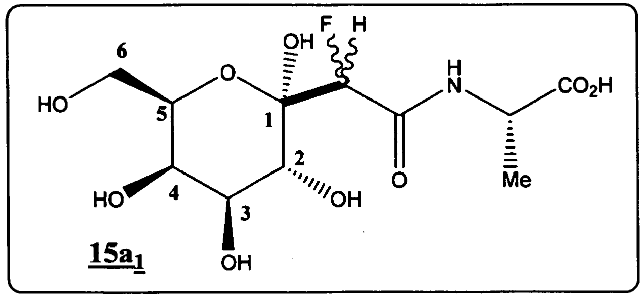

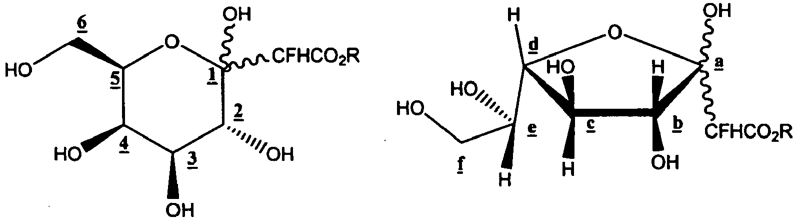

C₁₁H₁₈FNO₉ M= 327,26g.mol⁻¹
**RMN ¹H (D₂O, 300MHz)**
1,4 (d, 7,2Hz, 3H, C**H**₃) ; 3,6-3,0 (m, H**6**, H**e**, H**f**) ; 3,95 (s, H**4**) ; 4 (m, H2 et H**3**) ; 4,05 (t, 1H, H**5**) ; 4,1-4,3 (m , H**b**, H**c** et H**d**); 4,5 (m, 1H, C**H**); 5,1 (2d, 47Hz, C**H**F)
**RMN ¹³C (D₃O, 75,5MHz)**
15,0 (**C**H₃) ; 47,4 (**C**H); 59,8 (C**6**) ; 65,9 (C**4**) ; 68,1 et 68,9 (C**2** et C**3**) ; 70,9 (C**5**) ; 86,6 (d, 198Hz, **C**FH); 96,4 (d, 20,7Hz, C**1**); 170 (d, 19,6Hz, **C**OCFH) ; 171,8 (**C**O). **Masse** (ESI+) : 345,03 (M+Na)

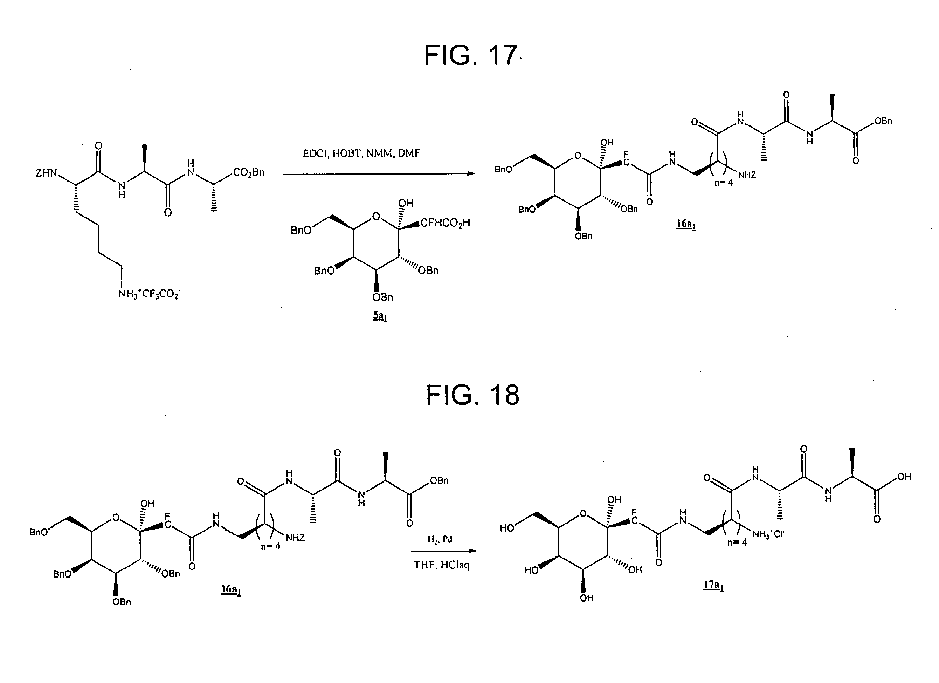

### Synthèse des composés 16a₁ (Figure 17)

Sur une suspension de l'acide **5a₁** (253 mg ; 0.42 mmol ; 1.0 éq.), du peptide (0.42 mmol ; 1.0 éq.), d'HOBT (65 mg ; 0.47 mmol ; 1.1 éq.), et de NMM (143 mg ; 1.41 mmol ; 3.3 éq.) dans le DMF (15 mL) sous atmosphère d'argon, l'EDCI (90 mg ; 0.47 mmol ; 1.1 éq.) est additionné après 15 minutes. La réaction est agitée à température ambiante pendant 24 heures et concentrée. Une solution d'acide chlorhydrique 1N (10 mL) est ajoutée ainsi que du dichlorométhane, et la phase aqueuse est extraite avec du DCM (3 x 20 mL). Les phases organiques sont lavées avec une solution saturée de NaCl (20 mL), séchées sur MgSO₄ et concentrées sous vide. Le résidu est purifié par chromatographie sur gel de silice avec un mélange cyclohexane / AcOEt (30 : 70) comme éluant pour isoler le composé **16a₁** sous forme de solide blanc avec un rendement global de 56%.

### Caractérisation de 16a₁

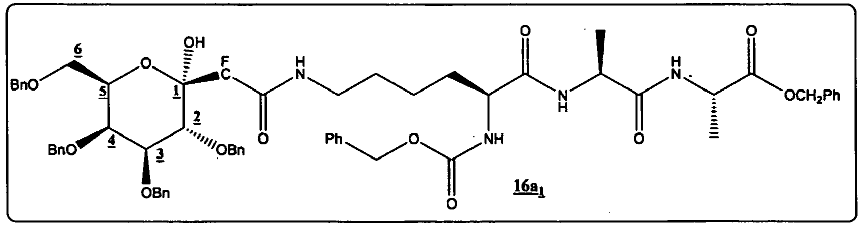

C₆₃H₇₀FN₄O₁₃ M= 1110,25g.mol⁻¹
Rf = , éluant : cyclohexane / acétate d'éthyle 0.
**RMN ¹⁹F (CDCl₃, 282,5 MHz)**
- 199,4 (d, J_{F-H}=48,4Hz)
**RMN ¹H (CDCl₃, 300MHz)**
1,22 et 1,30 (2d, 7,Hz, 6H, 2C**H**₃) ; 1,22-1,51 (m, 6H, 3C**H**₂lys) ; 3,01-3,07 (m, 1H, 1C**H**₂NH) ; 3,23-3,30 (m, 1H, 1C**H**₂NH) 3,37 (dd, 5,8Hz et 9,4Hz, 1H, 1H**6**) ; 3,47 (dd, 9,2Hz, 1H, 1H**6**) ; 4,01 (s, 1H, H**4**) ; 4,1 (dd, 1,8Hz) et 10Hz, 1H, H**3**) ; 4,15-4,24 (m, 3H, H**5**,C**H**Lys, H**2**) ; 4,45-4,73 (m, 6H, 2C**H**Ala, OC**H**₂Ph) 4,77 (s, 2H, OC**H**₂Ph) ; 4,93-5,21 (m, 6H, OC**H**₂Ph); 5,01 (d, 47,2Hz, 1H, C**H**F) ; 5,51 (d, 6,9Hz, 1H, N**H**) ; 5,65 (s, 1H, O**H**) ; 6,49 (d, 7,4Hz, 1H, N**H**) ; 6,62 (d, 7,3Hz, 1H, N**H**) ; 6,73 (s, 1H, N**H**) ; 7,18-7,23 (m, 25H, **H**ar).
**RMN ¹³C (CDCl₃, 75,5MHz)**
18,1 et 18,3 (2**C**H₃) ; 22,3 (**C**H₂) ; 28,7 (**C**H₂) ; 32,0 (**C**H₂); 38,5 (N**C**H₂) ; 48,3 et 48,9 (2**C**HAla); 54,8 (**C**HLys); 67,1 (O**C**H₂Ph) ; 67,2 (O**C**H₂Ph) ; 68,5 (C**6**) ; 70,4 (C**5**) ; 72,8 (OCH₂Ph) ; 73,4 (O**C**H₂Ph) ; 74,6 (C**4** et C**2**) ; 74,7 (O**C**H₂Ph) ; 75,5 (O**C**H₂Ph) ; 80,3 (C**3**) ; 86,8 (d, 200,7Hz, **C**FH); 98,1 (d, 20,7Hz, C**1**); 127,0 ; 127,6 ; 128,0;128,2; 128,3 (2C); 128,4 (2C); 128,5; 128,6; 128,7 (**C**ar.); 136,3; 138,0 ;138,2 ; 138,3 ; 138,5 ; 138,7 ; 138,8 ; 138,9 (**C**ar. quat.) ; 156,0 (**C**O) ; 169,4 (d, 19Hz, **C**OCFH) ; 171,7 et 172,6 (2**C**O).
**Masse** (ESI+) : 1128,33 (M+H₂O)

### Synthèse des composés 17a₁ (Figure 18)

Dans un ballon, le composé **16a₁** (0.028 mmol) est dissout dans le tétrahydrofurane (5 mL) avec la solution d'acide chlorhydrique 1N (1.2 éq.) et le palladium sur charbon et placé sous une atmosphère d'hydrogène. Le mélange est agité pendant 2 jours à température.ambiante. Le mélange réactionnel est filtré, et concentré. Le brut est repris avec du dichlorométhane (20 mL) qui est éliminé, puis avec de l'eau (10 mL) qui est filtrée. La phase aqueuse est ensuite concentrée pour ainsi laisser le produit désiré en tant qu'un solide blanc avec un rendement quantitatif.

### Caractérisation de 17a₁

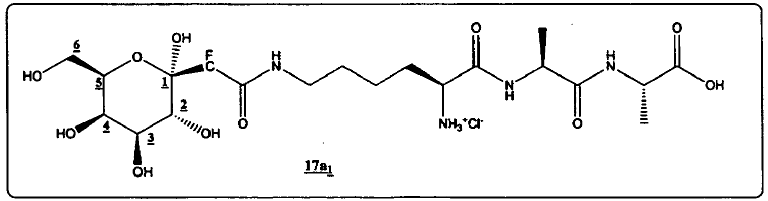

C₂₀H₃₅ClFN₄O₁₁ M= 561,96g.mol⁻¹
**RMN ¹⁹F (CDCl₃, 282,5 MHz)**
- 199,4 (d, J_{F-H}=48,4Hz)
**RMN ¹H (CDCl₃, 300MHz)**
1,22 et 1,30 (2d, 7,Hz, 6H, 2C**H**₃) ; 1,22-1,51 (m, 6H, 3C**H**₂lys); 3,01-3,07 (m, 1H, 1C**H**₂NH) ; 3,23-3,30 (m, 1H, 1C**H**₂NH) 3,37 (dd, 5,8Hz et 9,4Hz, 1H, 1H**6**) ; 3,47 (dd, 9,2Hz, 1H, 1H**6**) ; 4,01 (s, 1H, H**4**) ; 4,1 (dd, 1,8Hz et 10Hz, 1H, H3) ; 4,15-4,24 (m, 3H, H**5**,C**H**Lys, H**2**) ; 4,45-4,73 (m, 6H, 2C**H**Ala, OC**H**₂Ph) ; 4,77 (s, 2H, OC**H**₂Ph) ; 4,93-5,21 (m, 6H, OC**H**₂Ph) ; 5,01 (d, 47,2Hz, 1H, C**H**F) ; 5,51 (d, 6,9Hz), 1H, N**H**) ; 5,65 (s, 1H, O**H**) ; 6,49 (d, 7,4Hz, 1H, N**H**) ; 6,62 (d, 7,3Hz, 1H, N**H**) ; 6,73 (s, 1H, N**H**) ; 7,18-7,23 (m, 25H, **H**ar).
**RMN ¹³C (CDCl₃, 75,5MHz)**
18,1 et 18,3 (2**C**H₃) ; 22,3 (**C**H₂); 28,7 (**C**H₂) ; 32,0 (**C**H₂) ; 38,5 (N**C**H₂) ; 48,3 et 48,9 (2**C**HAla); 54,8 (**C**HLys); 67,1 (O**C**H₂Ph) ; 67,2 (O**C**H₂Ph) ; 68,5 (C**6**) ; 70,4 (C**5**) ; 72,8 (O**C**H₂Ph) ; 73,4 (O**C**H₂Ph) ; 74,6 (C**4** et C**2**) ; 74,7 (O**C**H₂Ph) ; 75,5 (O**C**H₂Ph); 80,3 (C**3**) ; 86,8 (d, 200,7Hz, **C**FH); 98,1 (d, 20,7Hz, C**1**); 127,0 ; 127,6 ; 128,0 ;128,2 ; 128,3 (2C); 128,4 (2C); 128,5; 128,6; 128,7 (**C**ar.) ; 136,3 ; 138,0 ;138,2 ; 138,3 ; 138,5 ; 138,7 ; 138,8 ; 138,9 (**C**ar. quat.) ; 156,0 (**C**O) ; 169,4 (d, 19Hz, **C**OCFH) ; 171,7 et 172,6 (2**C**O).
**Masse** (ESI+) : 1128,33 (M+H₂O)

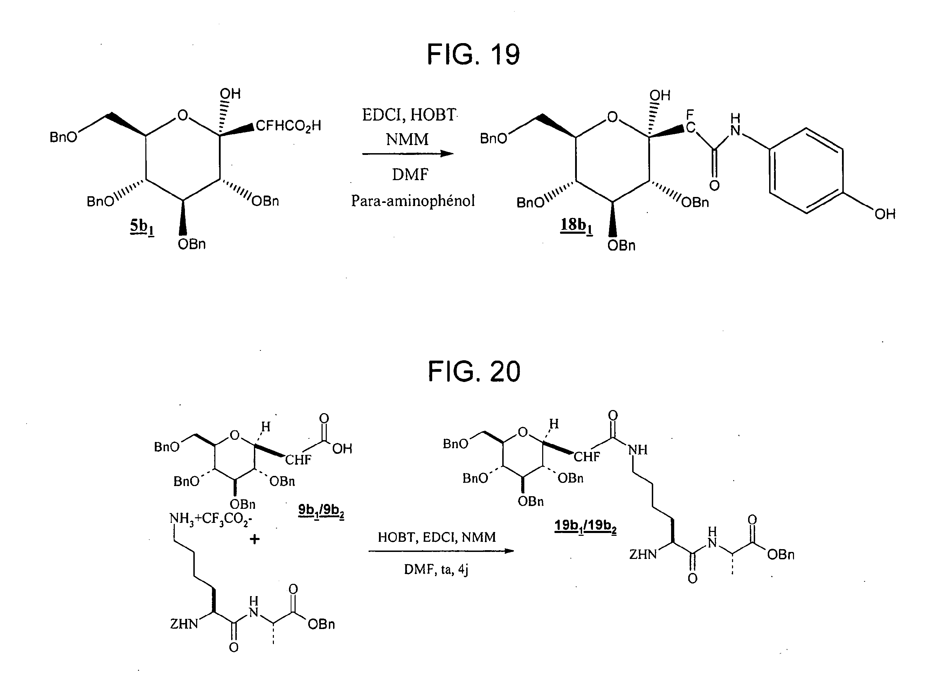

### Synthèse des composés 18b₁ (Figure 19)

Sur une suspension de l'acide **5b₁** (500 mg; 0.811 mmol; 1.05 éq.), le 4-aminophénol (84mg ; 0.773 mmol ; 1.0 éq.), d'HOBT (110 mg ; 0.811 mmol ; 1.05 éq.), et de NMM (80µL mg ; 0,811 mmol ; 1,05 éq.) dans le DMF (20 mL) sous atmosphère d'argon, l'EDCI (156 mg ; 0.811 mmol ; 1,05 éq.) est additionné après 15 minutes. La réaction est agitée à température ambiante pendant 24 heures et concentrée. Une solution d'acide chlorhydrique 1N (10 mL) est ajoutée ainsi que du dichlorométhane, et la phase aqueuse est extraite avec du DCM (3 x 20 mL). Les phases organiques sont lavées avec une solution saturée de NaCl (20 mL), séchées sur MgSO₄ et concentrées sous vide. Le résidu est purifié par chromatographie sur gel de silice avec un mélange cyclohexane / AcOEt (60: 40) comme éluant pour isoler le composé **18b₁** sous forme de solide blanc avec un rendement global de 44%.

### Caractérisation de 18b₁

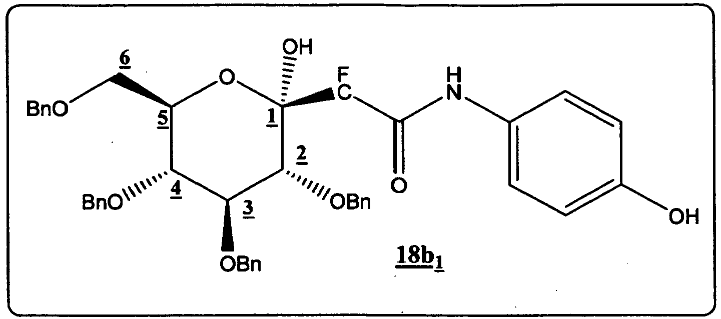

C₄₂H₄₁FNO₈ M= 706,78g.mol⁻¹
Rf = 0,27 éluant : cyclohexane / acétate d'éthyle (7/3).
**RMN ¹⁹F (CDCl₃, 282,5 MHz)**
- 197,6 (d, J_{F-H}=47Hz)
**RMN ¹H (CDCl₃, 300MHz)**
3,53 (dd, 11,3Hz, 1H, 1H**6**) ; 3,55-3,71 (m, 3H, 1H**6** , H**4**, H**2**); 3,98 (m, 1H, H**5**) ; 4,1 (t, 9,4Hz, 1H, H**3**) ; 4,36 (d, 12,2Hz, 1H, OC**H**₂Ph) ; 4,42 (d, 12,4Hz, 1H, OC**H**₂Ph) ; 4,53 (d, 10,9Hz, 1 H, OC**H**₂Ph) ; 4,64 (d, 11,5Hz, 1H, OC**H**₂Ph) ; 4,79 (d, 10,9Hz, 1H, OC**H**₂Ph) ; 4,86 (s, 2H, OC**H**₂Ph); 4,92 (d, 11,4Hz, 1H, OC**H**₂Ph) ; 4,95 (d, 48Hz, 1H, C**H**F) ; 5,46 (s, 1H, O**H**) ; 6,7 (d, 8,8Hz, 2H, **H**ar.) ; 7,13-7,25 (m, 22H, **H**ar) ; 7,86 (d, 5,5Hz, 1H, N**H**) ;.
**RMN ¹³C (CDCl₃, 75,5MHz)**
68,8 (C**6**) ; 72,4 (C**5**) ; 73,7 (O**CH**₂Ph); 75,4 (O**C**H₂Ph); 75,8 (O**C**H₂Ph); 76,2 (O**C**H₂Ph) ; 78,3 (C**2**) ; 78,6 (C**4**) ; 83,3 (C**3**) ; 87,1 (d, 202Hz, **C**FH); 98,0 (d, 20Hz, C**1**); 116,2 (2**C**ar.); 123,1 (2**C**ar.); 127,9-129,1 (**C**ar.); 138,2; 138,5 (2C) ;138,8 (**C**ar. quat.) ; 153,9 (**C**ar.OH) ; 167,5 (d, 18Hz, **C**OCFH).
**Masse** (ESI+) : 730,2 (M+23)

### Synthèse des composés 19b₁/19b₂ (Figure 20)

Sur une suspension de l'acide **9b₁/9b₂** (253 mg ; 0.42 mmol ; 1.0 éq.), du peptide (240 mg ; 0.42 mmol ; 1.0 éq.), d'HOBT (65 mg ; 0.47 mmol ; 1.1 éq.), et de NMM (143 mg; 1.41 mmol; 3.3 éq.) dans le DMF (15 mL) sous atmosphère d'argon, l'EDCI (90 mg ; 0.47 mmol ; 1.1 éq.) est additionné après 15 minutes. La réaction est agitée à température ambiante pendant 4 jours et concentrée. Une solution d'acide chlorhydrique 1N (10 mL) est ajoutée ainsi que du dichlorométhane, et la phase aqueuse est extraite avec du DCM (3 x 20 mL). Les phases organiques sont lavées avec une solution saturée de NaCl (20 mL), séchées sur MgSO₄ et concentrées sous vide. Le résidu est purifié par chromatographie sur gel de silice avec un mélange cyclohexane / AcOEt (1 : 1) comme éluant pour permettre la séparation des 2 diastéréoisomères sous forme de 2 solides blancs avec un rendement global 64 %. 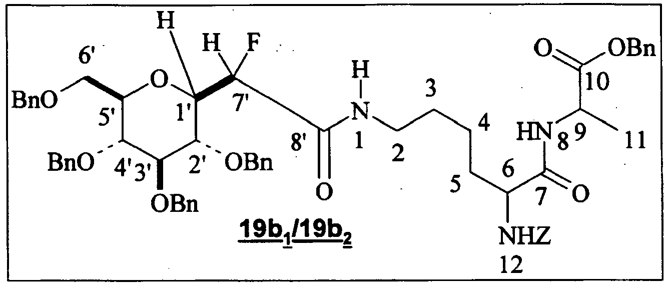 C₆₀H₆₆FN₃O₁₁ M= 1024,21 g.mol⁻¹

### Caractérisation de 19b₁

Rf= 0.52, éluant : cyclohexane / acétate d'éthyle (1 : 1).
**RMN ¹⁹F (CDCl₃, 282MHz)**
-197.6 (dd, ¹J_{F-H7'} 46.1, ²J_{F-H1'} 19.3)
**RMN ¹H (CDCl₃, 300MHz)**
1.26-1.68 (9H, m, H**_{3,}** H**₄**, H**₅**, C**H**₃), 2.82-2.89 (1H, m, H**2**), 3.11-3.19 (1H, m, H**2**), 3_{.}61 (1H, t, ³J 9_{.}7), 3_{.}71 (3H, sₐₚₚ, 2H**6'**), 3.88-3.94 (2H, m), 4.10-4.15 (1H, m, H**6**), 4.49-4.63 (6H, m, H**9**), 4.78-5.24 (7H, m, H**7'**), 5.50 (1H, d, ³J_{H12-H6} 7.9, H**12**), 6.38 (1H, d, ³J_{H1-H2} 2.7, H**1**), 6.74 (1H, d, ³J_{H8-H9} 7.1, H**8**), 7.12-7.33 (30H, m, **H**_{Ar}),
**RMN ¹³C (CDCl₃, 75MHz)**
18.1 (C**11**), 22.3 (C**4**), 27.9 (C**3**), 32.1 (C**5**), 38.4 (C**2**), 48.3 (C**9**), 54.6 (C**6**), 67.0, 67.3, 69.1, 73.5, 74.6, 75.2, 75.6, 78.2, 77.4 (d, ³J_{C2'-F} 7.4, C**2'**), 79.0 (d, ²J_{C1'-F} 20.0, C**1'**), 79.4, 87.3, 90.8 (d, ¹J_{C7'-F} 191.3, C7'), 127.1, 127.3, 127.6, 127.7, 127.8, 127.8, 127.9, 128.0, 128.0, 128.1, 128.1, 128.2, 128.3, 128.5, 128.5, 128.6, 128.6, 128.7, 128.7, 135.4, 136.4, 138.1, 138.3, 138.4, 138.5, 156.3, 167.2 (d, ²J_{C8'-F} 19.4, C**8'**), 174.2, 172.6, 171.4.

### Caractérisation de 19b₂

Rf= 0.43, éluant : cyclohexane / acétate d'éthyle (1 : 1).
**RMN ¹⁹F (CDCl₃, 282MHz)**
-206.2 (dd, ¹J_{F-H7'} 47.2, ²J_{F-H1'} 29.0)
**RMN ¹H (CDCl₃, 300MHz)**
1.68-1.26 (9H, m, C**H**₃, 2H**4**, 2H**5**, 2H**3**), 3.28-3.11 (2H, m, 2H2), 3.73-3.62 (3H, m), 3.94-3.88 (2H, m), 4.65-4.46 (6H, m, H**9**), 5.24-4.72 (7H, m, H**7'**), 5.73 (1H, d, ³J 7.6), 6.69 (1H, d, ³J6.9), 6.80 (1H, sₐₚₚ), 7.33-7.12 (30H, m, H_{Ar}),

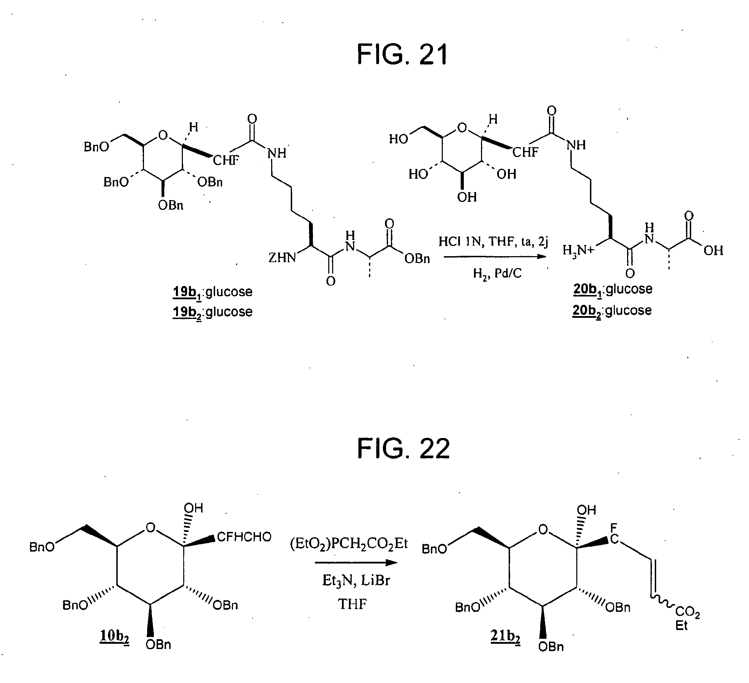

### Synthèse du composés 20b₁ (Figure 21)

Dans un ballon, le composé **19b₁** (30 mg ; 0.028 mmol) est dissout dans le tétrahydrofurane (5 mL) avec la solution d'acide chlorhydrique 1N (1.2 éq.) et le palladium sur charbon et placé sous une atmosphère d'hydrogène. Le mélange est agité pendant 2 jours à température ambiante. Le mélange réactionnel est filtré, et concentré. Le brut est repris avec du dichlorométhane (20 mL) qui est éliminé, puis avec de l'eau (10 mL) qui est filtrée. La phase aqueuse est ensuite concentrée pour ainsi laisser le produit désiré en tant qu'un solide blanc avec un rendement de 77%.

### Caractérisation de 20b₁

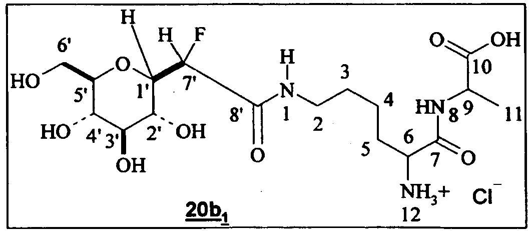

C₁₇H₃₀ClFN₃O₉ M= 439,44g.mol⁻¹
**RMN ¹⁹F (D₂O, 282MHz)**
-209.9 (1F, dd, ³J_{F-H1'} 29.0, ²J_{F-H7'} 46.1)
**RMN ¹H (D₂O, 300MHz)**
1.34 (3H, d, ³J_{H11-H9} 7.1, C**H**₃), 1.42-1.47 (2H, m, 2H**4**), 1.50-1.58 (2H, m, 2H**3**), 1.82-1.91 (2H, m, 2H**5**), 3.26-3.36 (4H, m, 2H**2**), 3.52-3,80 (3H, m, 2H**6'**, H**2'**), 3.95 (1H, t, ³J_{H6-H5} 6.5, H**6**), 4.15 (1H, q, ³J_{H9-H11} 7.1, H**9**), 5.27 (1H, d,²J_{H7'-F} 46.6, H**7'**)
**RMN ¹³C (D₂O, 75MHz)**
17.3 (C**11**),21.6 (C**4**), 28.2 (C**3**), 30.7 (C**5**), 39.0 (C**2**), 53.4 (C**6**), 60.8 (C**6'**) 68.7 (d, ³J_{C2'-F} 5.1, C**2'**), 77.5, 69.7, 78.4 (d, ²J_{Cl'-F} 18.1, C**1'**), 80.3, 88.5 (d, ¹J_{C7'-F} 191.8, C**7'**), 169.2 (C**10**).
**Spectrométrie de masse :** ESI+ : 440 (MH)+, 422 (MH-H₂O)+

### Synthèse du composés 20b₂ (Figure 21)

Dans un ballon, le composé **19b₂** (35 mg ; 0.032 mmol) est dissout dans le tétrahydrofurane (10 mL) avec la solution d'acide chlorhydrique 1N (1.2 éq.) et le palladium sur charbon et placé sous une atmosphère d'hydrogène. Le mélange est agité pendant 2 jours à température ambiante. Le mélange réactionnel est filtré, et concentré. Le brut est repris avec du dichlorométhane (20 mL) qui est éliminé, puis avec de l'eau (10 mL) qui est filtrée. La phase aqueuse est ensuite concentrée pour ainsi laisser le produit désiré en tant qu'un solide blanc avec un rendement de 75%.

### Caractérisation de 20b₂

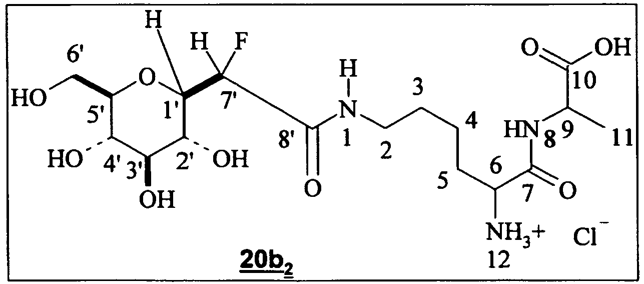

C₁₇H₃₀ClFN₃O₉ M= 439,44g.mol⁻¹
**RMN ¹⁹F (D₂O, 282MHz)**
-203.4 (1F, dd, ³J_{F-H1'} 25.8, ²J_{F-H7'} 48.3)
**RMN ¹H (D₂O, 300MHz)**
1.33 (3H, d, ³J_{H11-H9} 7.1, H**11**), 1.42-1.32 (2H, m, H**4**), 1.58-1.51 (2H, m, H**3**), 1.88-1.82 (2H, m, H**5**), 3.31-3.21 (2H, m, H**2**), 3.49-3.43 (2H, m), 3.64-3.58 (2H, m, H**2'**, H**6'**), 3.95-3.80 (3H, m, H**6**, H**1'**, H**6'**), 4.15 (1H, q, ³J_{H9-H11} 7.1, H**9**), 5.20 (1H, d, ²J_{H7'-F} 47.7, H**7'**),
**RMN¹³C (D₂O, 75MHz)**
17.2 (C**11**), 21.6 (C**4**), 28.1 (C**3**), 30.8 (C**5**), 38.8 (C**2**), 52.0 (C**9**), 53.4 (C**6**), 61.4 (C**6'**), 68.9 (d, ³J_{C2'-F} 7.0, C**2'**), 77.7, 69.9, 78.9 (d, ²J_{C1'-F} 19.1, C**1'**), 80.4, 90.6 (d, ¹J_{C7'-F} 190.9, C**7'**), 169.3 (C**10**).

### Synthèse du composé 21b₂ (Figure 22)

Dans un ballon sous atmosphère inerte contenant l'aldéhyde **10b₂** (130mg ; 0,216 mmol ; 1 éq.) en solution dans le THF anhydre (4 mL), Le triéthylphosphonoacétate (86µL, 0,433mmol, 2 éq.), le LiBr (38mg, 0,433mmol, 2 éq.) et la triéthylamine (61µL, 0,433mmol, 2 éq.) sont ajoutés et le milieu est laissé sous agitation pendant 12 heures. Le mélange est hydrolysé (20mL d'eau), puis extrait à l'acétate d'éthyle (3*15mL). Puis les phases organiques sont rassemblées et lavées avec de l'eau (15mL), puis une solution saturée de NaCl (15mL), séchées sur sulfate de magnésium, filtrées puis évaporées. Le brut est ensuite purifié par colonne chromatographique sur gel de silice avec un éluant cyclohexane / acétate d'éthyle (80: 20). Le produit est isolé avec 32% de rendement sous la forme d'une huile incolore.

### Caractérisation de 21b₂

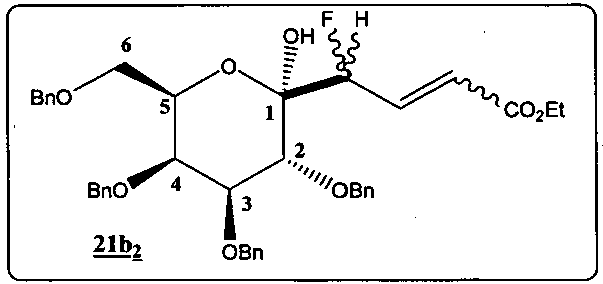

C₄₀H₄₃FO₈ M= 670,76 g.mol⁻¹
**RMN¹⁹F (CDCl₃, 282,5 MHz)**
-200,4 (dd, J_{F-H}=19,4Hz et 47,3Hz)
**RMN ¹H (CDCl₃, 300MHz)**
1,2 (t, 7,2Hz, 3H, C**H**₃) ; 3,45 (d, 1H, O**H**); 3,5 (d, 9Hz, 1H, H**2**); 3,65 (t, 9,4Hz, 1H, H**4**); 3,60-3,75 (m, 2H, H**6**); 3,95 (m, 1H, H**5**); 4 (t, 10,5Hz, 1H, H**3**; 4,07 (qdt, 7,2Hz, 2H, C**H**₂); 4,47 (d, 12,3Hz,1H, OC**H**₂Ph) ; 4,55 (d, 12,2Hz,1H OC**H**₂Ph) ; 4,58 (d, 10,7Hz, 1H, OC**H**₂Ph) ; 4,60 (d, 11,1Hz,1H, OC**H**₂Ph) ; 4,76 (d, 11,5Hz,1H, OC**H**₂Ph); 4,8 (d, 11,5Hz), 1H, OC**H**₂Ph); 4,83 (d, 10,1Hz,1H, OC**H**₂Ph); 4,90 (d, 10,9Hz,1H, OC**H**₂Ph); 4,97 (ddd, 1,7Hz, 5,9Hz et 46Hz, 1H, C**H**F) ; 5,86 (dt, 1,7Hz et 15,7Hz, 1H, **H**) ; 6,76 (ddd, 4,8Hz, 15,7Hz et 20Hz, 1H, C**H**F) ; 7,3 (m, 20H, **H**ar.).
**RMN ¹³C (CDCl₃, 75,5MHz)**
14,5 (**C**H₃); 61,1 (**C**H₂) ; 68,7 (C**6**) ; 72,6 (C**5**) ; 73,8 (O**C**H₂Ph) ; 75,1 (O**C**H₂Ph); 75,4 (O**C**H₂Ph); 76,0( (O**C**H₂Ph) ; 78,3 (C**4** ); 78,5 (C**2**) ; 84,0 (C**3**) ; 92,7 (d, 181Hz, **C**HF); 97,5 (d , 20Hz, C**1**); 124,2 (d, 11,6Hz, **C**H=); 128,0; 128,3; 128,6 ; 128,8 (2C) ; 128,9 ; 129,0 (**C**ar.) ; 137,7 ;138,4 ; 138,7 (2C) (**C**ar. quat.), 165,8 (**C**O).

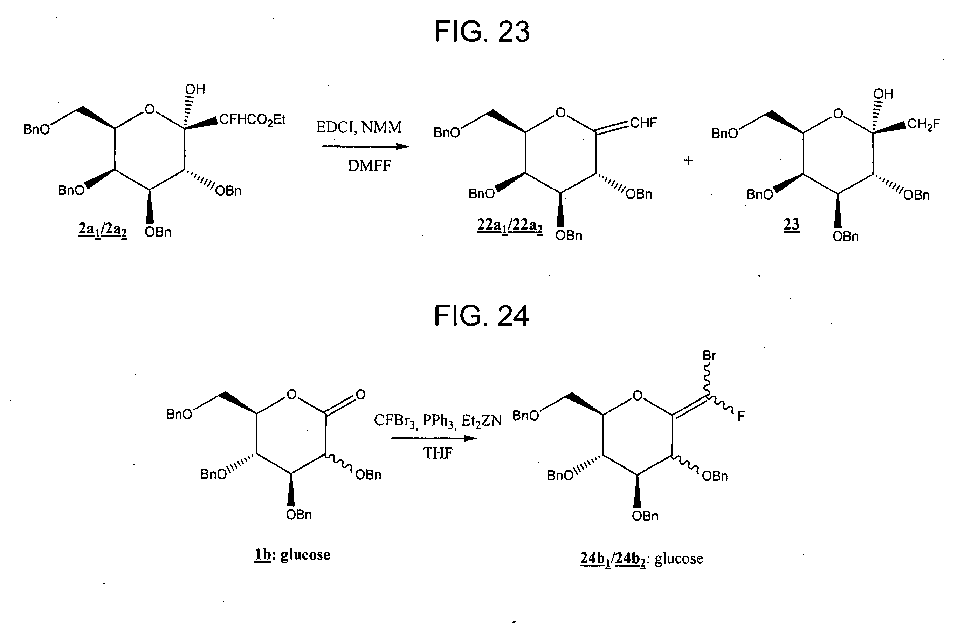

### Synthèse des composés 22a₁/22a₂ et 23 (Figure 23)

Dans un ballon sous atmosphère inerte contenant un mélange des acides **5a₁/5a₂** (120mg; 0,195 mmol ; 1 eq.), et de N-methylmorpholine NMM (64 µL ; 65,6 mmol ; 3 eq.) dans le DMF (5 mL), l'EDCI (41 mg ; 0,214 mmol ; 1,1 eq.) est ajouté. L'agitation est maintenue pendant 24 heures, puis le solvent est évaporé. Le milieu est repris dans le dichlorométhane et lavé deux fois avec une solution d'HCl 1M. La phase organique est séchée sur sulfate de magnésium, filtrée et concentrée. Le brut est ensuite purifié sur colonne chromatographique et les deux isomères des composés sont isolés avec un mélange 9/1 cyclohexane/acétate d'éthyle et un rendement de 35%, le composés secondaire est isolé avec un mélange cyclohexane/acétate d'éthyle et un rendement de 10%.

### Caractérisation de 22a₁/22a₂

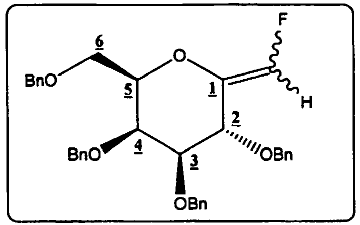

C₃₅H₃₅FO₅ M= 554,65 g.mol⁻¹
**RMN ¹⁹F (CDCl₃, 282,5MHz)**
-165,0 (d, 81Hz, 1F) → 41%
-159,1 (d, 77Hz, 1F) → 59%
**RMN ¹H (CDCl₃, 300MHz)**
3,63-3,76 (m, 3H, 2H6 et H**3**) ; 3,98 (td, 2,8 et 6Hz, 1H, H**5**); 4,05 (t, 2,8Hz,1H, H**4**); 4,14 (dd, 1,8 et 7,6Hz, 1H, H**2**) ; 4,40-4,81 (m, 8H, 4OC**H**₂Ph) ; 6,43 (dd, 1,4 et 77Hz, 1H, C**H**Fmajo) ; 6,87 (d, 89Hz, 1H, C**H**Fmino) ; 7,23 (m, 20H, **H**ar)
**RMN ¹³C (CDCl₃,75,5MHz)**
68,5 (M) et 69,1 (m) (2C**₆**) ; 73,2 (O**C**H₂Ph) ; 73,3 (O**C**H₂Ph) ; 73,9 (O**C**H₂Ph) ; 74,2 (C**2** et C**4**) ; 74,3 (O**C**H₂Ph); 79,1 (C**5**) ; 81,1 (C**3**) ; 128,0-128,9 (**C**ar.) ; 138,1 ; 138,4 ; 138,6 (2C) (**C**ar. quat.).

### Caractérisation de 23

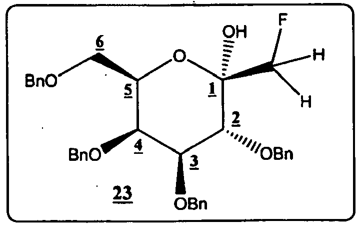

C₃₅H₃₇FO₆ M= 572,66 g.mol⁻¹
**RMN ¹⁹F (CDCl₃, 282,5MHz)**
-230,3 (t, 47Hz, 1F)
**RMN ¹H (CDCl₃, 300MHz)**
3,63-3,73 (m, 4H dont 2H**6**) ; 3,90-3,96 (m, 2H); 4,2 (d, 47,3Hz,1H, C**H**₂F); 4,46 (d, 12,3Hz, 1H, OC**H**₂Ph) ; 4,52 (d, 10,8Hz, 1H, OC**H**₂Ph); 4,57 (d, 12,2Hz, 1H, OC**H**₂Ph) ; 4,6 (d, 11Hz, 1H, OC**H**₂Ph) ; 4,74 (d, 10,8Hz, 1H, OC**H**₂Ph) ; 4,80 (d, 11,1Hz, 1H, OC**H**₂Ph) ; 4,84 (d, 11,1Hz, 1H, OC**H**₂Ph); 4,86 (d, 11Hz, 1H, OC**H**₂Ph); 7,08-7,02 (m, 20H, **H**ar)
**RMN ¹³C (CDCl₃,75,5MHz)**
68,8 (C**6**) ; 72,5 (C**5** ou C**2**) 73,9 (O**C**H₂Ph) ; 75,3 (O**C**H₂Ph) ; 75,9 (O**C**H₂Ph) ; 76,1 (O**C**H₂Ph) ; 78,3 (C**5** ou C**2**) ; 78,6 (C**4**) ; 83,6 (C**3**) ; 83,9 (d, 179Hz, **C**FH₂) ; 96,6 (d, 19Hz, C**1**); 128,0-128,9 (**C**ar.) ; 137,8 ; 138,4 ; 138,6 ; 138,8 (**C**ar. quat.).

### Synthèse des composés 24b₁/24b₂(Figure µ24)

Dans un ballon sous atmosphère inerte contenant la triphénylphosphine (230mg; 0,88mmol; 1,2éq.), le tribromofluorométhane CFBr₃ (86µL; 0,88mmol; 1,2éq.) et la lactone **1b** (394 mg ; 0,731 mmol ; 1 eq.) dans le THF anhydre, une solution de diéthylzinc Et₂Zn 1M dans l'hexane ou le toluène (880µL; 0,88mmol, 1,2éq.) est ajouté goutte à goutte lentement. Le mélange est agité pendant 4 heures, puis on additionne du MeOH et on concentre le milieu réactionnel Le brut est ensuite purifié sur colonne chromatographique et les deux isomères des composés sont recueillis ensemble avec un mélange 95/5 cyclohexane/acétate d'éthyle et un rendement de 25%.

### Caractérisation de 24b₁/24b₂

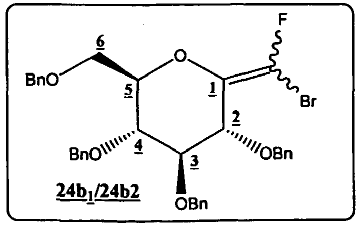

C₃₅H₃₄BrFO₅ M= 633,54 g.mol⁻¹
Rf = 0.67, éluant : cyclohexane / acétate d'éthyle (8 : 2).
**RMN ¹⁹F (CDCl₃, 282,5MHz)**
-99,0 (s, 1F)
-118,6 (s, 1F)
**RMN ¹H (CDCl₃, 300MHz)**
3,63-3,7 (m, 3H dont 2H**6**); 3,80-3,85 (m, 1H); 4,18 (td, 0,5H); 4,29-4,63 (m, 9,5H); 7,10-7,24 (m, 20H, **H**ar)

### Test en présence de Glycosidase

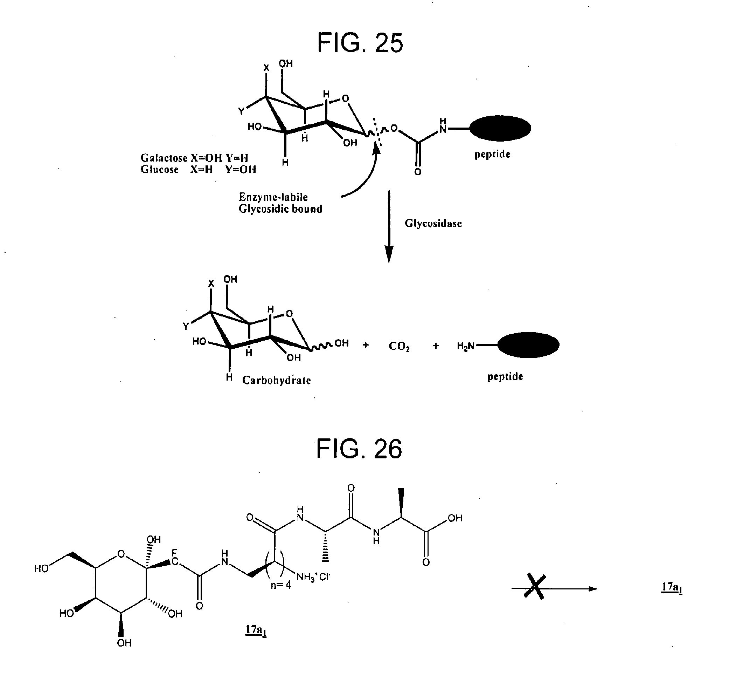

Pour démontrer, la résistance de nos composés face aux glycosidases et ainsi prouver leur stabilité, on a engagé le composé **17a₁** dans des réactions avec des galactosidases. Il est en effet connu que les composés suivants subissent des dégradations enzymatiques **(****figure 25****).**
Le protocole utilisé est le suivant **(****figure 26****)**
Une solution du composé **17a₁** (17,72mg) dans l'eau (500µL) est additionnée à une solution de tampon phosphate (0,07M ; pH7, 4mL) contenant des α-galactosidase (5 units) and β-galactosidase (6,25 units) à 37°C. La réaction est suivie en RMN 19F NMR. Des prélèvements sont réalisés à 24 heures, puis 48, 72, 96 et 120 heures. Aucun changement n'est observe et on retrouve le produit de départ.

## Revendications

1. Composé C-glycoside de formule I : 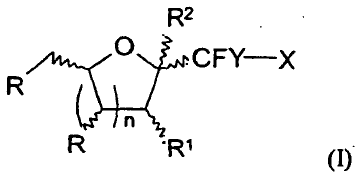 où
n est un nombre entier égal à 1 ou 2,
Y représente un atome d'hydrogène ou un atome de chlore ou un brome,
X est un atome d'hydrogène ou une chaîne alkyle linéaire ou ramifié possédant au moins une fonction amine, amide, acide, ester, carbonyle, alcool, aryle ou un groupe carbonyle, ester, amide, amine, alcool,
R identiques ou différents, représentent un groupement OH ou OR' où R' est un groupement alkyle linéaire ou ramifié, benzyle, benzoyle, acétyle, pivaloyle, trialkylsilyle, tertiobutyldiphénylsilyle ou un ou plusieurs sucres,
R¹ représente OR', NR"R"', N₃, ou un phtalimide
R" et R"', identiques ou différents, représentent un atome d'hydrogène ou un groupe alkyle linéaire ou ramifié, aryle, benzyle, benzoyle, acétyle, alkyloxycarbonyle, allyloxycarbonyle, benzyloxycarbonyle,
R² représente un atome d'hydrogène ou un halogène ou un groupement OH, OR', NR"R"' ou N₃,
ainsi que ses dérivés à l'état de base, de sel d'addition à un acide minéral ou organique, d'hydrate ou de solvate physiologiquement ou pharmaceutiquement acceptable,
à l'exclusion des composés suivants :
- 3,4,6-tri-O-benzoyl-1-déoxy-1-fluoro-β-D-fructofuranoside de méthyle,
- Ester éthylique de l'acide 2-déoxy-2-fluoro-4,5,7-tris-O-(phénylméthyl)-D-arabino-3-heptulofuranosonique,
- acide 2-déoxy-2-fluoro-4,5,7-tris-O-(phénylméthyl)-D-arabino-3-heptulofuranosonique,
- 1-déoxy-1-fluoro-3,4,6-tris-O-(phénylméthyl)-D-fructofuanose,
- diacétate de 1-déoxy- 1-fluoro-3,4-bis-O-(phénylméthyl)-D-fructofuranose, et
- 1-déoxy-1-fluoro-3,4-bis-O-(phénylméthyl)-D-fructofuranose.

2. Composé selon la revendication 1, **caractérisé en ce que** les groupes alkyles linéaires ou ramifiés sont des groupes possédant de 1 à 15 atomes de carbones.

3. Procédé de préparation d'un composé de formule I selon la revendication 1 pour laquelle Y représente un atome d'hydrogène, **caractérisé en ce qu'**il comprend une réaction d'addition de Reformatsky d'un bromofluoroacétate d'alkyle en présence de zinc sur les lactones de formule II suivantes : 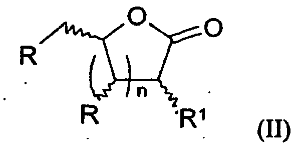 avec n, R, R¹ tels que définis dans la revendication 1.

4. Procédé de préparation d'un composé de formule I selon la revendication 1 pour lequel X et Y représentent des atomes d'hydrogène et R² représente un groupement OH, **caractérisé en ce qu'**un composé de formule I tel que défini à la revendication 1 pour lequel R² = OH, Y = H et X = CO₂H est mis à réagir avec un agent de couplage peptidique, tel que le 3-éthyl-1(N,N-diméthylaminopropylcarbodiimide ou la dicyclohexyl carbodiimide, et en présence d'une amine tertiaire, telle que la N-méthylmorpholine ou la diisopropylethylamine.

5. Procédé de préparation d'un composé de formule I selon la revendication 1 pour lequel Y représente un atome d'hydrogène, **caractérisé en ce qu'**il comprend une réaction d'un dibromofluoroacétate d'alkyle en présence de diéthylzinc et de triphénylphosphine sur des lactones de formule II telles que définies à la revendication 3.

6. Procédé de préparation d'un composé de formule I selon la revendication 1 pour lequel Y représente un atome d'halogène tel que le chlore et le brome, **caractérisé en ce qu'**il comprend une réaction de dihalogénofluoroacétate d'alkyle en présence de diéthylzinc sur des lactones de formule II telles que définies à la revendication 3.

7. Procédé selon l'une des revendications 3 à 6, **caractérisé en ce que** les lactones de formule II sont obtenues par des étapes de protection par benzylation du sucre, suivi de l'hydrolyse acide de la position anomérique, puis de son oxydation.

8. Procédé de préparation d'un composé de formule 1 selon la revendication 1 pour lequel R² représente un atome de clore ou de brome, **caractérisé en ce qu'**un composé de formule I tel que défini à la revendication 1 pour lequel R² = OH est halogéné.

9. Procédé de préparation d'un composé de formule I selon la revendication 1 pour lequel R² représente un atome d'hydrogène, **caractérisé en ce qu'**un composé de formule I tel que défini à la revendication 1 pour lequel R² = Cl ou Br est réduit.

10. Composé de formule III : 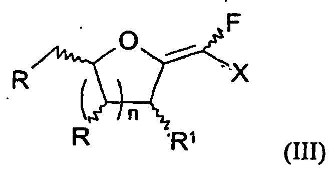 avec n, R, R¹ et X tels que définis dans la revendication 1.

11. Procédé de préparation d'un composé de formule III selon la revendication 10 pour lequel X = Br, **caractérisé en ce qu'**il comprend la réaction d'une lactone de formule II telle que définie à la revendication 3 en présence de tribromofluorométhane, de triphénylphosphine et de diéthylzinc.

12. Procédé de préparation d'un composé de formule III selon la revendication 10 pour lequel X = H, par mise en réaction d'un composé de formule I selon la revendication 1 pour lequel X = CO₂H, R² = OH et Y = H en présence d'un agent de couplage peptidique, tel que le 3-éthyl-l(N,N-diméthylaminopropylcarbodiimide ou la dicyclohexyl carbodiimide, et en présence d'une amine tertiaire, telle que la N-méthylmorpholine ou la diisopropylethylamine.

13. Procédé de préparation d'un composé de formule générale 1 selon la revendication 1 pour lequel R²=H et Y=H, par réduction de la double liaison du composé de formule générale III tel que défini à la revendication 9.

14. Composé selon la revendication 1, **caractérisé en ce qu'**il répond à la formule IV suivante : 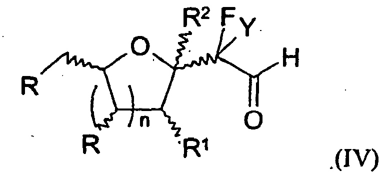 où
n est un nombre entier égal à 2,
Y représente un atome d'hydrogène,
R² représente un atome d'hydrogène ou un groupement OH ou OR'.

15. Composé de formule V suivante : 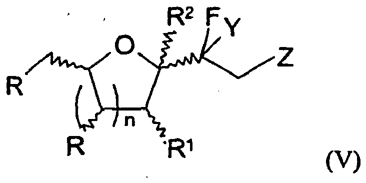 où
n est un nombre entier égal à 2,
Y représente un atome d'hydrogène,
R² représente un atome d'hydrogène ou un groupement OH ou OR',
Z représente un groupement OH, OR³ avec R³= alkyl, benzyl, mesyl, tosyl ou triflate, ou un atome d'halogène tel que Cl, Br, I.

16. Composé de formule VI suivante : 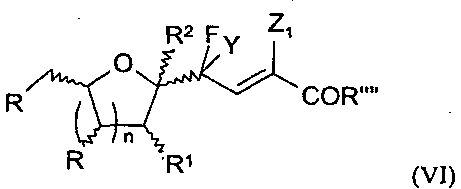 où
n est un nombre entier égal à 2,
Y représente un atome d'hydrogène,
R² représente un atome d'hydrogène ou un groupement OH ou OR',
Z₁ représente H ou NR"R'" avec R" et R"', identiques ou différents, représentant un atome d'hydrogène ou un groupe alkyle linéaire ou ramifié, aryle, benzyle, benzoyle, acétyle, alkyloxycarbonyle, allyloxycarbonyle ou benzyloxycarbonyle, ,
R"" représentent OR" ou NR"R"' ou un acide aminé.

17. Composé de formule VII suivante : 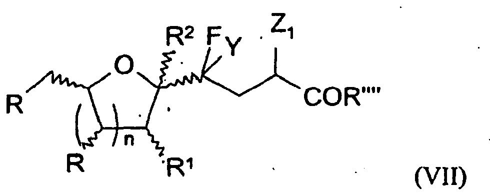 où
n est un nombre entier égal à 2,
Y représente un atome d'hydrogène,
R² représente un atome d'hydrogène ou un groupement OH ou OR',
Z₁ représente H ou NR"R'" avec R" et R"' identiques ou différents, représentent un atome d'hydrogène ou un groupe alkyle linéaire ou ramifié, aryle, benzyle, benzoyle, acétyle, alkyloxycarbonyle, allyloxycarbonyle ou benzyloxycarbonyle,
R"" représentent OR" ou NR"R"' ou un acide aminé.

18. Composé de formule VIII suivante : 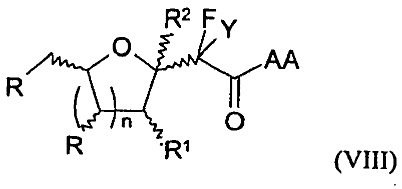 où
n est un nombre entier égal à 2,
Y représente un atome d'hydrogène,
R² représente un atome d'hydrogène ou un groupement OH ou OR', et AA représente un acide aminé ou un peptide.

19. Composé de formule IX suivante : 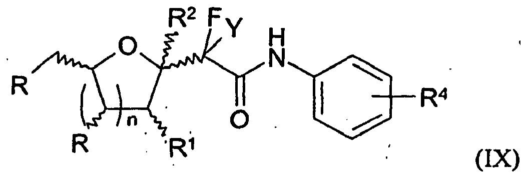 où
n est un nombre entier égal à 2,
Y représente un atome d'hydrogène,
R² représente un atome d'hydrogène ou un groupement OH ou OR',
R⁴ représente un hydrogène, halogène, NR"R"', OH ou OR",
avec R" et R"', identiques ou différents, représentent un atome d'hydrogène ou un groupe alkyle linéaire ou ramifié, aryle, benzyle, benzoyle, acétyle, alkyloxycarbonyle, allyloxycarbonyle ou benzyloxycarbonyle.

20. Composé de formule I selon la revendication 1 pour lequel R² représente un groupement OH, **caractérisé en ce qu'**il se présente sous les formes ouverte, furanose et pyranose du sucre, lorsqu'il est en solution dans des solvants polaires et protiques.

21. Composé selon l'une des revendications 1, 2, 10 ou 14 à 19 choisi parmi :

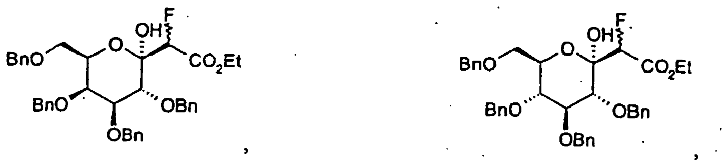

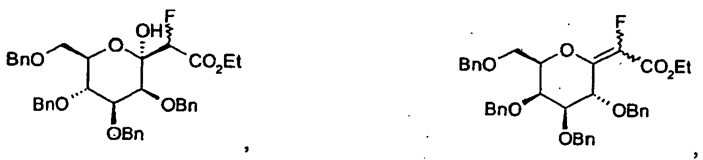

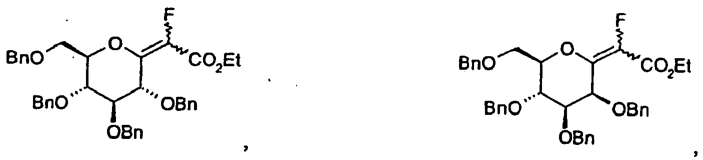

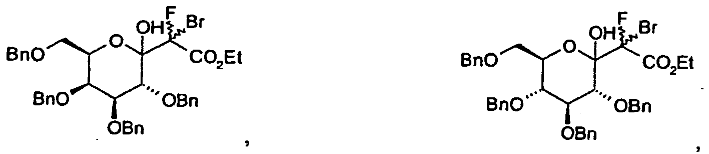

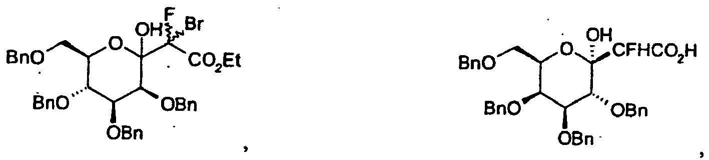

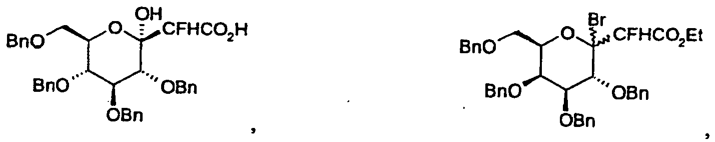

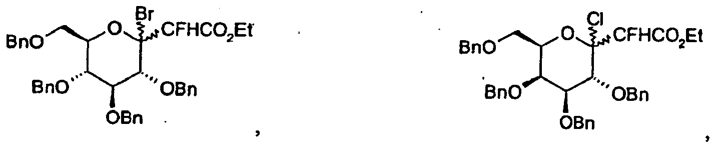

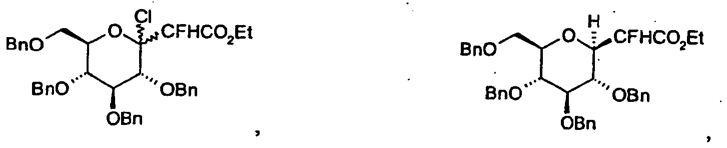

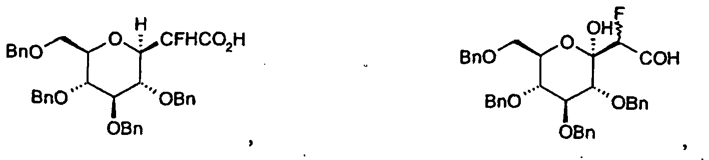

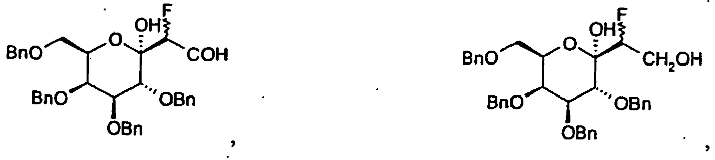

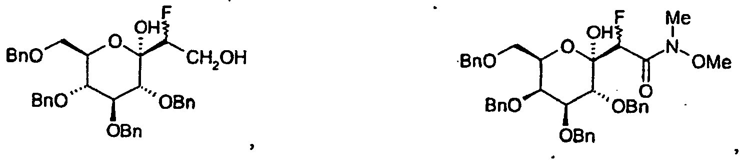

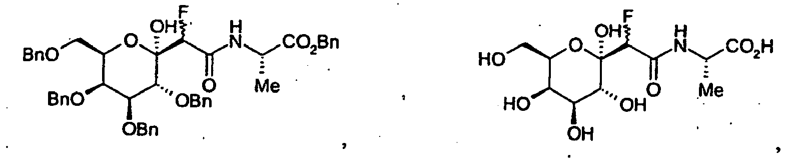

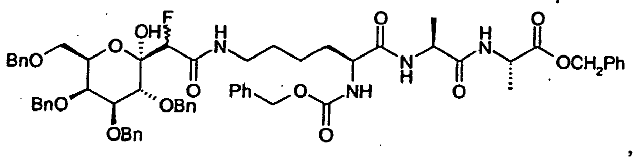

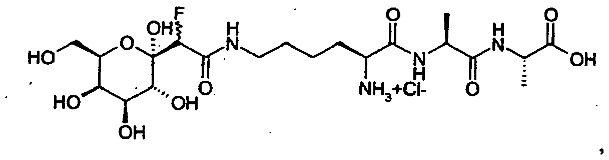

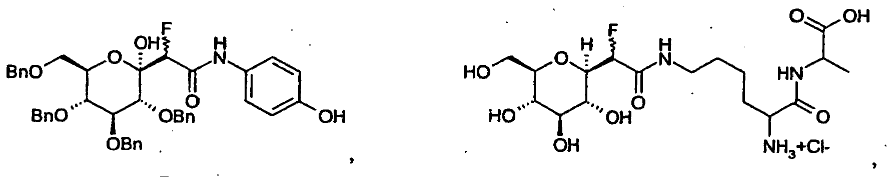

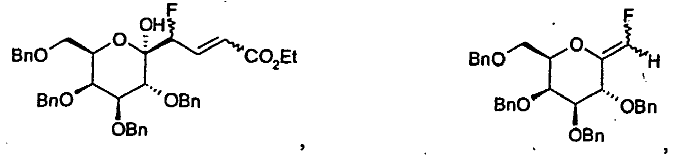

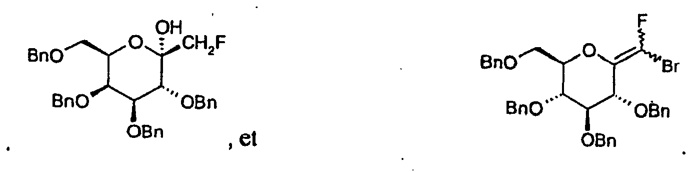

## Claims

1. A C-glycoside compound of formula (I): 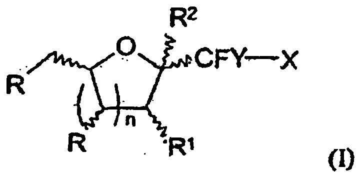 in which:
n is an integer equal to 1 or 2,
Y represents an atom of hydrogen, chlorine or bromine,
X is an atom of hydrogen or a linear or branched alkyl chain with at least one amine, amide, acid, ester, carbonyl, alcohol or aryl function or a carbonyl, ester, amide, amine or alcohol group,
R units are identical or different and represent an OH or OR' group,
wherein R' is a linear or branched alkyl, benzyl, benzoyl, acetyl, pivaloyl, trialkylsilyl, tertiobutyldiphenylsilyl group or one or more sugars,
R¹ represents OR', NR"R''', N₃, or a phthalimide, R" and R"', identical or different, represent an atom of hydrogen or a linear or branched alkyl, aryl, benzyl, benzoyl, acetyl, alkyloxycarbonyl, allyloxycarbonyl or benzyloxycarbonyl group,
R² represents an atom of hydrogen, a halogen or an OH, OR', NR"R''' or N₃ group,
as well as derivatives of same in form of a base, a mineral or organic acid addition salt, a a physiologically or pharmaceutically acceptable hydrate or solvate, with the exception of the following compounds:
- methyl 3,4,6-tri-O-benzoyl-1-deoxy-1-fluoro-β-D-fructofuranoside,
- 2-deoxy-2-fluoro-4,5,7-tris-O-(phenylmethyl)-D-arabino-3-heptulofuranosonic acid ethyl ester,
- 2-deoxy-2-fluoro-4,5,7-tris-O-(phenylmethyl)-D-arabino-3-heptulofuranosonic acid,
- 1-deoxy-1-fluoro-3,4,6-tris-O-(phenylmethyl)-D-fructofuranose,
- 1-deoxy-1-fluoro-3,4-bis-O-(phenylmethyl)-D-fructofuranose diacetate, and
- 1-deoxy-1-fluoro-3,4-bis-O-(phenylmethyl)-D-fructofuranose.

2. The compound according to claim 1, wherein the linear or branched alkyl groups are groups with 1 to 15 carbon atoms.

3. A method for preparing a compound of formula (I) according to claim 1 in which Y represents an atom of hydrogen, wherein it comprises a Reformatsky addition reaction of an alkyl bromofluoroacetate in the presence of zinc with the lactones of following formula (II): 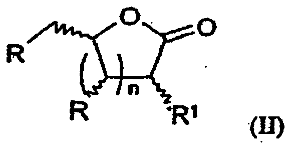 with n, R and R¹ as defined in claim 1.

4. A method for preparing a compound of formula (I) according to claim 1 in which X and Y represent hydrogen atoms and R² represents an OH group, wherein a compound of formula (I) as defined in claim 1 in which R²=OH, Y=H and X=CO₂H is reacted with a peptide coupling agent such as 3-ethyl-1(N,N-dimethylaminopropyl)carbodiimide or dicyclohexyl carbodiimide in the presence of a tertiary amine such as N-methylmorpholine or diisopropylethylamine.

5. A method for preparing a compound of formula (I) according to claim 1 in which Y represents a hydrogen atom, wherein it comprises a reaction of an alkyl dibromofluoroacetate in the presence of diethylzinc and triphenylphosphine with the lactones of formula (II) as defined in claim 3.

6. A method for preparing a compound of formula (I) according to claim 1 in which Y represents a halogen atom such as chlorine and bromine wherein it comprises a reaction of alkyl dihalofluoroacetate in the presence of diethylzinc with the lactones of formula (II) as defined in claim 3.

7. A method according to one of the claims 3 to 6, wherein the lactones of formula (II) are obtained by steps of protection by benzylation of sugar, followed by acid hydrolysis of the anomeric position and then its oxidation.

8. A method for preparing a compound of formula (I) according to claim 1 in which R² represents a chlorine or bromine atom, wherein a compound of formula (I) as defined in claim 1 in which R²=OH is halogenated.

9. A method for preparing a compound of formula (I) according to claim 1 in which R² represents an atom of hydrogen, wherein a compound of formula (I) as defined in claim 1 in which R²=Cl or Br is reduced.

10. A compound of formula (III): 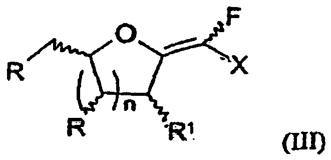 with n, R, R¹ and X as defined in claim 1.

11. A method for preparing a compound of formula (III) according to claim 10 in which X=Br, wherein it comprises a reaction of a lactone of formula (II) as defined in claim 3 in the presence of tribromofluoromethane, triphenylphosphine and diethylzinc.

12. A method for preparing a compound of formula (III) according to claim 10 in which X=H by reacting a compound of formula (I) according to claim 1 in which X=CO₂H, R²=OH and Y=H in the presence of a peptide coupling agent such as 3-ethyl-1(N,N-dimethylaminopropyl)carbodiimide or dicyclohexyl carbodiimide in the presence of a tertiary amine such as N-methylmorpholine or diisopropylethylamine.

13. A method for preparing a compound of the general formula (I) according to claim 1 wherein R²=H and Y=H by the reduction of the double bond of the compound of general formula (III) as defined in claim 9.

14. A compound according to claim 1, wherein it is of following formula (IV): 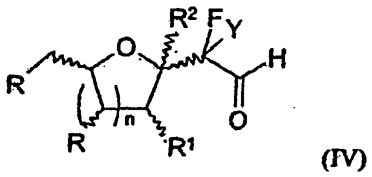 in which:
n is an integer equal to 2,
Y represents a hydrogen atom,
R² represents a hydrogen atom or an OH or OR' group.

15. A compound of following formula (V): 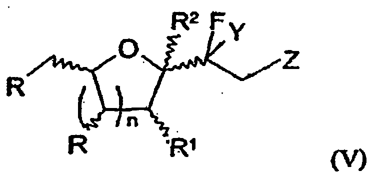 in which:
n is an integer equal to 2,
Y represents a hydrogen atom,
R² represents a hydrogen atom or an OH or OR' group,
Z represents an OH group, OR³ with R³=alkyl, benzyl, mesyl, tosyl or triflate, or a halogen atom such as Cl, Br or I.

16. A compound of following formula (VI): 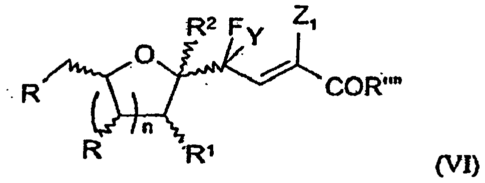 in which:
n is an integer equal to 2,
Y represents a hydrogen atom,
R² represents a hydrogen atom or an OH or OR' group,
Z₁ represents H or NR"R''' with R" and R''', identical or different, representing a hydrogen atom or a linear or branched alkyl, aryl, benzyl, benzoyl, acetyl, alkyloxycarbonyl, allyloxycarbonyl or benzyloxycarbonyl group,
R"" represents OR" or NR"R''' or an amino acid.

17. A compound of following formula (VII): 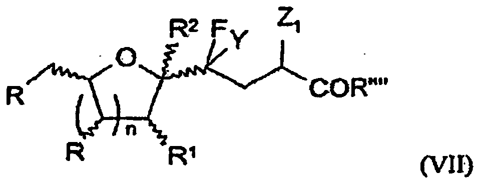 in which:
n is an integer equal to 2,
Y represents an atom of hydrogen,
R² represents an atom of hydrogen or an OH or OR' group,
Z₁ represents H or NR"R"' with R" and R"', identical or different, representing an atom of hydrogen or a linear or branched alkyl, aryl, benzyl, benzoyl, acetyl, alkyloxycarbonyl, allyloxycarbonyl or benzyloxycarbonyl group,
R"" represents OR" or NR"R" ' or an amino acid.

18. A compound of following formula (VIII): 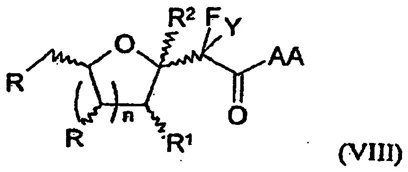 in which:
n is an integer equal to 2,
Y represents an atom of hydrogen,
R² represents an atom of hydrogen or an OH or OR' group, and
AA represents an amino acid or a peptide.

19. A compound of following formula (IX): 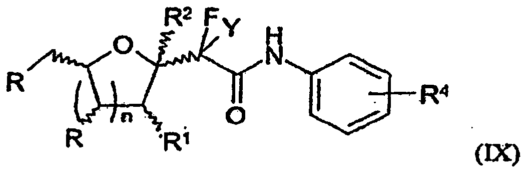 in which:
n is an integer equal to 2,
Y represents an atom of hydrogen,
R² represents an atom of hydrogen or an OH or OR' group,
R⁴ represents a hydrogen, halogen, NR"R"', OH or OR",
with R" and R"', identical or different, representing an atom of hydrogen or a linear or branched alkyl, aryl, benzyl, benzoyl, acetyl, alkyloxycarbonyl, allyloxycarbonyl or benzyloxycarbonyl group.

20. A compound of formula (I) according to claim 1, in which R² represents an OH group, wherein it is present in the forms open furanose and pyranose of the sugar when it is in solution in polar and protic solvents.

21. A compound according to one of claims 1, 2, 10 or 14 to 19 chosen among:

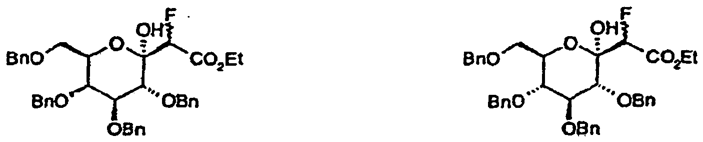

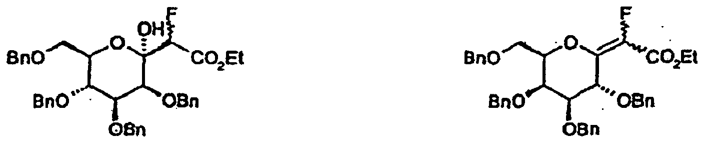

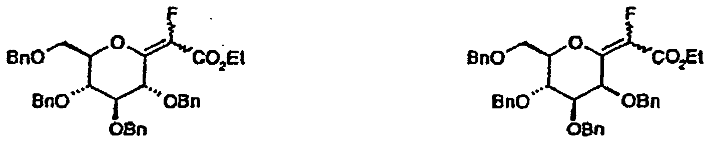

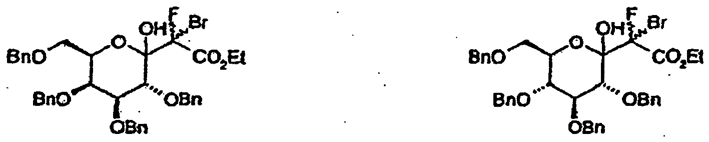

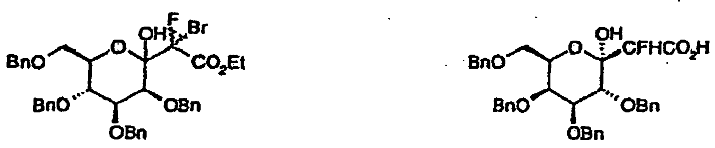

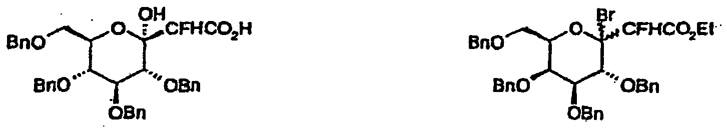

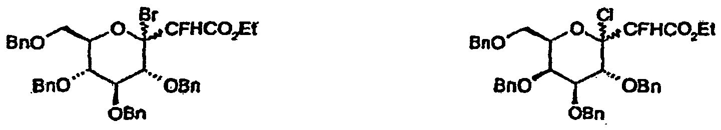

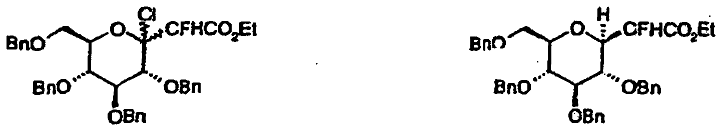

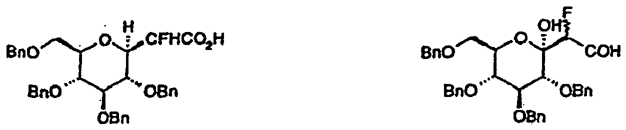

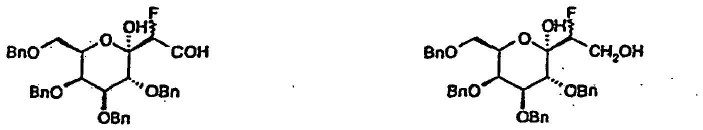

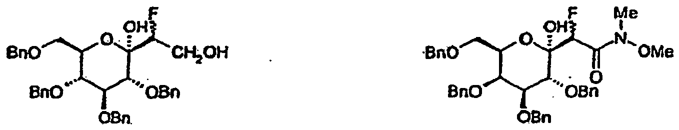

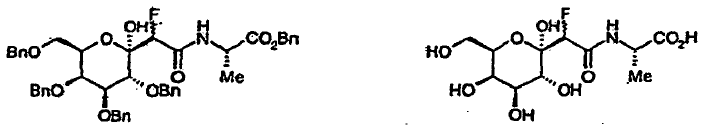

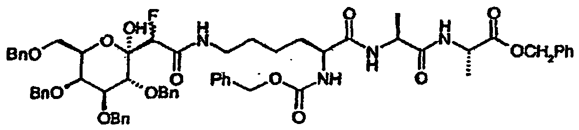

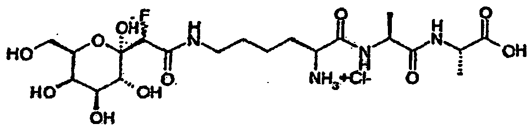

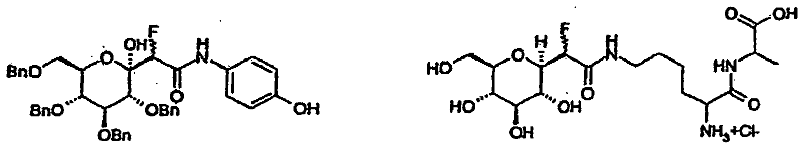

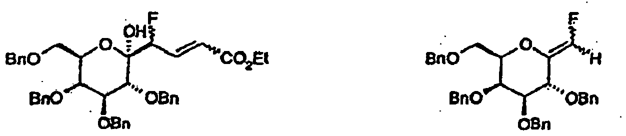

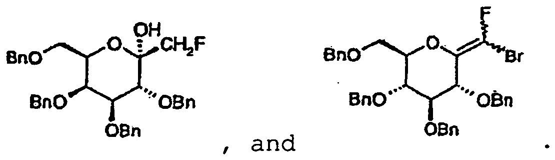

## Patentansprüche

1. C-Glycosid-Verbindung der Formel I: 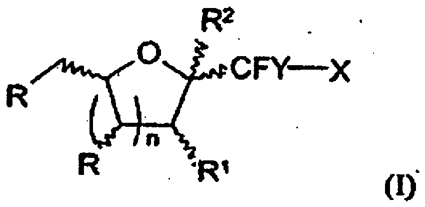 wobei
n eine ganze Zahl gleich 1 oder 2 ist,
Y ein Wasserstoffatom oder ein Chlor- oder Bromatom repräsentiert,
X ein Wasserstoffatom ist oder eine lineare oder verzweigte Alkylkette, die mindestens eine Amin-, Amid-, Säure-, Ester-, Carbonyl-, Alkohol- oder Arylfunktion oder eine Carbonyl-, Ester-, Amid-, Amin- oder Alkoholgruppe aufweist,
R identisch oder verschieden sind und eine OH- oder OR'-Gruppe repräsentieren, wobei R' eine lineare oder verzweigte Alkylgruppe oder eine Benzyl-, Benzoyl-, Acetyl-, Pivaloyl-, Trialkylsilyl- oder tert-Butyldiphenylsilylgruppe oder einen oder mehrere Zucker repräsentiert,
R¹ OR', NR"R"', N₃ oder ein Phthalimid repräsentiert, R" und R"' identisch oder verschieden sind und ein Wasserstoffatom oder eine lineare oder verzweigte Alkylgruppe oder eine Aryl-, Benzyl-, Benzoyl-, Acetyl-, Alkyloxycarbonyl-, Allyloxycarbonyl- oder Benzyloxycarbonylgruppe repräsentieren,
R² ein Wasserstoffatom oder ein Halogen oder eine OH-, OR'-, NR"R"'- oder N₃-Gruppe repräsentiert,
sowie deren Derivate als Base, als Additionssalz mit einer Mineralsäure oder organischen Säure, als Hydrat oder als Solvat, die physiologisch oder pharmazeutisch akzeptabel sind,
mit Ausnahme der folgenden Verbindungen:
- Methyl-(3,4,6-Tri-O-benzoyl-1-deoxy-1-fluoro-β-D-fructofuranosid),
- Ethylester der 2-Deoxy-2-fluoro-4,5,7-tris-O-(phenylmethyl)-D-arabino-3-heptulofuranosonsäure,
- 2-Deoxy-2-fluoro-4,5,7-tris-O-(phenylmethyl)-D-arabino-3-heptulofuranosonsäure,
- 1-Deoxy-1-fluoro-3,4,6-tris-O-(phenylmethyl)-D-fructofuranose,
- Diacetat der 1-Deoxy-1-fluoro-3,4-bis-O-(phenylmethyl)-D-fructofuranose, und
- 1-Deoxy-1-fluoro-3,4-bis-O-(phenylmethyl)-D-fructofuranose.

2. Verbindung nach Anspruch 1, **dadurch gekennzeichnet, dass** die linearen oder verzweigten Alkylgruppen jeweils Gruppen mit 1 bis 15 Kohlenstoffatomen sind.

3. Verfahren zur Herstellung einer Verbindung der Formel I nach Anspruch 1, bei der Y ein Wasserstoffatom repräsentiert, **dadurch gekennzeichnet, dass** es eine Reformatsky-Additionsreaktion eines Alkyl-Bromofluoroacetats in Gegenwart von Zink an die Lactone der folgenden Formel II umfasst: 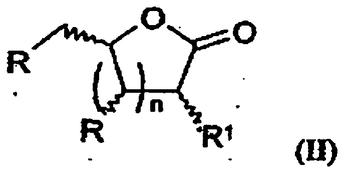 wobei n, R, R¹ wie in Anspruch 1 definiert sind.

4. Verfahren zur Herstellung einer Verbindung der Formel I nach Anspruch 1, bei der X und Y Wasserstoffatome repräsentieren und R² eine OH-Gruppe repräsentiert, **dadurch gekennzeichnet, dass** eine Verbindung der Formel I, wie in Anspruch 1 definiert, bei der R² = OH, Y = H und X = CO₂H ist, mit einem peptidischen Kupplungsagens zur Reaktion gebracht wird, wie 3-Ethyl-1-(N,N-dimethylaminopropyl)-carbodiimid oder Dicyclohexylcarbodiimid, und in Gegenwart eines tertiären Amins, wie N-Methylmorpholin oder Diisopropylethylamin.

5. Verfahren zur Herstellung einer Verbindung der Formel I nach Anspruch 1, bei der Y ein Wasserstoffatom repräsentiert, **dadurch gekennzeichnet, dass** es eine Reaktion eines Alkyl-Dibromofluoroacetats in Gegenwart von Diethylzink und Triphenylphosphin mit Lactonen der Formel II, wie in Anspruch 3 definiert, umfasst.

6. Verfahren zu Herstellung einer Verbindung der Formel I nach Anspruch 1, bei der Y ein Halogenatom wie Chlor oder Brom repräsentiert, **dadurch gekennzeichnet, dass** es eine Reaktion eines Alkyl-Dihalogenofluoroacetats in Gegenwart von Diethylzink mit Lactonen der Formel II, wie in Anspruch 3 definiert, umfasst.

7. Verfahren nach einem der Ansprüche 3 bis 6, **dadurch gekennzeichnet, dass** die Lactone der Formel II erhalten wurden durch Schritte des Schützens des Zuckers durch Benzylierung, gefolgt von der sauren Hydrolyse der anomeren Position, und dann deren Oxidation.

8. Verfahren zur Herstellung einer Verbindung der Formel I nach Anspruch 1, bei der R² ein Chlor- oder Bromatom repräsentiert, **dadurch gekennzeichnet, dass** eine Verbindung der Formel I, wie in Anspruch 1 definiert, bei der R² = OH ist, halogeniert wird.

9. Verfahren zur Herstellung einer Verbindung der Formel I nach Anspruch 1, bei der R² ein Wasserstoffatom repräsentiert, **dadurch gekennzeichnet, dass** eine Verbindung der Formel I, wie in Anspruch 1 definiert, bei der R² = Cl oder Br ist, reduziert wird.

10. Verbindung der Formel III: 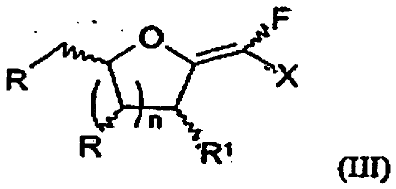 wobei n, R, R¹ und X wie in Anspruch 1 definiert sind.

11. Verfahren zur Herstellung einer Verbindung der Formel III nach Anspruch 10, bei der X = Br ist, **dadurch gekennzeichnet, dass** es die Reaktion eines Lactons der Formel II, wie in Anspruch 3 definiert, in Gegenwart von Tribromofluoromethan, Triphenylphosphin und Diethylzink umfasst.

12. Verfahren zur Herstellung einer Verbindung der Formel III nach Anspruch 10, bei der X = H ist, durch die Reaktion einer Verbindung der Formel I nach Anspruch 1, bei der X = CO₂H, R² = OH und Y = H ist, in Gegenwart eines peptidischen Kupplungsagens, wie 3-Ethyl-1-(N,N-dimethylaminoproyl)carbodiimid oder Dicyclohexylcarbodiimid, und in Gegenwart eines tertiären Amins, wie N-Methylmorpholin oder Diisopropylethylamin.

13. Verfahren zur Herstellung einer Verbindung der allgemeinen Formel I nach Anspruch 1, bei der R² = H und Y = H ist, durch Reduktion der Doppelbindung der Verbindung der allgemeinen Formel III, wie in Anspruch 9 definiert.

14. Verbindung nach Anspruch 1, **dadurch gekennzeichnet, dass** sie der folgenden Formel IV entspricht: 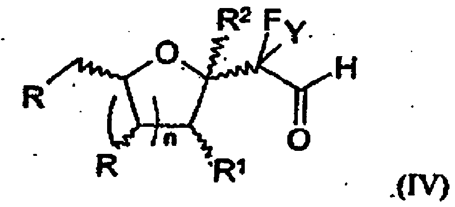 wobei
n eine ganze Zahl gleich 2 ist,
Y ein Wasserstoffatom repräsentiert,
R² ein Wasserstoffatom oder eine OH- oder OR'-Gruppe repräsentiert.

15. Verbindung der folgenden Formel V: 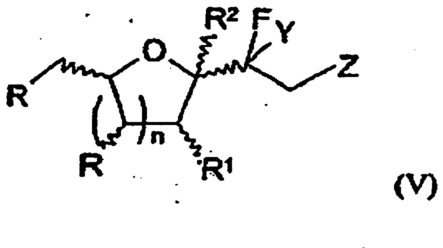 wobei
n eine ganze Zahl gleich 2 ist,
Y ein Wasserstoffatom repräsentiert,
R² ein Wasserstoffatom oder eine OH- oder OR'-Gruppe repräsentiert,
Z eine OH- oder OR³-Gruppe repräsentiert mit R³ = Alkyl, Benzyl, Mesyl, Tosyl oder Triflat, oder ein Halogenatom wie Cl, Br oder I.

16. Verbindung der folgenden Formel VI: 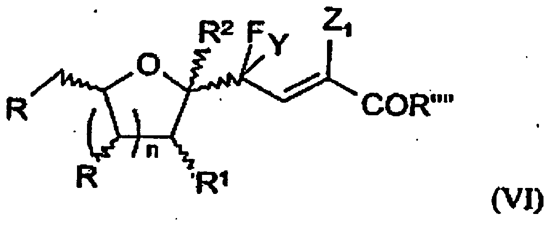 wobei
n eine ganze Zahl gleich 2 ist,
Y ein Wasserstoffatom repräsentiert,
R² ein Wasserstoffatom oder eine OH- oder OR'-Gruppe repräsentiert,
Z₁ H oder NR"R"' repräsentiert, wobei R" und R'" identisch oder verschieden sind und ein Wasserstoffatom oder eine lineare oder verzweigte Alkylgruppe oder eine Aryl-, Benzyl-, Benzoyl-, Acetyl-, Alkyloxycarbonyl-, Allyloxycarbonyl- oder Benzyloxycarbonylgruppe repräsentieren, R"" OR" oder NR"R"' oder eine Aminosäure repräsentiert.

17. Verbindung der folgenden Formel VII: 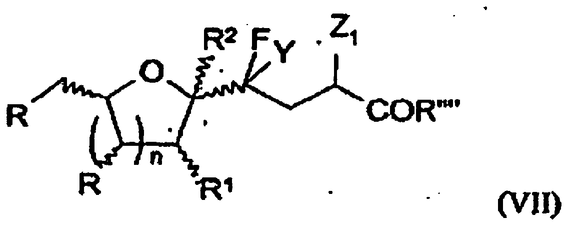 wobei
n eine ganze Zahl gleich 2 ist,
Y ein Wasserstoffatom repräsentiert, R² ein Wasserstoffatom oder eine OH- oder OR'-Gruppe repräsentiert,
Z₁ H oder NR"R'" repräsentiert, wobei R" und R'" identisch oder verschieden sind und ein Wasserstoffatom oder eine lineare oder verzweigte Alkylgruppe oder eine Aryl-, Benzyl-, Benzoyl-, Acetyl-, Alkyloxycarbonyl-, Allyloxycarbonyl- oder Benzyloxycarbonylgruppe repräsentieren,
R"" OR" oder NR"R'" oder eine Aminosäure repräsentiert.

18. Verbindung der folgenden Formel VIII: 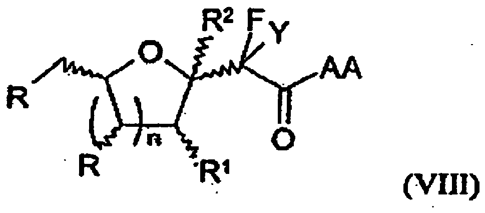 wobei
n eine ganze Zahl gleich 2 ist,
Y ein Wasserstoffatom repräsentiert,
R² ein Wasserstoffatom oder eine OH- oder OR'-Gruppe repräsentiert, und
AA eine Aminosäure oder ein Peptid repräsentiert.

19. Verbindung der folgenden Formel IX: 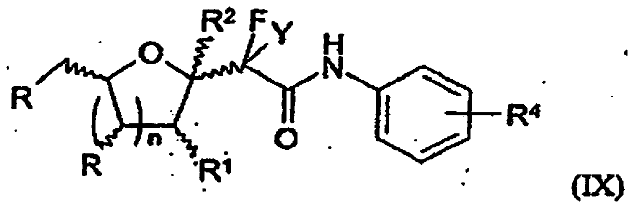 wobei
n eine ganze Zahl gleich 2 ist,
Y ein Wasserstoffatom repräsentiert,
R² ein Wasserstoffatom oder eine OH- oder OR'-Gruppe repräsentiert,
R⁴ ein Wasserstoff, Halogen, NR"R"', OH oder OR" repräsentiert, wobei R" und R'" identisch oder verschieden sind und ein Wasserstoffatom oder eine lineare oder verzweigte Alkylgruppe oder eine Aryl-, Benzyl-, Benzoyl-, Acetyl-, Alkyloxycarbonyl-, Allyloxycarbonyl- oder Benzyloxycarbonylgruppe repräsentieren.

20. Verbindung der Formel I nach Anspruch 1, bei der R² eine OH-Gruppe repräsentiert, **dadurch gekennzeichnet, dass** sie in offener Form, in Furanoseform und in Pyranoseform vorliegt, wenn sie in polaren und protischen Lösungsmitteln gelöst ist.

21. Verbindung nach einem der Ansprüche 1, 2, 10 oder 14 bis 19, ausgewählt aus:
